# EUROPEAN PATENT APPLICATION

(11) **EP 0 574 257 A2**
(43) Date of publication of application: **15.12.1993**
(21) Application number: 93304500.7
(22) Date of filing: 10.06.1993
(51) Int. Cl.: C07K 15/00, C12N 15/12, C12N 5/08, C12P 21/02, C12P 21/08, G01N 33/48

(54) **Amino-hydroxy-methyl-isoxazole-propionate binding human glutamate receptors**

(30) Priority: 10.06.1992 US 896611; 10.06.1992 US 896612; 10.06.1992 US 896437
(71) Applicant: Allelix Biopharmaceuticals Inc., Mississauga, Ontario, L4V 1V7 (CA)
(72) Inventor: Allelix Biopharmaceuticals Inc., Mississauga, Ontario, L4V 1V7 (CA)
(74) Representative: Mercer, Christopher Paul

(57) **Abstract**

Described herein are isolated polynucleotides which code for a family of AMPA-type human CNS receptors. The receptors are characterized structurally and the construction and use of cell lines expressing these receptors are disclosed.

## Description

### Background of the Invention

### Field of the Invention

This invention is concerned with applications of recombinant DNA technology in the field of neurobiology. More particularly, the invention relates to the cloning and expression of DNA coding for excitatory amino acid (EAA) receptors, especially human EAA receptors.

### Background of the Invention

In the mammalian central nervous system (CNS), the transmission of nerve impulses is controlled by the interaction between a neurotransmitter substance released by the "sending" neuron which then binds to a surface receptor on the "receiving" neuron to cause excitation thereof. L-glutamate is the most abundant neurotransmitter in the CNS, and mediates the major excitatory pathway in vertebrates. Glutamate is therefore referred to as an excitatory amino acid (EAA) and the receptors which respond to it are variously referred to as glutamate receptors, or more commonly as EAA receptors.

Using tissues isolated from mammalian brain, and various synthetic EAA receptor agonists, knowledge of EAA receptor pharmacology has been refined somewhat. Members of the EAA receptor family are now grouped into three main types based on differential binding to such agonists. One type of EAA receptor, which in addition to glutamate also binds the agonist NMDA (N-methyl-D-aspartate), is referred to as the NMDA type of EAA receptor. Two other glutamate-binding types of EAA receptor, which do not bind NMDA, are named according to their preference for binding with two other EAA receptor agonists, namely AMPA (α-amino-3-hydroxy-5-methyl-isoxazole-4-propionate), and kainate. Particularly, receptors which bind glutamate but not NMDA, and which bind with greater affinity to kainate than to AMPA, are referred to as kainate type EAA receptors. Similarly, those EAA receptors which bind glutamate but not NMDA, and which bind AMPA with greater affinity than kainate are referred to as AMPA type EAA receptors.

The glutamate-binding EAA receptor family is of great physiological and medical importance. Glutamate is involved in many aspects of long-term potentiation (learning and memory), in the development of synaptic plasticity, in epileptic seizures, in neuronal damage caused by ischemia following stroke or other hypoxic events, as well as in other forms of neurodegenerative processes. However, the development of therapeutics which modulate these processes has been very difficult, due to the lack of any homogeneous source of receptor material with which to discover selectively binding drug molecules, which interact specifically at the interface of the EAA receptor. The brain derived tissues currently used to screen candidate drugs are heterogeneous receptor sources, possessing on their surface many receptor types which interfere with studies of the EAA receptor/ligand interface of interest. The search for human therapeutics is further complicated by the limited availability of brain tissue of human origin. It would therefore be desirable to obtain cells that are genetically engineered to produce only the receptor of interest. With cell lines expressing cloned receptor genes, a substrate which is homogeneous for the desired receptor is provided, for drug screening programs.

Recently, genes encoding substituent polypeptides of EAA receptors from non-human sources, principally rat, have been discovered. Hollmann et al., Nature 342: 643, 1989 described the isolation from rat of a gene referred to originally as GluR-K1 (but now called simply GluR1). This gene encodes a member of the rat EAA receptor family, and was originally suspected as being of the kainate type. Subsequent studies by Keinanen et al., Science 249: 556, 1990, showed, again in rat, that a gene called GluR-A, which was identical to the previously isolated GluR1, in fact encodes a receptor not of the kainate type, but rather of the AMPA type. These two groups of researchers have since reported as many as five related genes isolated from rat sources. Boulter et al., Science 249: 1033, 1990, revealed that, in addition to GluR1, the rat contains 3 other related genes, which they called GluR2, GluR3, and GluR4, and Bettler et al., Neuron 5: 583. 1990 described GluR5. Keinanen et al., supra, described genes called GluR-A, GluR-B, GluR-C and GluR-D which correspond precisely to GluR1, GluR2, GluR3 and GluR4 respectively. Sommer et al., Science 249: 1580, 1990 also showed, for GluR-A, GluR-B, GluR-C and GluR-D two alternatively spliced forms for each gene. These authors, as well as Monyer et al., Neuron 6: 799, 1991 were able to show that the differently spliced versions of these genes are differentially expressed in the rat brain.

There has emerged from these molecular cloning advances a better understanding of the structural features of EAA receptors and their subunits, as they exist in the rat brain. According to the current model of EAA receptor structure, each is heteromeric in structure, consisting of individual membrane-anchored subunits, each having four transmembrane regions, and extracellular domains that dictate ligand binding properties to some extent and contribute to the ion-gating function served by the receptor complex. Keinanen et al, supra, have shown for example that each subunit of the rat GluR receptor, including those designated GluR-A, GluR-B, GluR-C and GluR-D, display cation channel activity gated by glutamate, by AMPA and by kainate, in their unitary state. When expressed in combination however, for example GluR-A in combination with GluR-B, gated ion channels with notably larger currents are produced by the host mammalian cells.

In the search for therapeutics useful to treat CNS disorders in humans, it is highly desirable of course to provide a screen for candidate compounds that is more representative of the human situation than is possible with the rat receptors isolated to date. It is particularly desirable to provide cloned genes coding for human receptors, and cell lines expressing those genes, in order to generate a proper screen for human therapeutic compounds. These, accordingly, are objects of the present invention.

### Summary of the Invention

The present invention provides isolated polynucleotides that code for a family of AMPA-binding human EAA receptors, herein referred to as "GluR receptors". By providing polynucleotides that code specifically for CNS receptors native to humans, the present invention provides means for evaluating the human nervous system, and particularly for assessing potentially therapeutic interactions between the AMPA-binding human EAA receptors and selected natural and synthetic ligands.

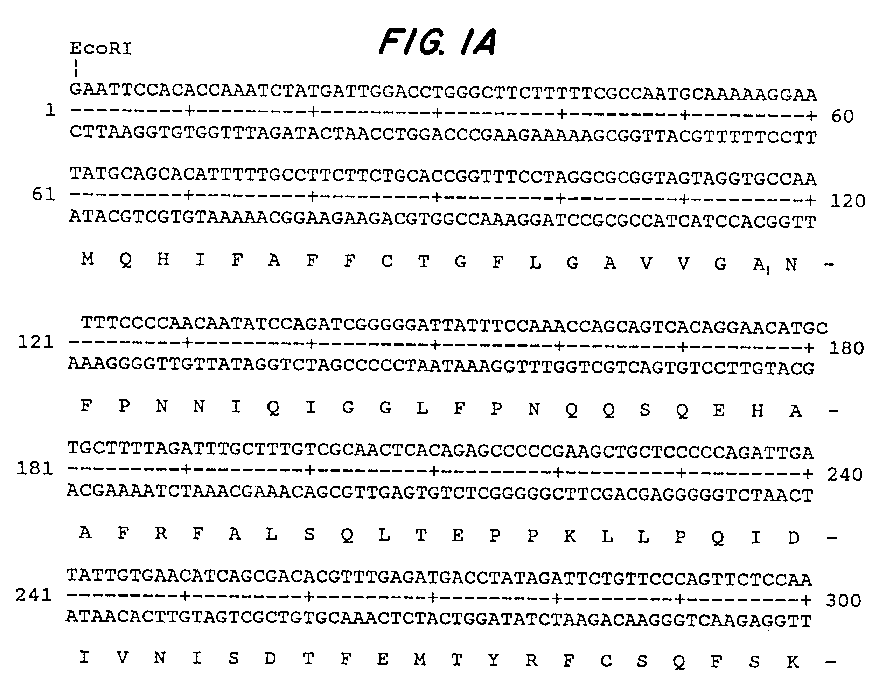

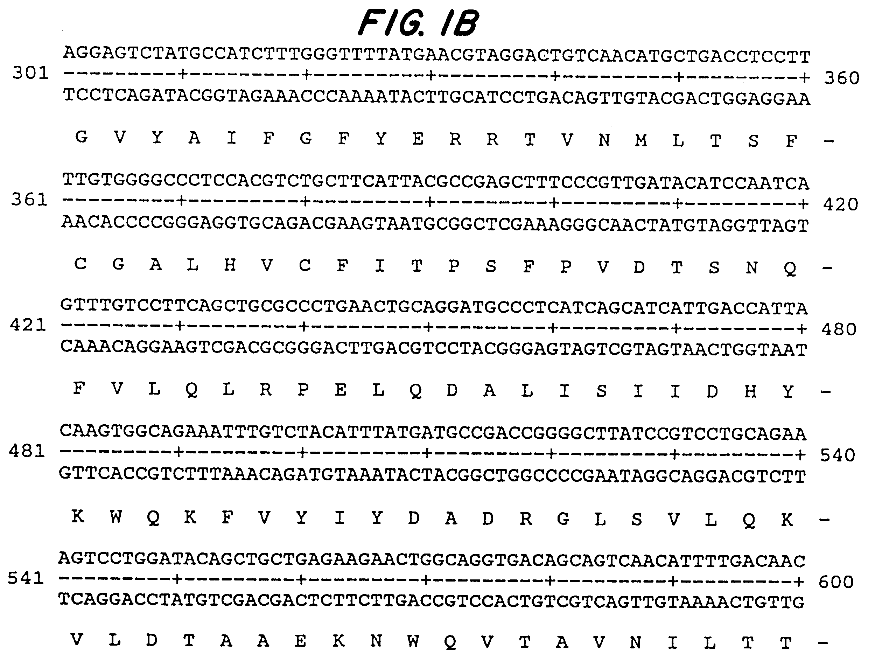

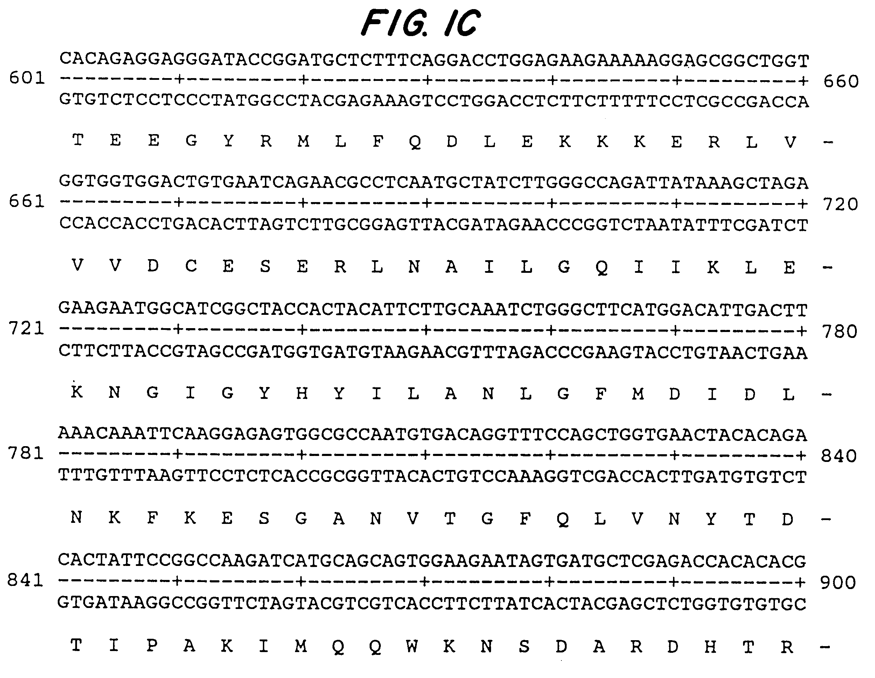

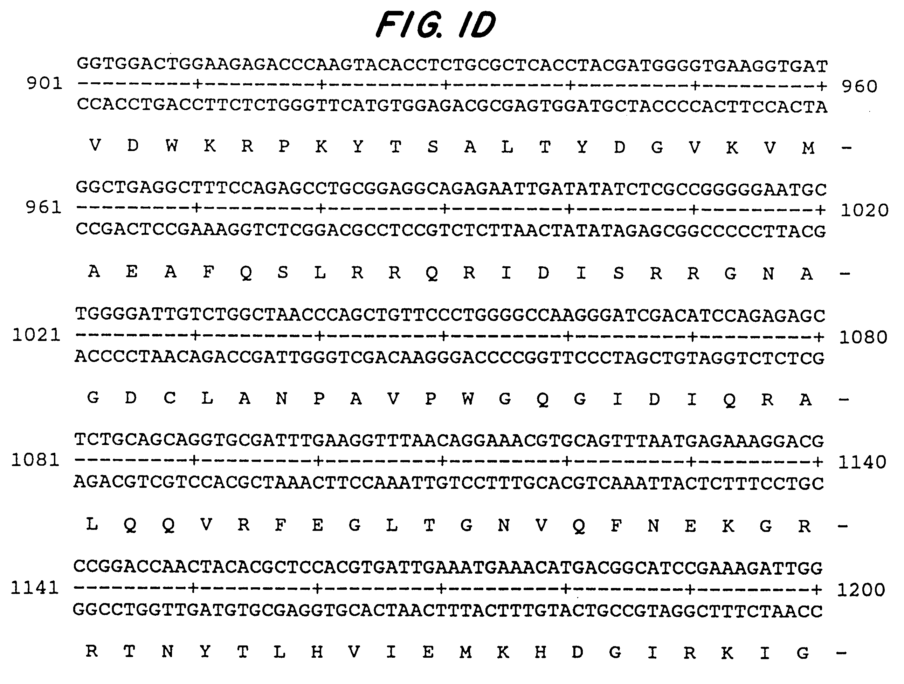

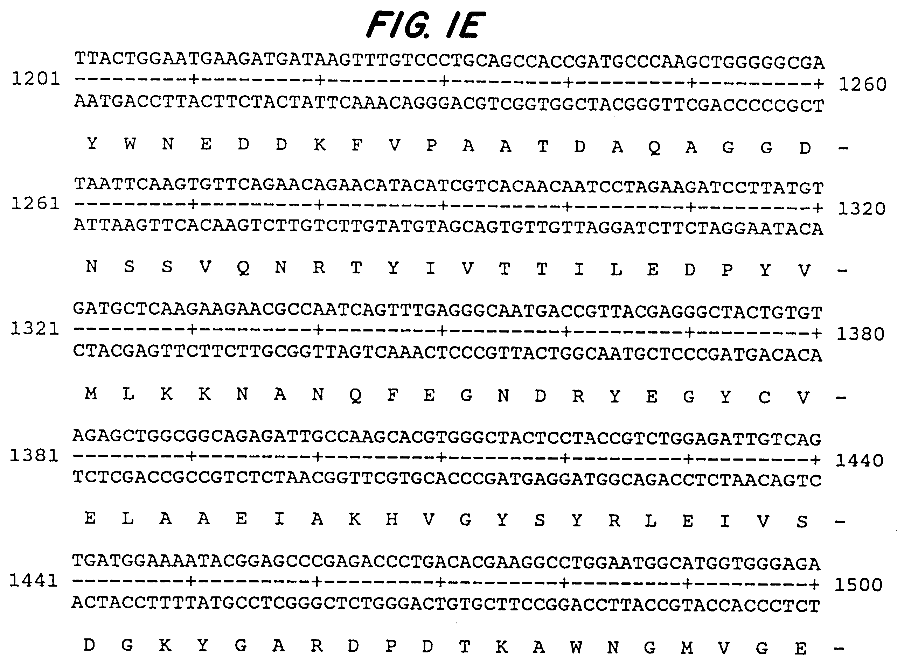

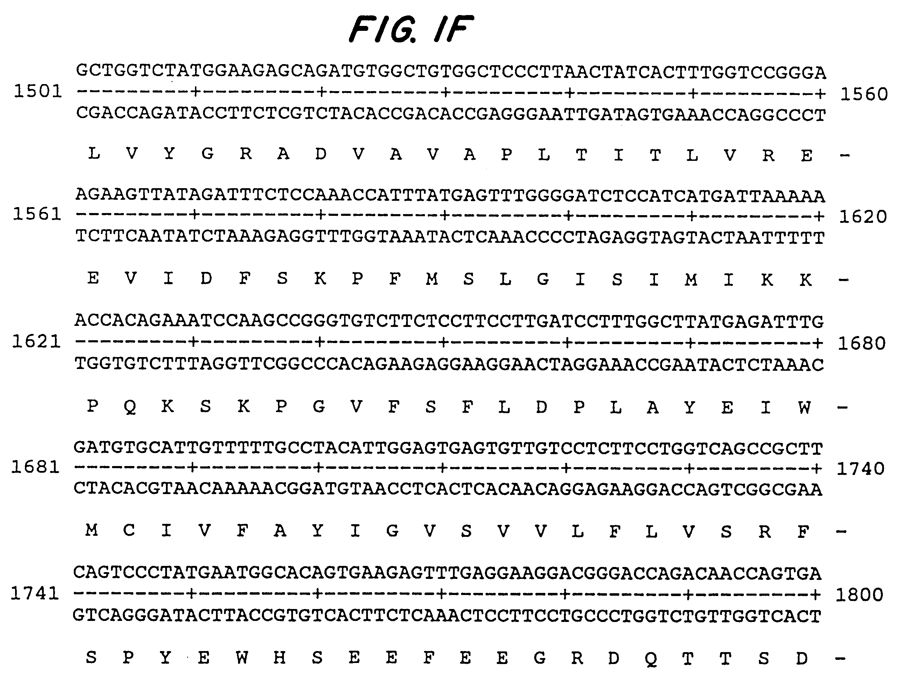

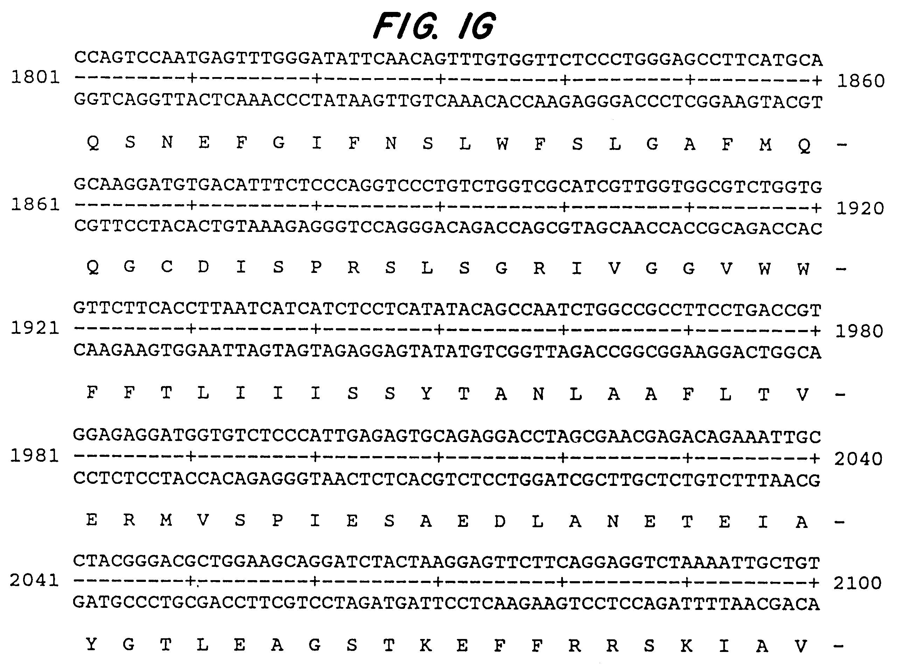

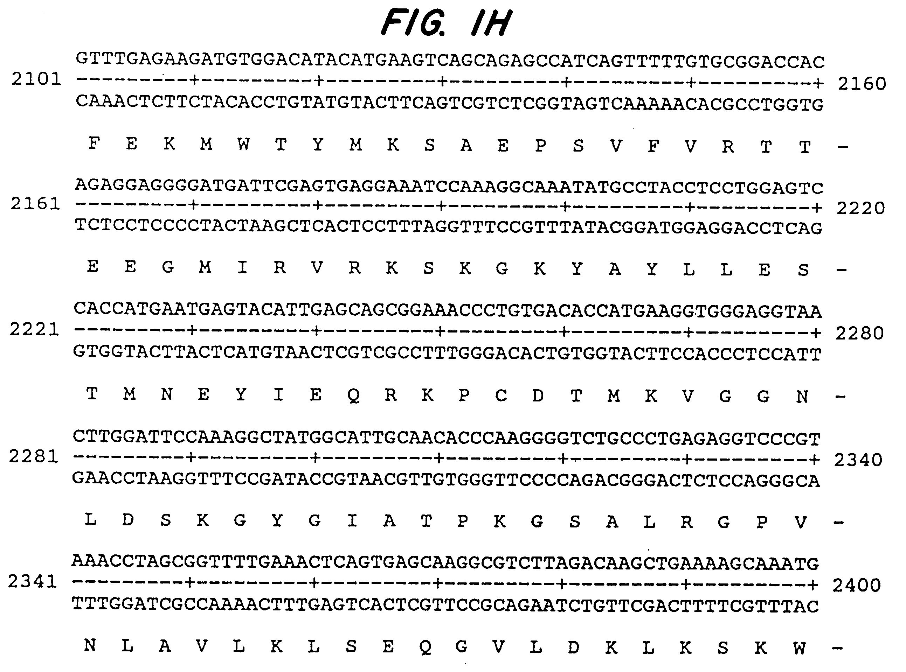

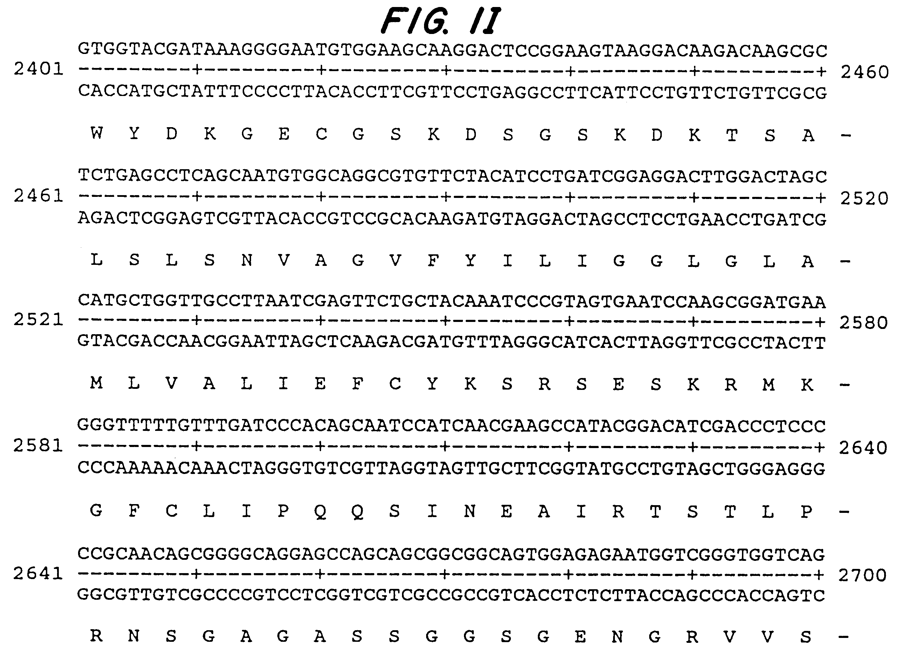

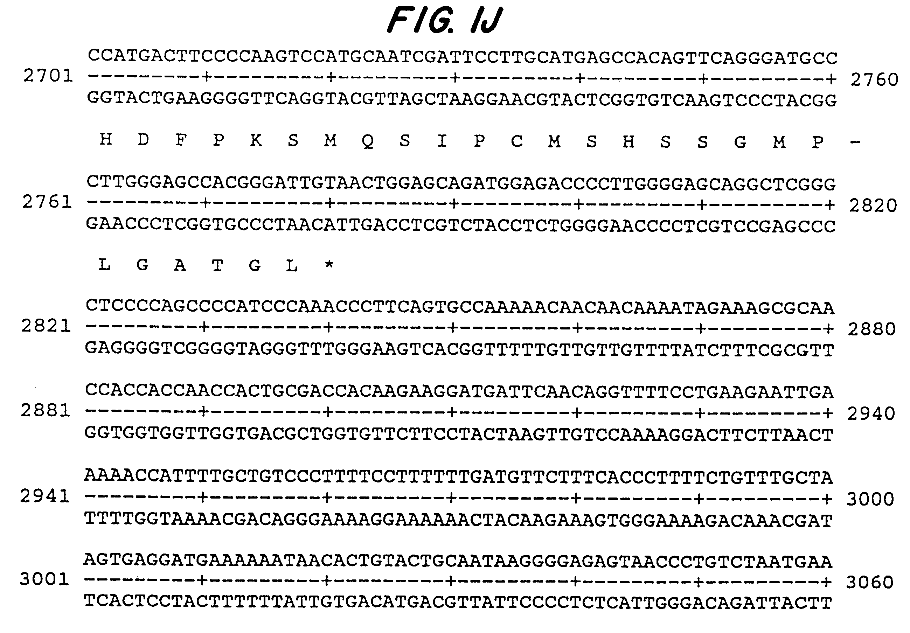

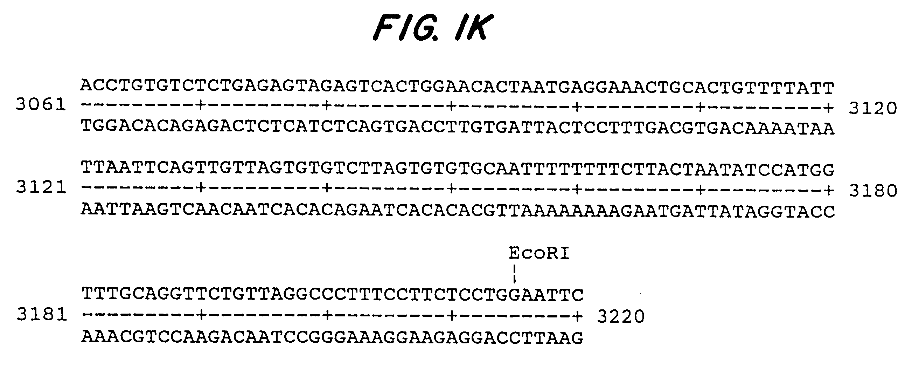

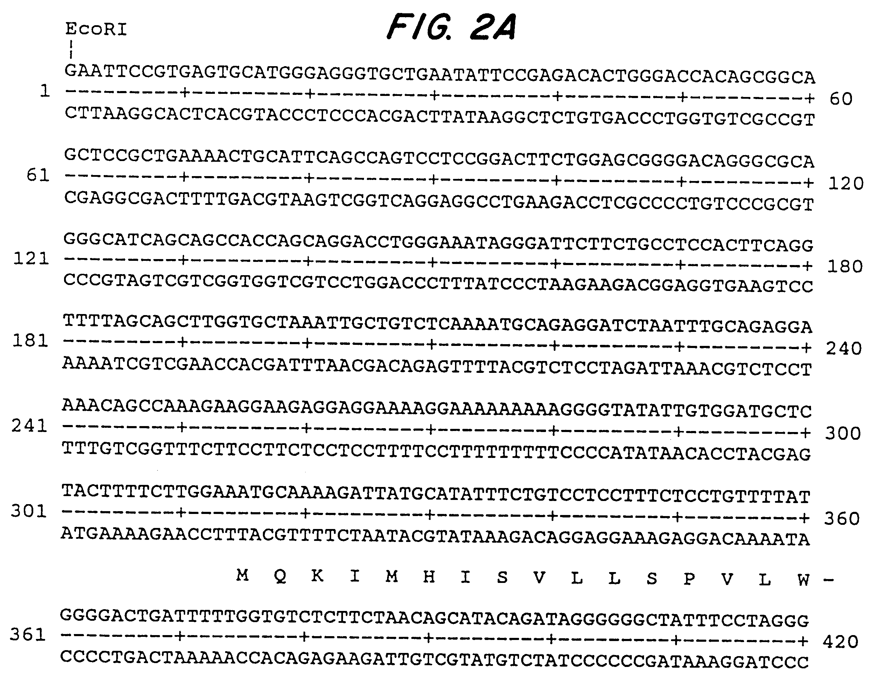

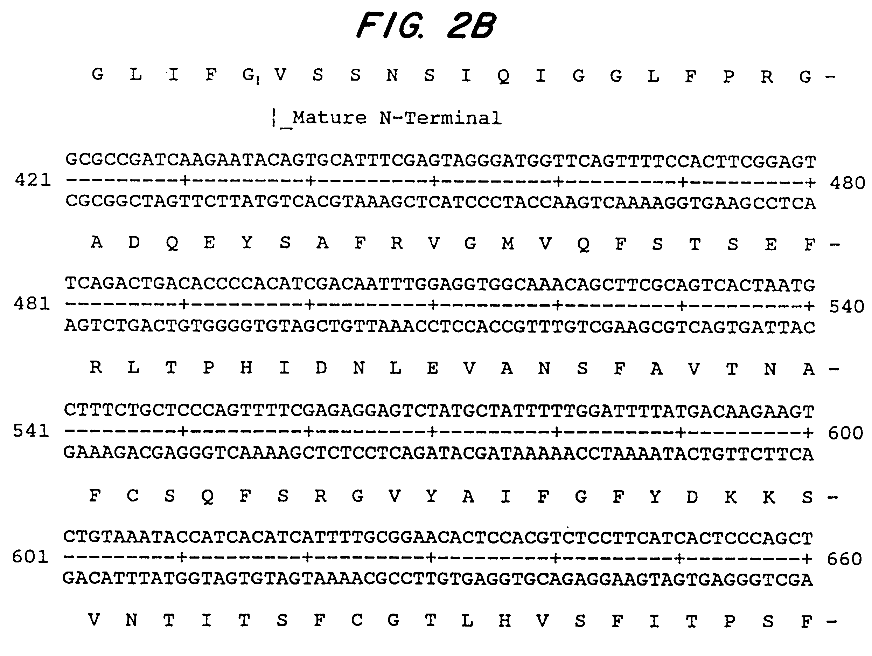

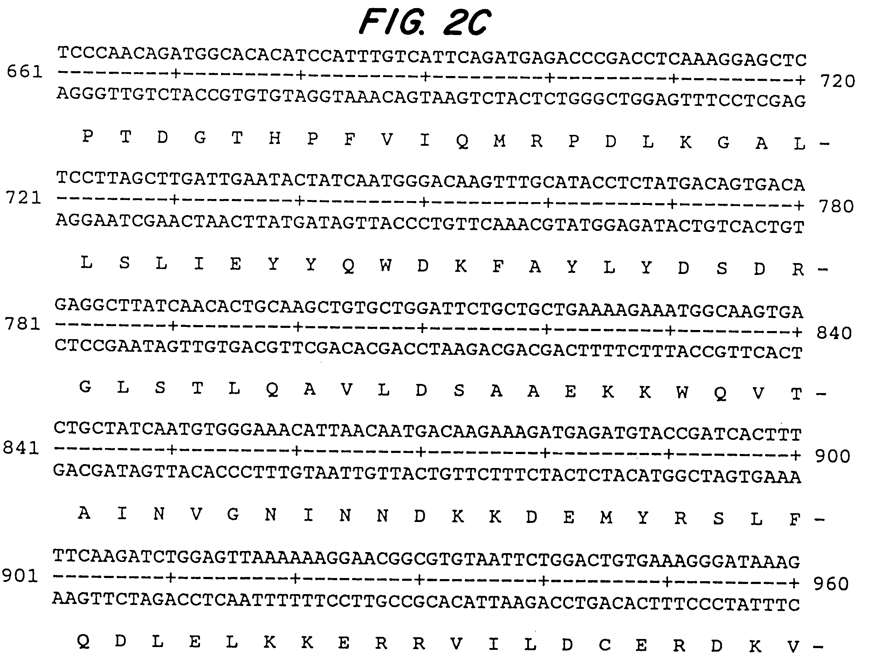

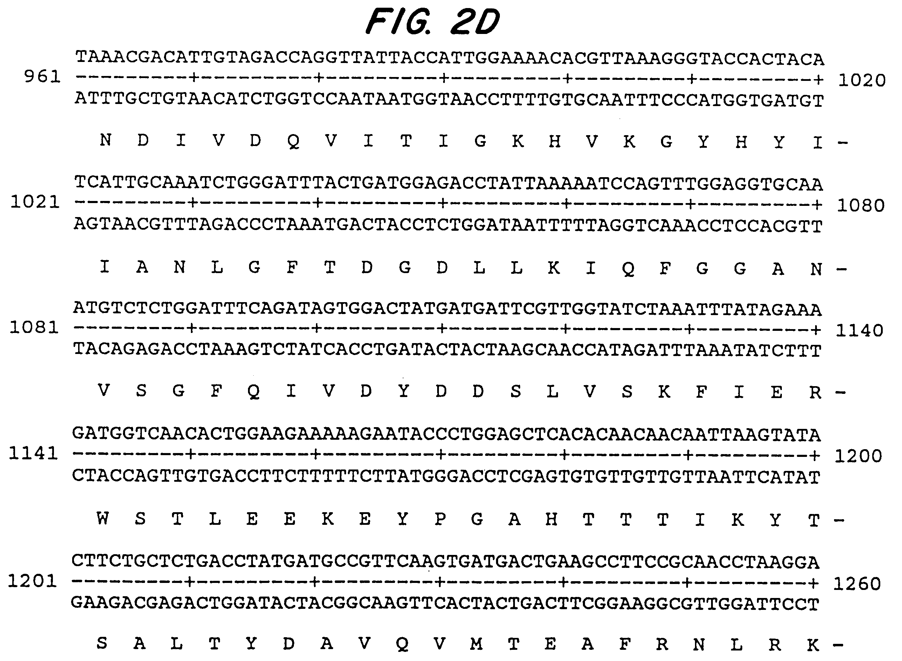

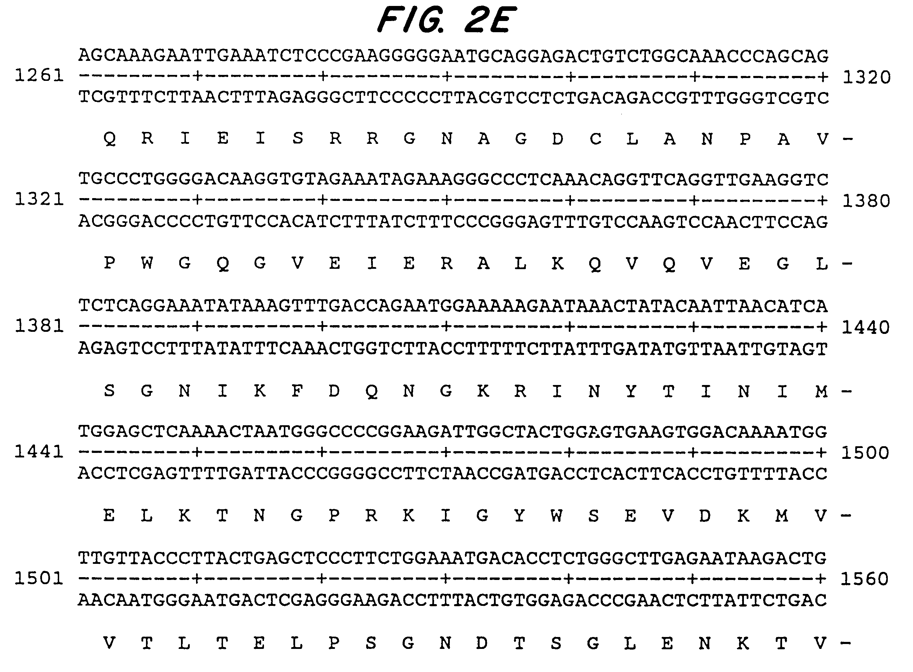

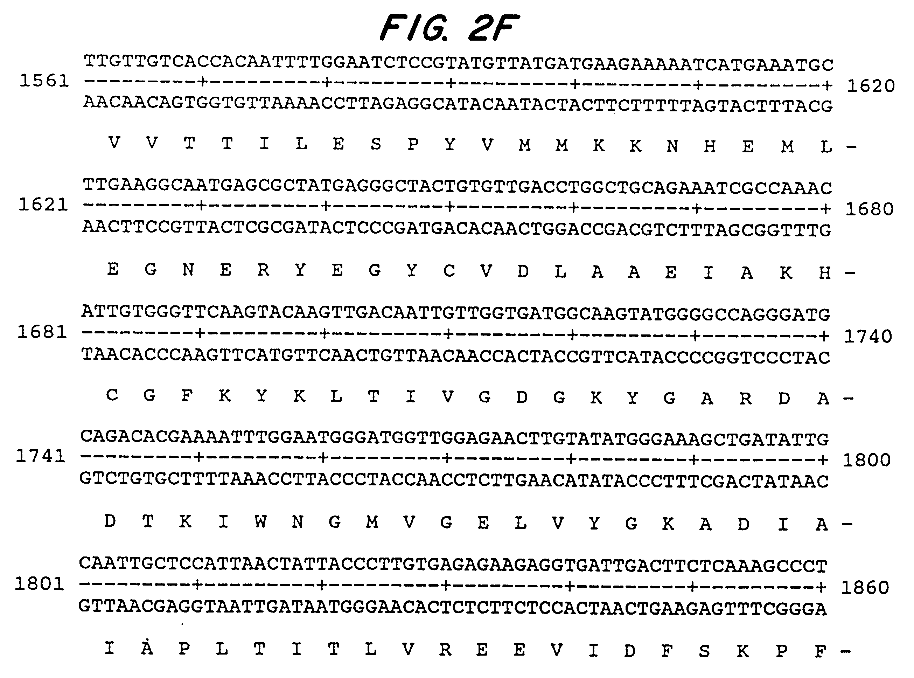

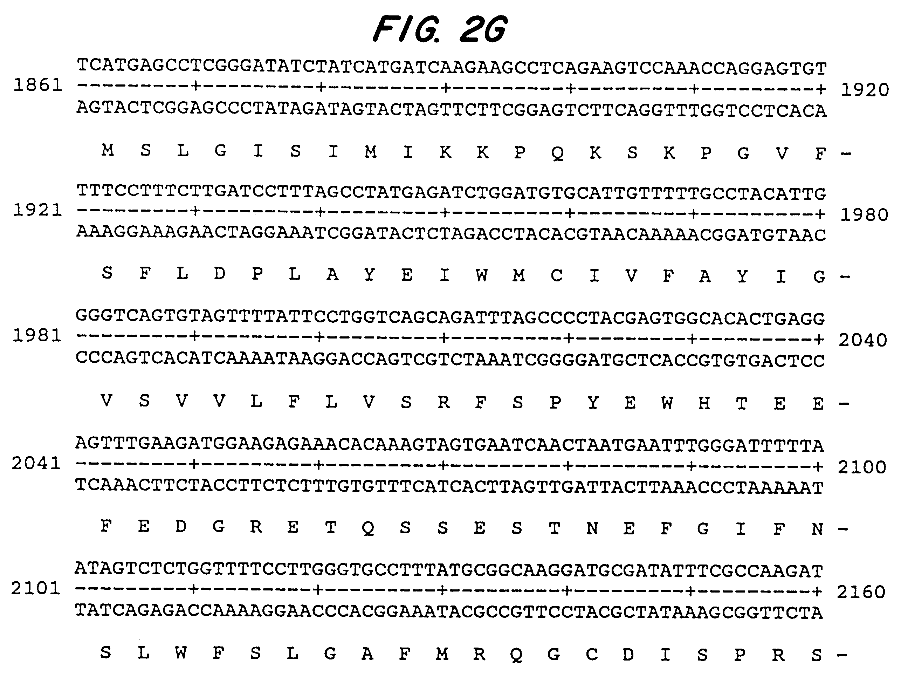

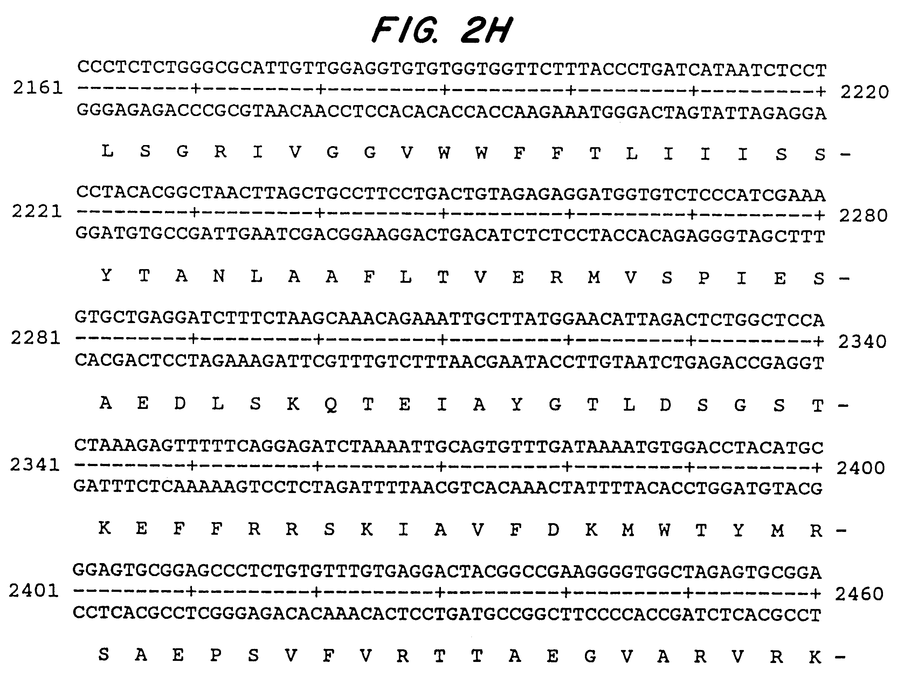

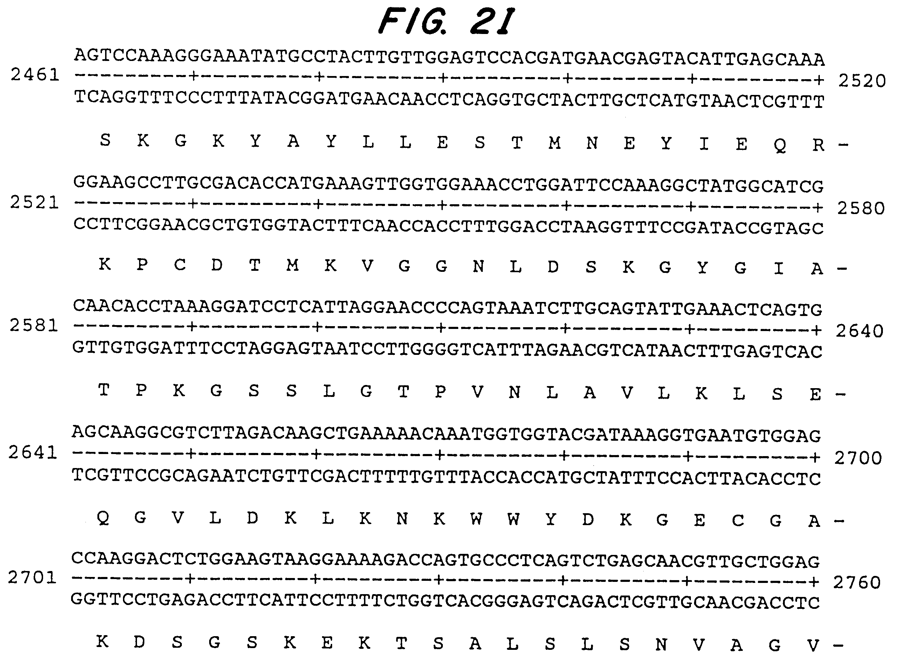

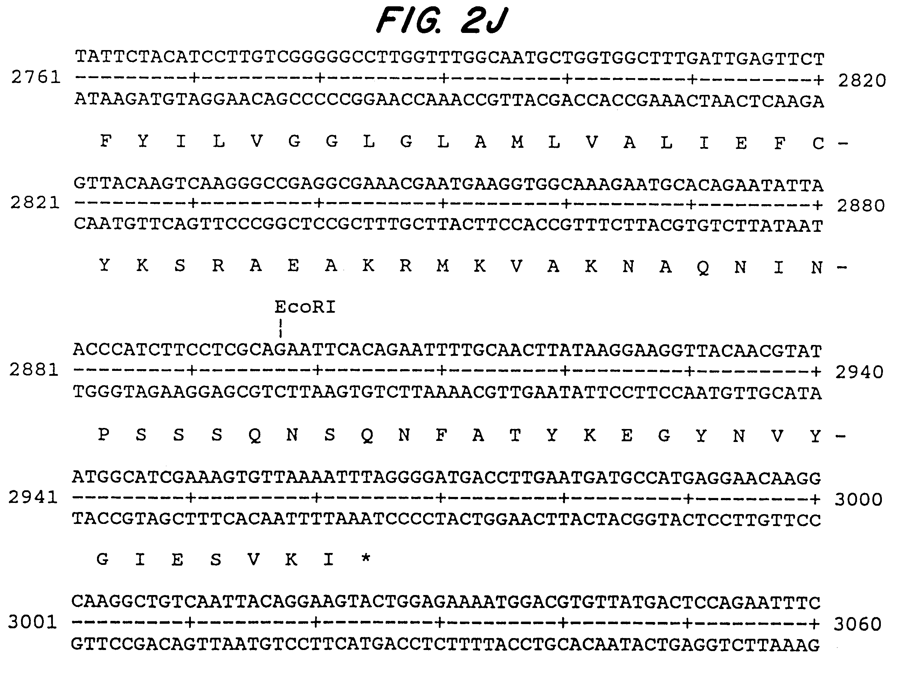

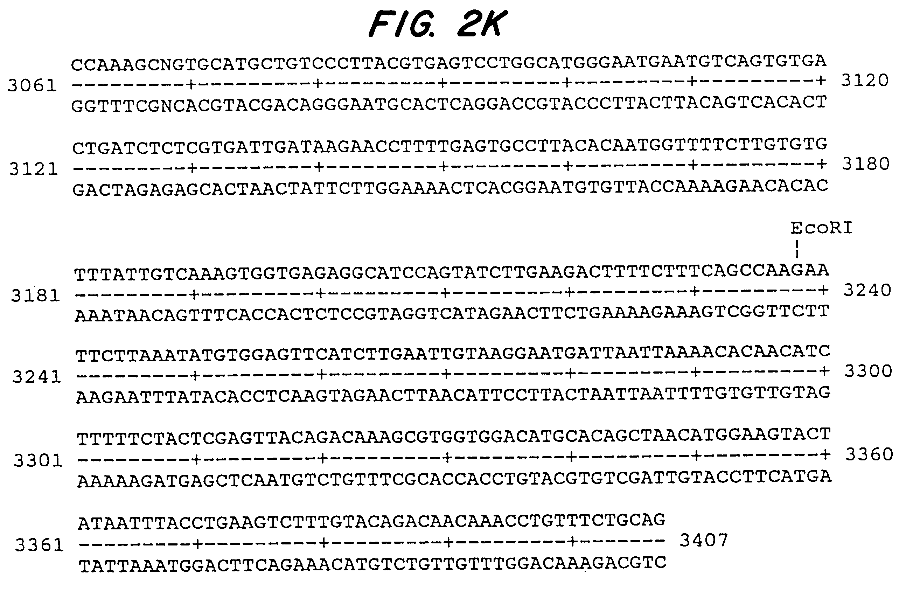

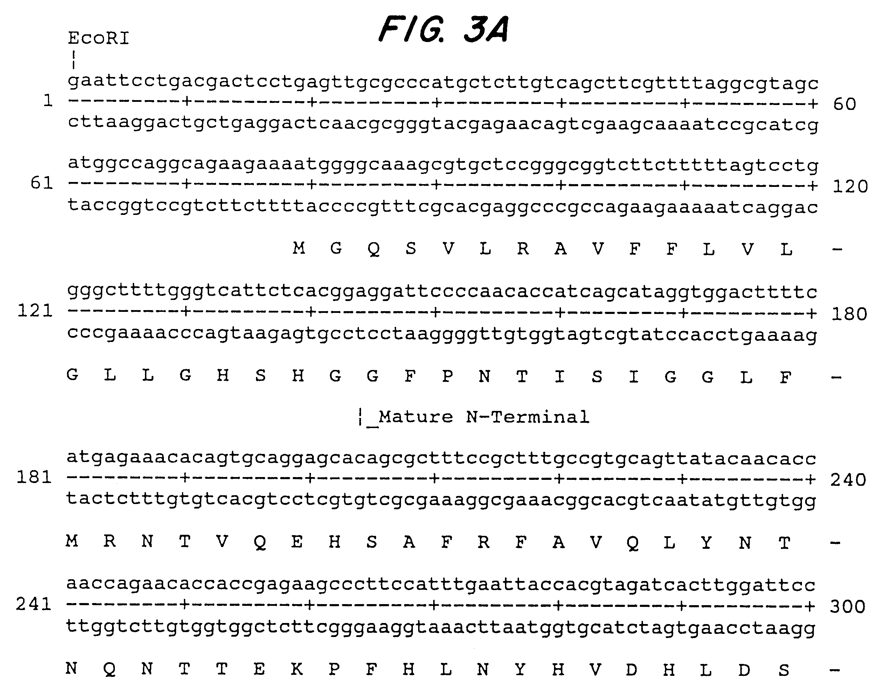

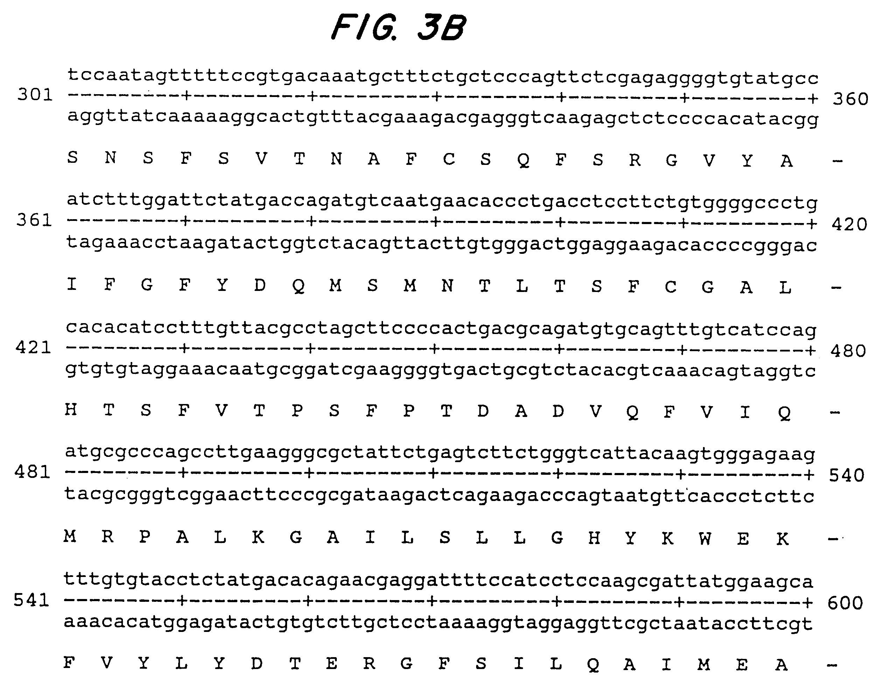

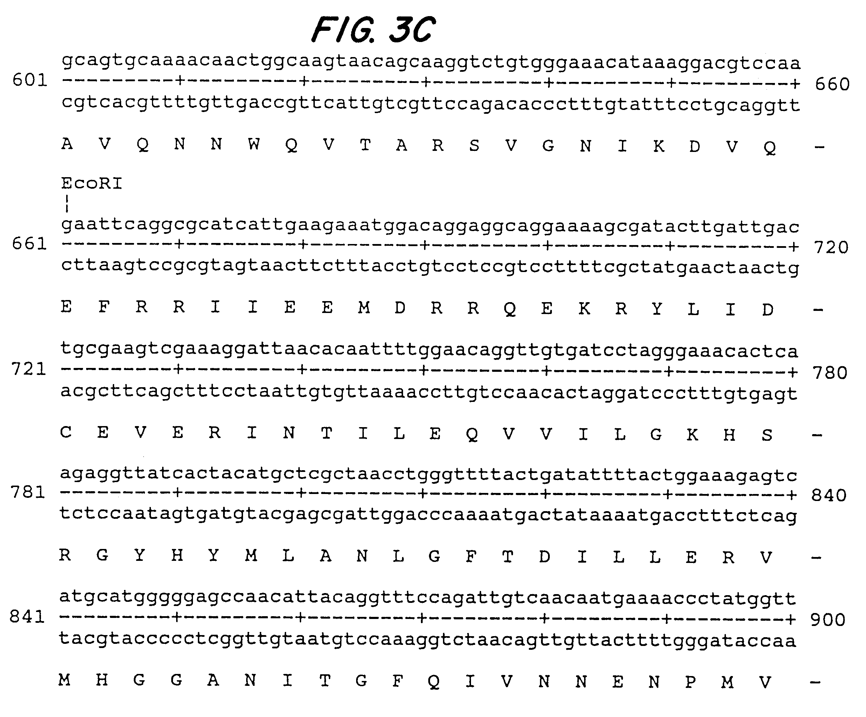

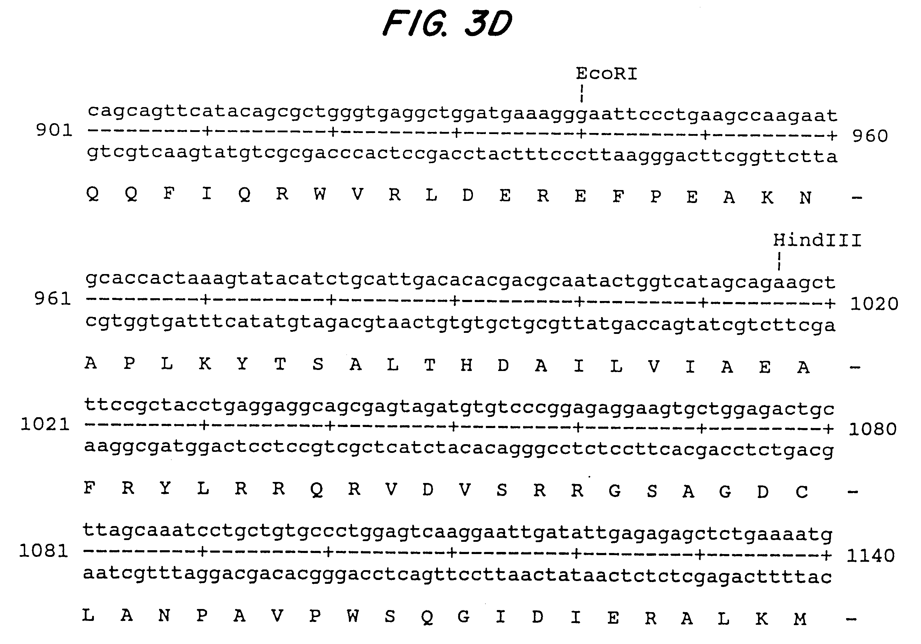

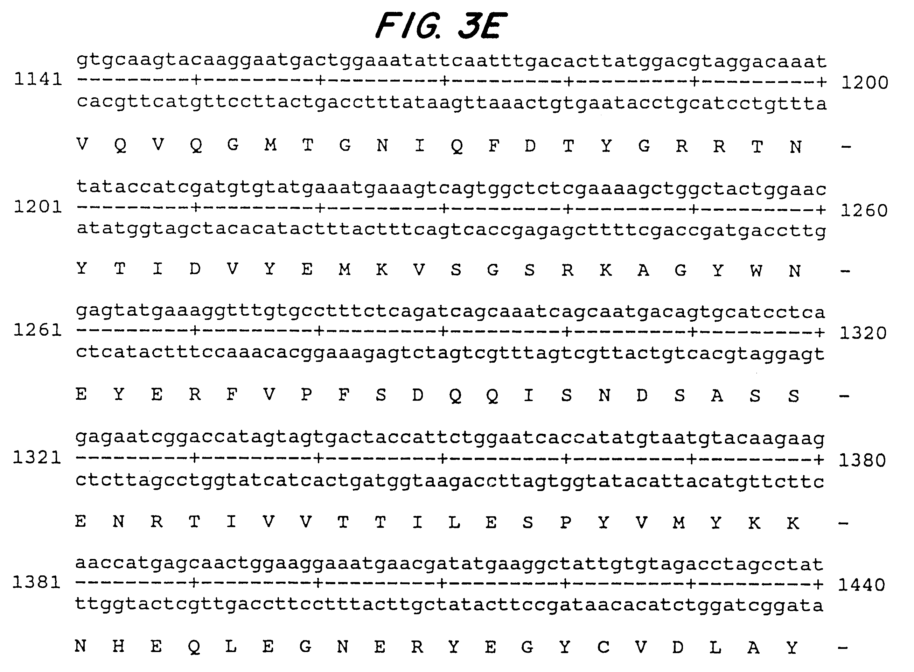

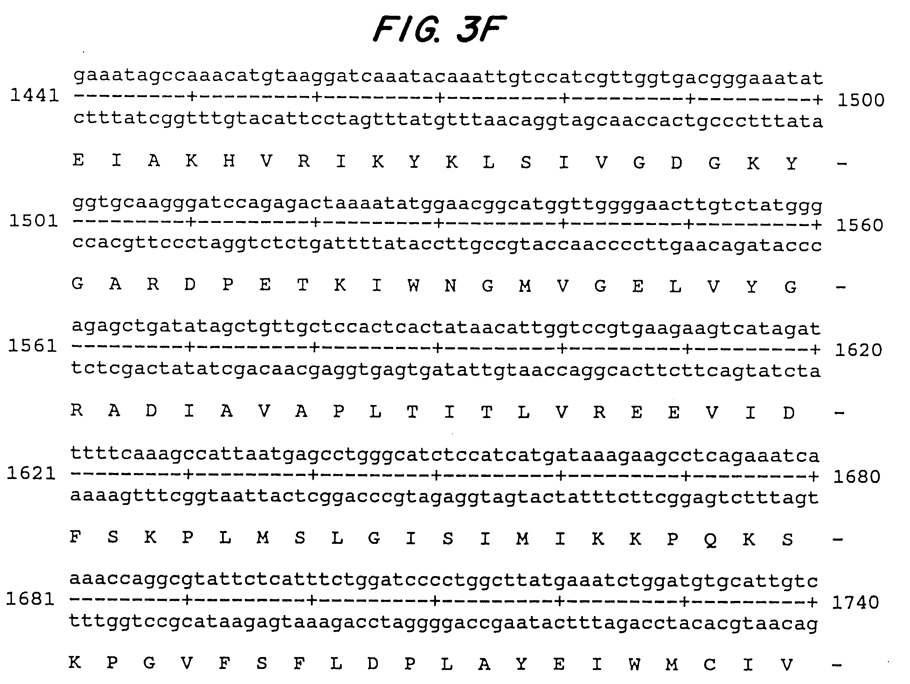

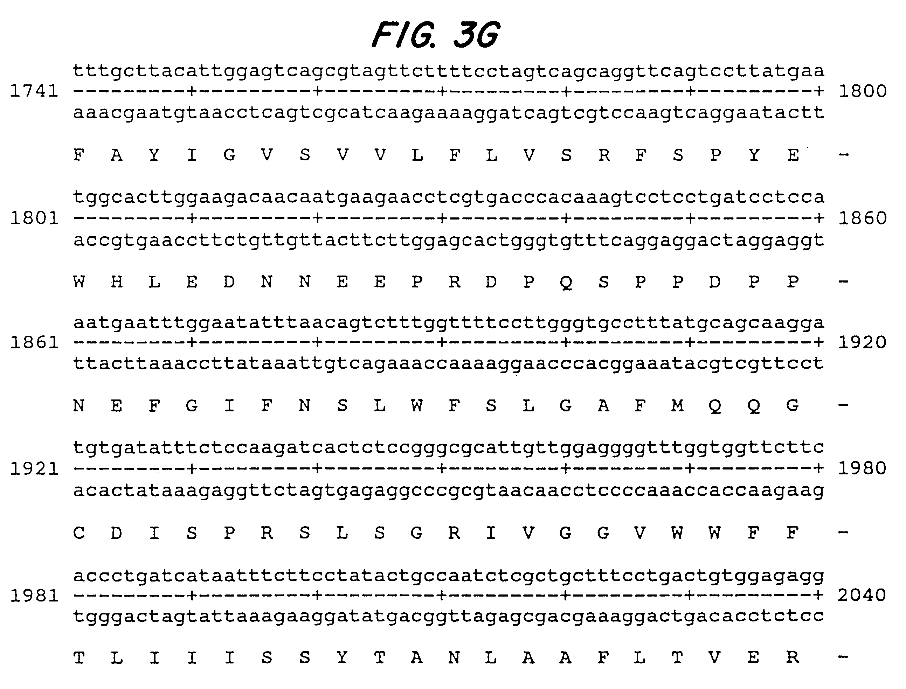

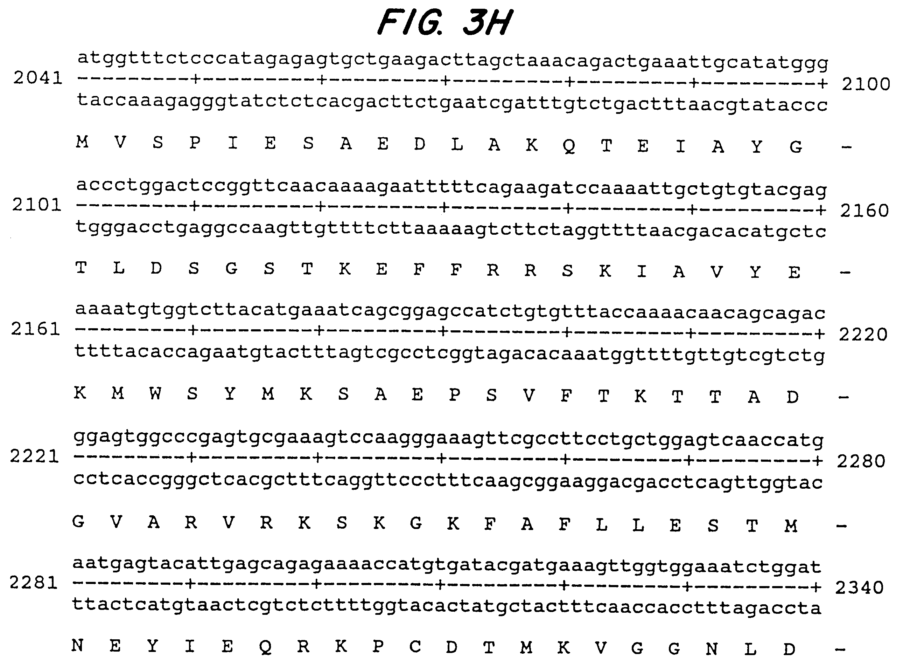

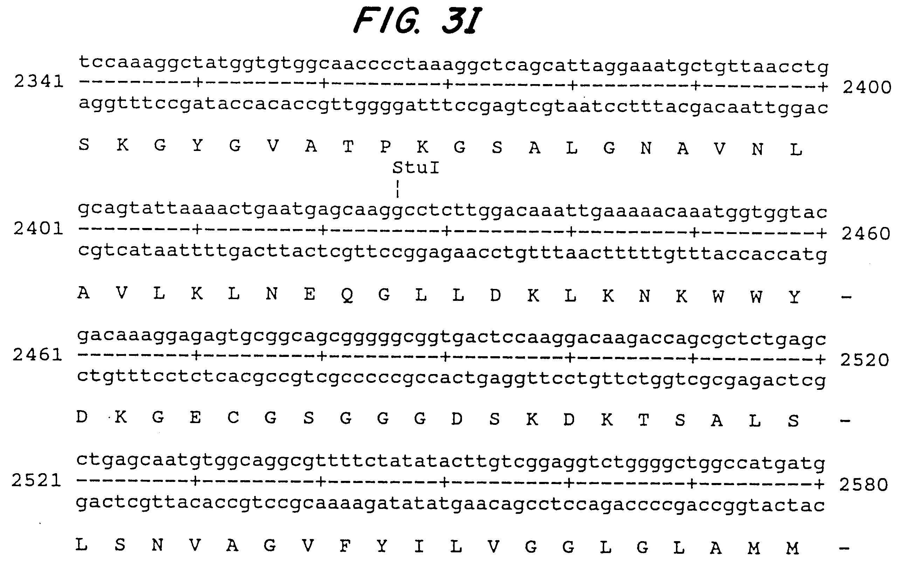

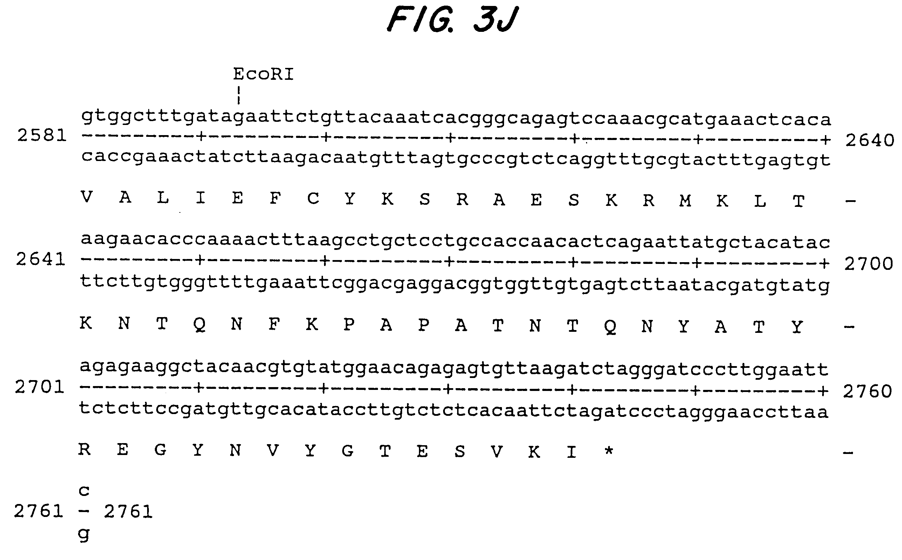

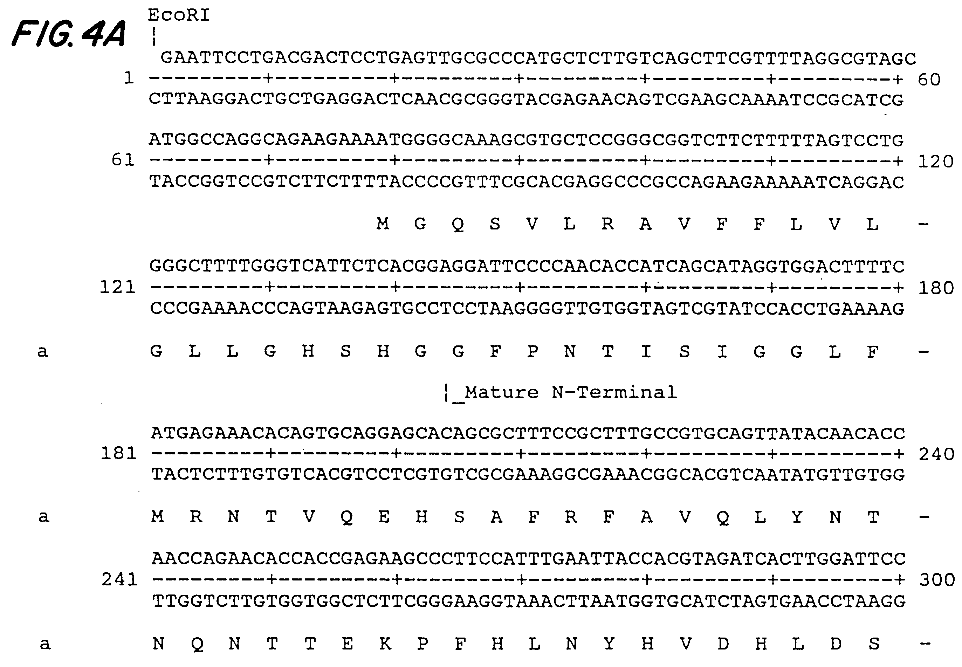

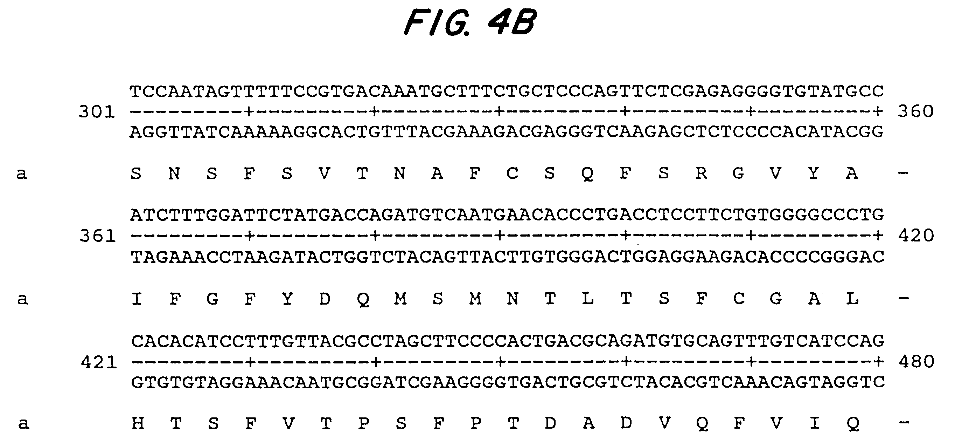

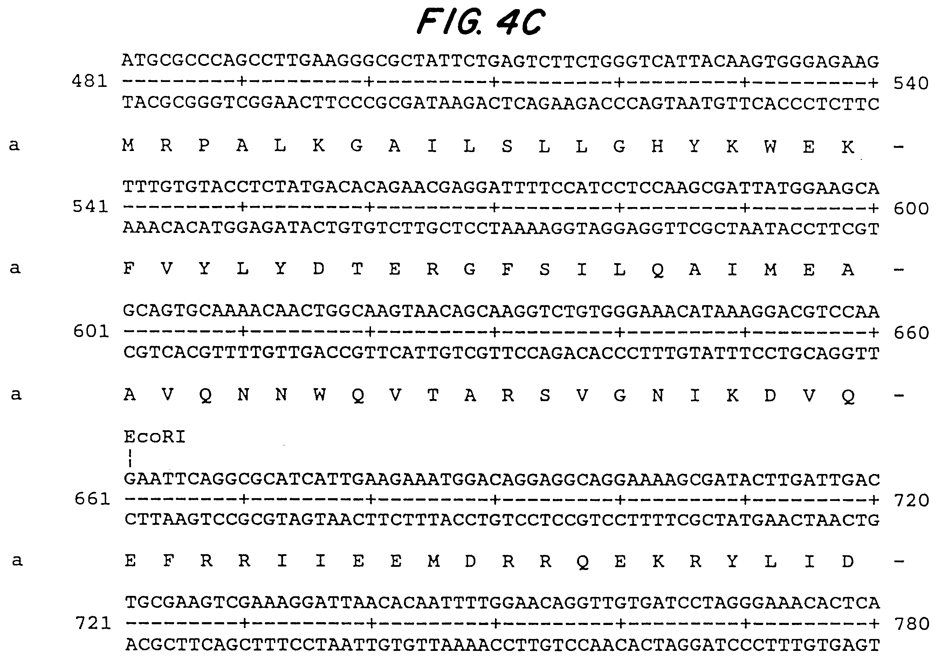

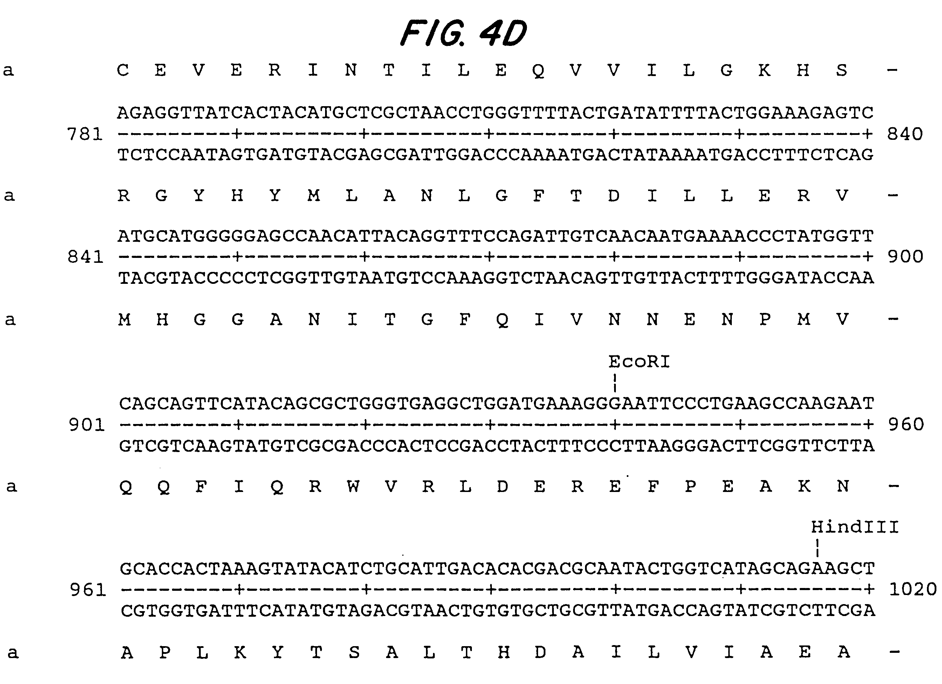

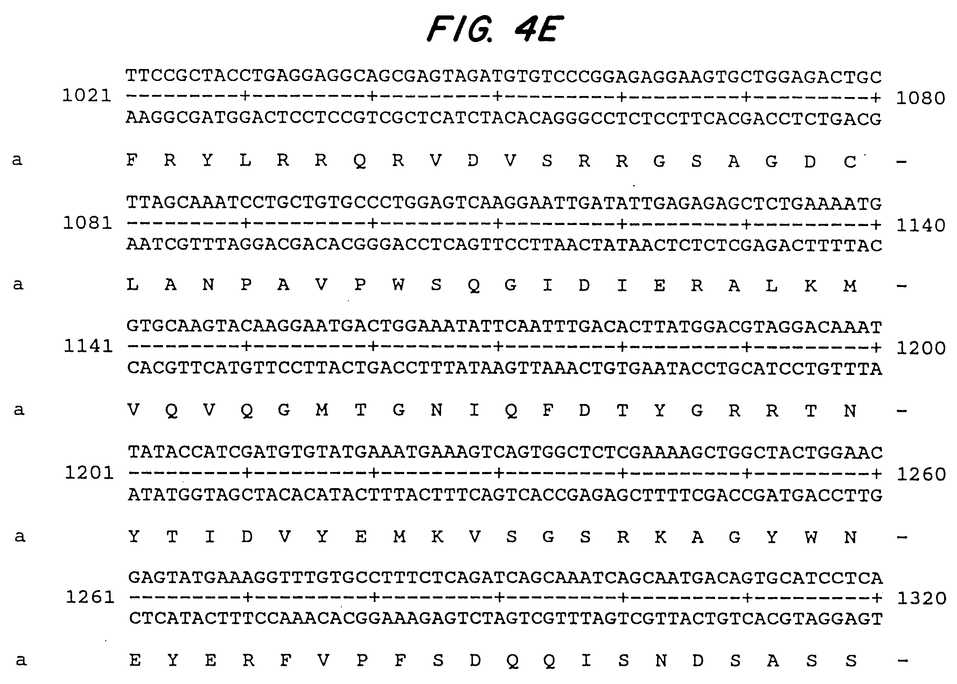

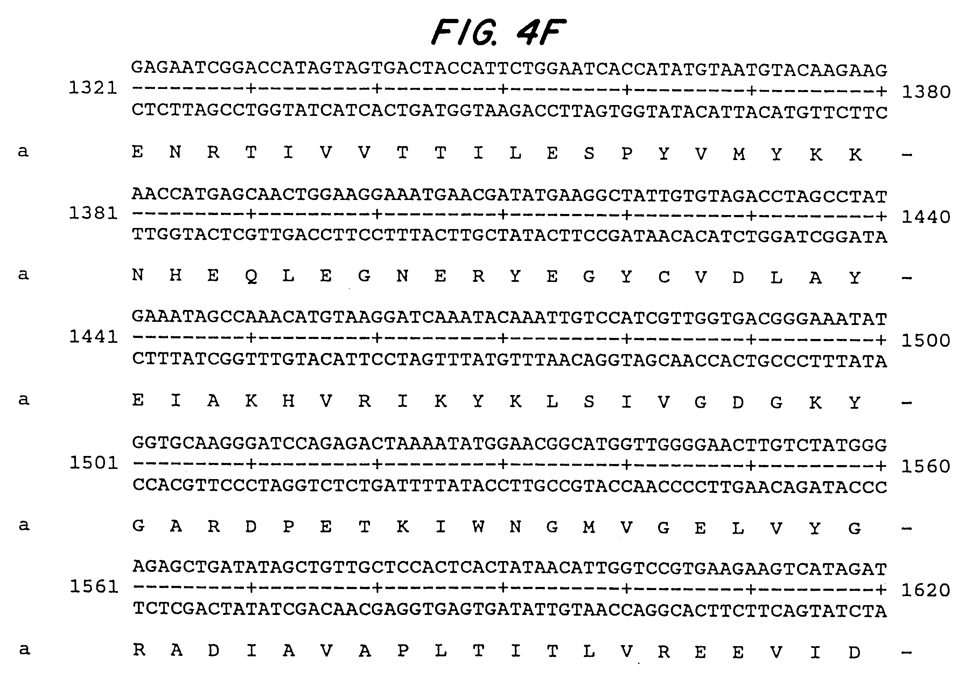

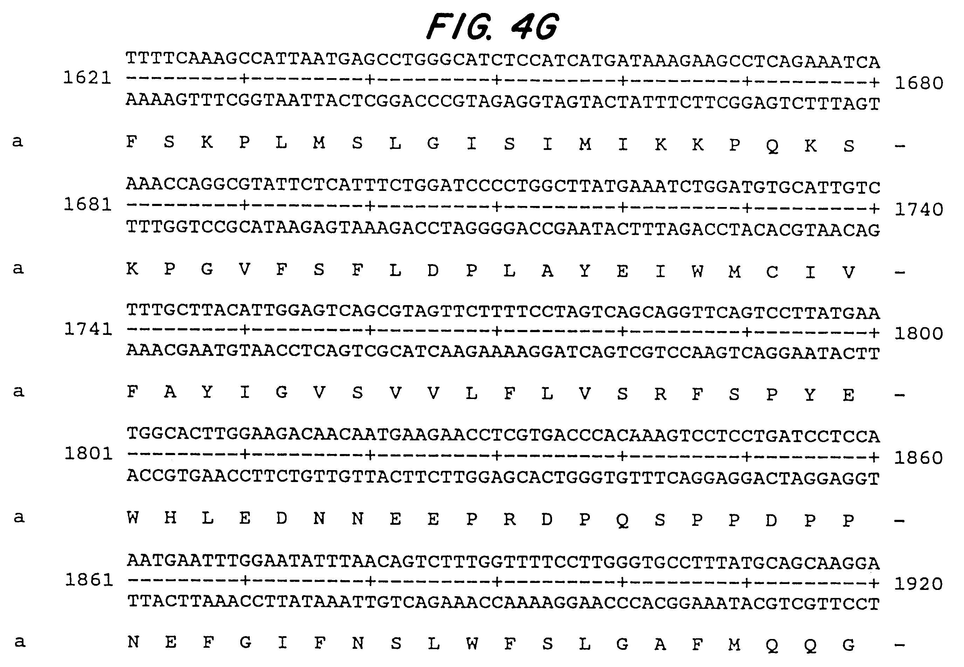

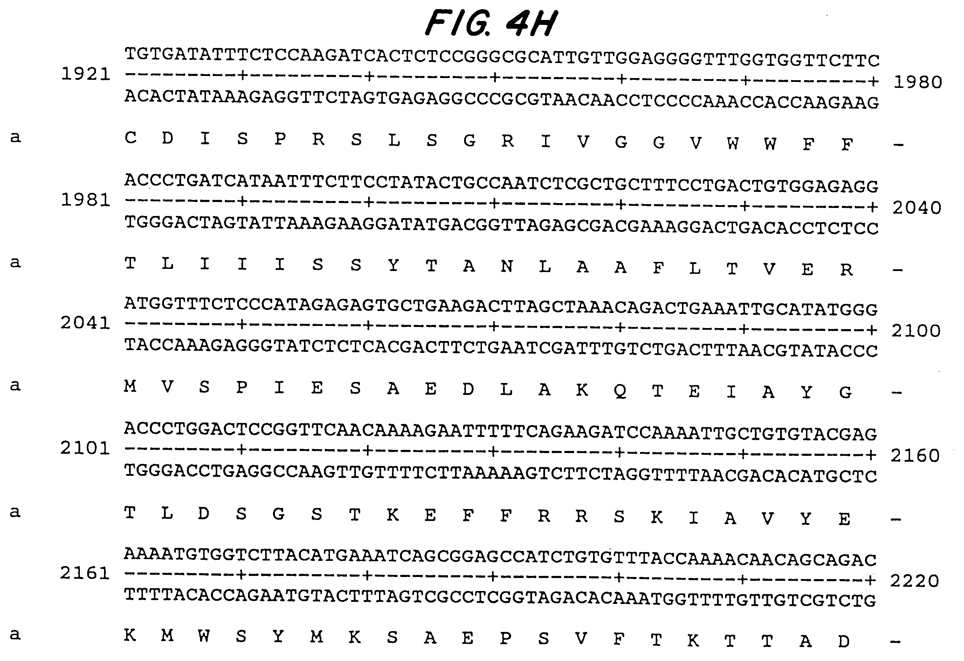

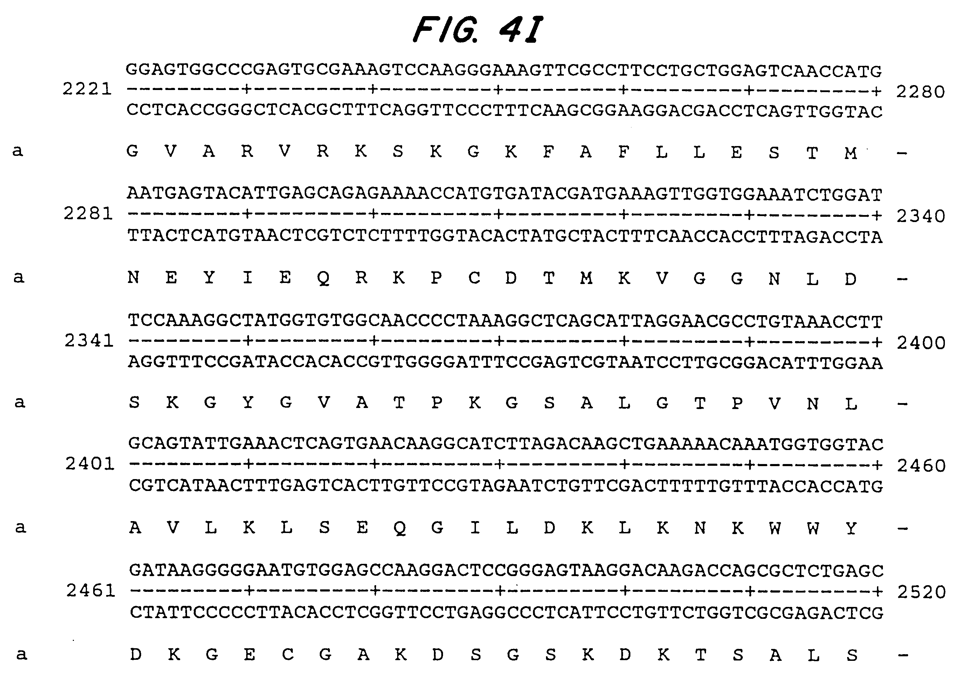

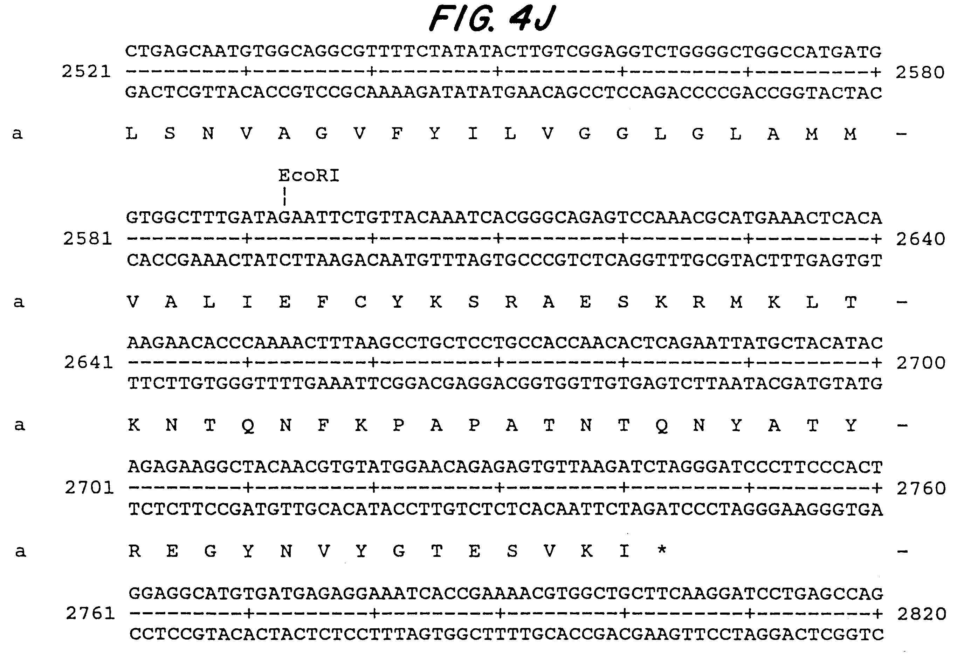

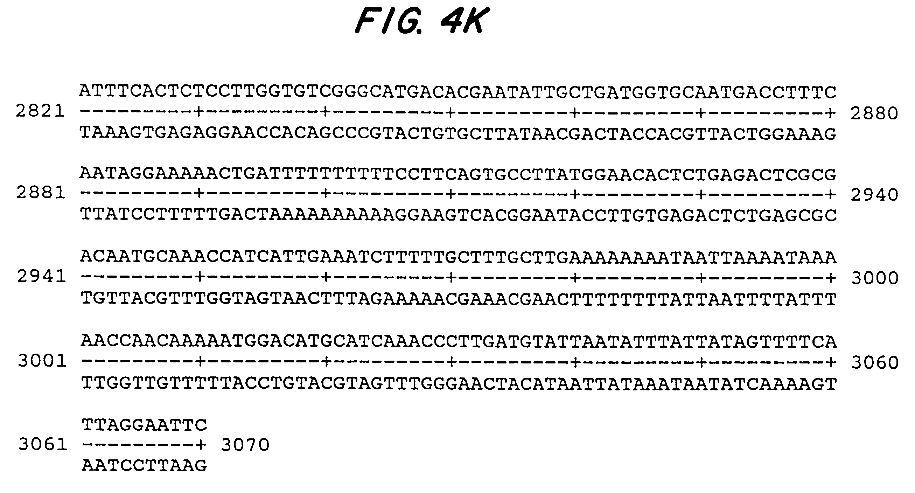

In one of its aspects, the present invention provides an isolated polynucleotide that codes for an EAA receptor belonging to the human GluR family. Alternatively, the polynucleotide may code for an AMPA-binding fragment of a human GluR receptor, or for an AMPA-binding variant of a human GluR receptor. According to specific embodiments of the present invention, the isolated polynucleotide encodes the human GluR1B receptor, the amino acid sequence of which is identified in Figure 1 (SEQ ID NO: 2), the human GluR2B receptor the amino acid sequence of which is identified in Figure 2 (SEQ ID NO: 4), and the human GluR3A receptor, the amino acid sequence of which is identified in Figure 3 (SEQ ID NO: 6). According to another embodiment of the invention, the polynucleotide encodes an AMPA-binding variant of the human GluR receptor. One such variant is identified herein as the human GluR3B receptor, the amino acid sequence of which is identified in Figure 4 (SEQ ID NO: 8). In various specific embodiments of the present invention, the polynucleotide consists of DNA e.g. cDNA, or of RNA e.g. messenger RNA. In other embodiments of the present invention, the polynucleotide may be coupled to a reporter molecule, such as a radioactive label, for use in autoradiographic studies of human GluR receptor tissue distribution. In further embodiments of the present invention, fragments of the polynucleotides of the invention, including radiolabelled versions thereof, may be employed either as probes for detection of glutamate receptor-encoding polynucleotides, as primers appropriate for amplifying such polynucleotides present in a biological specimen, or as templates for expression of a GluR receptor or AMPA-binding fragments or variants thereof.

According to another aspect of the present invention, there is provided a cellular host that produces an AMPA-type human glutamate receptor, and is characterized by the incorporation therein of a polynucleotide of the present invention. In embodiments of the present invention, the polynucleotide is a DNA molecule and is incorporated for expression and secretion in the cellular host, to yield, upon culturing, a functional, membrane-bound human GluR receptor. In other embodiments of the present invention, the polynucleotide is an RNA molecule which is introduced into the cellular host to yield a human GluR receptor as a functional, membrane-bound product of translation.

According to another aspect of the invention, there is provided a process for obtaining a substantially homogeneous source of a human EAA receptor useful for performing ligand binding assays, which comprises the steps of culturing a genetically engineered cellular host of the invention, and then recovering the cultured cells. Optionally, the cultured cells may be treated to obtain membrane preparations thereof, for use in the ligand binding assays.

According to another aspect of the present invention, there is provided a method for assaying interaction between a test ligand and a human EAA receptor, comprising the steps of incubating the test ligand under appropriate conditions with a human GluR receptor source, i.e., a cellular host of the invention or a membrane-preparation derived therefrom, and then determining the extent or result of binding between the substance and the receptor source.

These and other aspects of the invention are now described in greater detail with reference to the accompanying drawings, in which:

### Brief Description of the Drawings

Figure 1 provides a DNA sequence coding for the human GluR1B receptor, and the amino acid sequence thereof (SEQ ID NOS: 1 and 2);
Figure 2 provides a DNA sequence coding for the human GluR2B receptor, and the amino acid sequence thereof (SEQ ID NOS: 3 and 4);
Figure 3 provides a DNA sequence coding for the human GluR3A receptor, and the amino acid sequence thereof (SEQ ID NOS: 5 and 6);
Figure 4 provides a DNA sequence coding for the human GluR3B receptor, and the amino acid sequence thereof (SEQ ID NOS: 7 and 8);
Figure 5 provides the amino acid sequence of the human GluR3A receptor (SEQ ID NO: 9) and the human GluR3B receptor (SEQ ID NO: 10) in a region of dissimilarity;
Figure 6 depicts the strategy employed in cloning the human GluR3A receptor-encoding DNA illustrated in Figure 3;
Figure 7 depicts the strategy employed in cloning the human GluR3B receptor-encoding DNA illustrated in Figure 4;
Figure 8 depicts the strategy employed in generating recombinant DNA expression constructs incorporating the GluR3A receptor-encoding DNA;
Figure 9 depicts the strategy employed in generating recombinant DNA expression constructs incorporating the GluR1B receptor-encoding DNA (SEQ ID NOS: 11 and 12 are also shown in this figure);
Figure 10 depicts the strategy employed in cloning the human GluR2B receptor-encoding DNA illustrated in Figure 2;
Figure 11 depicts the strategy employed in generating recombinant DNA expression constructs incorporating the GluR2B receptor-encoding DNA;
Figure 12 illustrates the AMPA-binding property of the human GluR1B receptor;
Figure 13 illustrates the AMPA-binding property of the human GluR2B receptor;
Figure 14 illustrates the AMPA-binding property of the human GluR3A receptor;
Figures 15 & 16 illustrate a Scatchard analysis of human GluR1B and GluR2B receptor AMPA binding; and
Figure 17 graphically shows AMPA competition binding data for the GluR2B receptor.

### Detailed Description of the Preferred Embodiments

The invention relates to human CNS receptors of the AMPA-binding type, and is directed more particularly to novel receptors belonging to a family herein referred to as "GluR receptors", and provides isolated polynucleotides that code for such receptors. The term "isolated" is used herein with reference to intact polynucleotides that are generally less than about 4,000 nucleotides in length and which are otherwise isolated from DNA coding for other human proteins.

As used herein, the term "GluR receptors" is intended to embrace the human GluR1B, GluR2B and GluR3A receptors, AMPA-binding variants related thereto, as well as AMPA-binding fragments of the GluR1B, GluR2B and GluR3A receptors. Receptor variants within the scope of the present invention are functional variants of a parent receptor, i.e., one of GluR1B, GluR2B, GluR3A and GluR3B, which include conservative amino acid substitutions.

The term "AMPA-binding", as used herein with respect to receptors, and variants and fragments thereof, refers to a ligand binding profile which reveals glutamate binding and relative greater binding affinity for AMPA than for either glutamate, kainate or NMDA, as determined using assays of conventional design, such as the assays herein described.

In the present specification, an AMPA-binding receptor is said to be "functional" if a cellular host producing it exhibits *de novo* channel activity when exposed appropriately to AMPA, as determined by the established electrophysiological assays described for example by Hollmann et al., *supra,* or by any other assay appropriate for detecting conductance across a cell membrane.

Members of the human GluR family of the invention possess structural features characteristic of the EAA receptors in general, including extracellular N- and C-terminal regions, as well as four internal hydrophobic domains which serve to anchor the receptor within the cell surface membrane.

More specifically, the GluR1B receptor is a protein characterized structurally as a single polypeptide chain that is produced initially in precursor form bearing an 18 amino acid residue N-terminal signal peptide, and is transported to the cell surface in mature form, lacking the signal peptide and consisting of 888 amino acids arranged in the sequence illustrated, by single letter code, in Figure 1 (SEQ ID NOS: 1 and 2). Unless otherwise stated, the term human GluR receptor, either generally or with reference to a particular member of the receptor family, refers to the mature form of the receptor. Thus, the amino acid residues of these receptors are numbered in Figures 1-4 with reference to the mature protein sequence. With respect to structural domains of the GluR1B receptor, hydropathy analysis reveals four putative transmembrane domains, one spanning residues 521-540 inclusive (TM-1), another spanning residues 567-585 (TM-2), a third spanning residues 596-614 (TM-3) and the fourth spanning residues 788-808 (TM-4). Based on this assignment, it is likely that the human GluR1B receptor structure, in its natural membrane-bound form, consists of a 520 amino acid N-terminal extracellular domain, followed by a hydrophobic region containing four transmembrane domains and an extracellular, 80 amino acid C-terminal domain.

The GluR2B receptor, in precursor form bears a 21 amino acid residue N-terminal signal peptide, and in mature form, consists of 862 amino acids arranged in the sequence illustrated, by single letter code, in Figure 2 (SEQ ID NOS: 3 and 4). With respect to structural domains of the receptor, hydropathy analysis reveals four putative transmembrane domains, one spanning residues 525-544 inclusive (TM-1), another spanning residues 571-589 (TM-2), a third spanning residues 600-618 (TM-3) and the fourth spanning residues 792-812 (TM-4). Based on this assignment, it is likely that the human GluR2B receptor structure, in its natural membrane-bound form, consists of a 524 amino acid N-terminal extracellular domain, followed by a hydrophobic region containing four transmembrane domains and an extracellular, 50 amino acid C-terminal domain.

The GluR3A member of the human GluR family bears a 22 amino acid residue N-terminal signal peptide in precursor form, and is transported to the cell surface in mature form, lacking the signal peptide and consisting of 866 amino acids arranged in the sequence illustrated, by single letter code, in Figure 3 (SEQ ID NOS: 5 and 6). The four putative transmembrane domains of the GluR3A receptor are as follows: one spans residues 527-546 inclusive (TM-1), another spans residues 575-593 (TM-2), a third spans residues 604-622 (TM-3) and the fourth spans residues 796-816 (TM-4). Based on this assignment, it is likely that the human GluR3A receptor structure, in its natural membrane-bound form, consists of a 526 amino acid N-terminal extracellular domain, followed by a hydrophobic region containing four transmembrane domains and an extracellular, 50 amino acid C-terminal domain.

Structurally related variants of the GluR parent receptors identified above also exist. Specifically, a structurally related variant of the human GluR3A receptor, namely the GluR3B receptor, has also been identified. This variant occurs naturally in human brain tissue, and like GluR3A, the GluR3B receptor is 866 amino acids in length, as shown in Figure 4 (SEQ ID NOS: 7 and 8), in its mature, membrane-bound form. The GluR3B receptor initially bears a signal peptide identical to that borne on the GluR3A receptor. Four transmembrane domains are also apparent from the GluR3B sequence, and indicate that these domains lie in the same amino acid regions identified in connection with the GluR3A receptor.

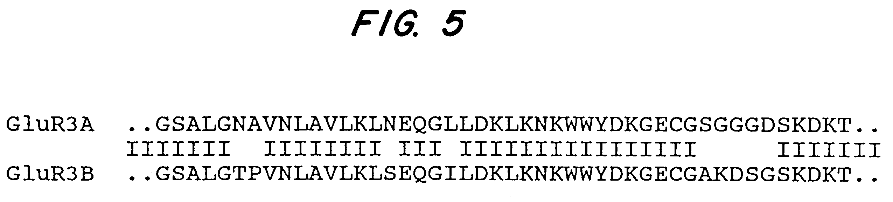

With respect to primary structure, the human GluR3B receptor differs from the GluR3A receptor in a 36 amino acid region separating transmembrane domains TM-3 and TM-4, i.e. residues 748-783. For comparison, the sequences of GluR3A and GluR3B in this region are compared in Figure 5 (SEQ ID NOS: 9 and 10).

Binding assays performed with various ligands, and with membrane preparations derived from mammalian cells engineered genetically to produce the human GluR receptors in membrane-bound form indicate that the human GluR receptors bind selectively to AMPA, relative particularly to kainate and NMDA. This feature, coupled with the medically significant connection between AMPA-type receptors and neurological disorders and disease indicate that the present receptors, as well as AMPA-binding fragments and variants thereof, will serve as valuable tools in the screening and discovery of ligands useful to modulate *in vivo* interactions between such receptors and their natural ligand, glutamate. Thus, a key aspect of the present invention resides in the construction of cells that are engineered genetically to produce a human GluR receptor, to serve as a ready and homogeneous source of receptor for use in *vitro* ligand binding and/or channel activation assays.

For use in the ligand binding assays, it is desirable to construct by application of genetic engineering techniques a host cell, either prokaryotic or eukaryotic, that produces a human GluR receptor as a heterologous and membrane-bound product. According to one embodiment of the invention, the construction of such engineered cells is achieved by introducing into a selected host cell a recombinant DNA construct in which DNA coding for a secretable form of the desired human GluR receptor, i.e., a form bearing its native signal peptide or a functional, heterologous equivalent thereof, is linked operably with expression controlling elements that are functional in the selected host to drive expression of the receptor-encoding DNA, and thus elaborate the desired human GluR receptor protein. Such cells are herein characterized as having the receptor-encoding DNA incorporated "expressibly" therein. The receptor-encoding DNA is referred to as "heterologous" with respect to the particular cellular host if such DNA is not naturally found in the particular host. The particular cell type selected to serve as host for production of the human GluR receptor can be any of several cell types currently available in the art, including both prokaryotic and eukaryotic cells, but should not of course be a cell type that in its natural state elaborates a surface receptor that can bind excitatory amino acids, and so confuse the assay results sought from the engineered cell line. Generally, such problems are avoided by selecting as host a non-neuronal cell type, and can further be avoided using non-human cell lines, as is conventional. It will be appreciated that neuronal- and human-type cells may nevertheless serve as expression hosts, provided that "background" binding to the test ligand is accounted for in the assay results.

According to one embodiment of the present invention, the cell line selected to serve as host for human GluR receptor production is a mammalian cell. Several types of such cell lines are currently available for genetic-engineering work, and these include the chinese hamster ovary (CHO) cells for example of K1 lineage (ATCC CCL 61) including the Pro5 variant (ATCC CRL 1281); the fibroblast-like cells derived from SV40-transformed African Green monkey kidney of the CV-1 lineage (ATCC CCL 70), of the COS-1 lineage (ATCC CRL 1650) and of the COS-7 lineage (ATCC CRL 1651); murine L-cells, murine 3T3 cells (ATCC CRL 1658), murine C127 cells, human embryonic kidney cells of the 293 lineage (ATCC CRL 1573), human carcinoma cells including those of the HeLa lineage (ATCC CCL 2), and neuroblastoma cells of the lines IMR-32 (ATCC CCL 127), SK-N-MC (ATCC HTB 10) and SK-N-SH (ATCC HTB 11).

A variety of gene expression systems have been adapted for use with these hosts and are now commercially available, and any one of these systems can be selected to drive expression of human GluR receptor-encoding DNA. These systems, available typically in the form of plasmidic vectors, incorporate expression cassettes the functional components of which include DNA constituting expression controlling sequences, which are host-recognized and enable expression of the receptor-encoding DNA when linked 5' thereof. The systems further incorporate DNA sequences which terminate expression when linked 3' of the receptor-encoding region. Thus, for expression in the selected mammalian cell host, there is generated a recombinant DNA expression construct in which DNA coding for a secretable form of the receptor is linked with expression controlling DNA sequences recognized by the host, and which include a region 5' of the receptor-encoding DNA to drive expression, and a 3' region to terminate expression. The plasmidic vector harboring the recombinant DNA expression construct typically incorporates such other functional components as an origin of replication, usually virally-derived, to permit replication of the plasmid in the expression host and desirably also for plasmid amplification in a bacterial host, such as E.coli. To provide a marker-enabling selection of stably transformed recombinant cells, the vector will also incorporate a gene conferring some survival advantage on the transformants, such as a gene coding for neomycin resistance in which case the transformants are plated in medium supplemented with neomycin.

Included among the various recombinant DNA expression systems that can be used to achieve mammalian cell expression of the receptor-encoding DNA are those that exploit promoters of viruses that infect mammalian cells, such as the promoter from the cytomegalovirus (CMV), the Rous sarcoma virus (RSV), simian virus (SV40), murine mammary tumor virus (MMTV) and others. Also useful to drive expression are promoters such as the LTR of retroviruses, insect cell promoters such as those regulated by temperature, and isolated from Drosophila, as well as mammalian gene promoters such as those regulated by heavy metals i.e.the metalothionein gene promoter, and other steroid-inducible promoters.

For incorporation into the recombinant DNA expression vector, DNA coding for a selected human GluR receptor, e.g. one of the human GluR1B, GluR2B or GluR3A receptors, or an AMPA-binding fragment or variant thereof, e.g. GluR3B, can be obtained by applying selected techniques of gene isolation or gene synthesis. As described in more detail in the examples herein, human GluR receptors are encoded within the genome of human brain tissue, and can therefore be obtained from human DNA libraries by careful application of conventional gene isolation and cloning techniques. This typically will entail extraction of total messenger RNA from a fresh source of human brain tissue, preferably cerebellum or hippocampus tissue, followed by conversion of message to cDNA and formation of a library in for example a bacterial plasmid, more typically a bacteriophage. Such bacteriophage harboring fragments of the human DNA are typically grown by plating on a lawn of susceptible E. coli bacteria, such that individual phage plaques or colonies can be isolated. The DNA carried by the phage colony is then typically immobilized on a nitrocellulose or nylon-based hybridization membrane, and then hybridized, under carefully controlled conditions, to a radioactively (or otherwise) labelled oligonucleotide probe of appropriate sequence to identify the particular phage colony carrying receptor-encoding DNA or fragment thereof. It will be understood, for example, that selective hybridization, i.e. hybridization of a DNA sequence that is completely complementary to the probe, will be conducted under stringent hybridization conditions. Typically, the gene or a portion thereof so identified is subcloned into a plasmidic vector for nucleic acid sequence analysis.

In specific embodiments of the invention, the GluR1B receptor is encoded by the DNA sequence illustrated in Figure 1 (SEQ ID NO: 1), the GluR2B receptor is encoded by the DNA sequence illustrated in Figure 2 (SEQ ID NO: 3) and the GluR3A and GluR3B receptors are encoded by the DNA sequences illustrated respectively in Figures 3 (SEQ ID NO: 5) and 4 (SEQ ID NO: 7). Alternatively, codons within the illustrated DNA sequences coding for the GluR receptors may be replaced by synonymous codon equivalents, such synonymous codon replacements being well-known in the art.

The illustrated DNA sequences constitute cDNA sequences identified in human brain cDNA libraries in the manner exemplified herein. Having herein provided the nucleotide sequence of various members of the human GluR receptor family, however, it will be appreciated that polynucleotides encoding the receptors can be obtained by other routes. Automated techniques of gene synthesis and/or amplification can be performed to generate DNA coding therefor. Because of the length of the human GluR receptor-encoding DNA, application of automated synthesis may require staged gene construction, in which regions of the gene up to about 300 nucleotides in length are synthesized individually and then ligated in correct succession by overhang complementarity for final assembly. Individually synthesized gene regions can be-amplified prior to assembly, using established polymerase chain reaction (PCR) technology.

By the application of automated gene synthesis techniques, there is provided a means to generate polynucleotides that encode variants of naturally occurring human GluR receptors, i.e. GluR1B, GluR2B, GluR3A and GluR3B. It will be appreciated, for example, that polynucleotides coding for the human GluR receptors herein described can be generated by substituting synonymous codons for those represented in the naturally occurring polynucleotide sequences herein identified. In addition, polynucleotides coding for human GluR receptor variants can be generated which for example incorporate one or more e.g. 1-10, single amino acid substitutions, deletions or additions. Since it will for the most part be desirable to retain the natural ligand binding profile of the receptor for screening purposes, it is desirable to limit amino acid substitutions, for example to the so-called conservative replacements in which amino acids of like charge are substituted, and to limit substitutions to those sites less critical for receptor activity e.g. within about the first 20 N-terminal residues of the mature receptor, and such other regions as are elucidated upon receptor domain mapping.

With appropriate template DNA in hand, the technique of PCR amplification may also be used to directly generate all or part of the final gene. In this case, primers are synthesized which will prime the PCR amplification of the final product, either in one piece, or in several pieces that may be ligated together. This may be via step-wise ligation of blunt ended, amplified DNA fragments, or preferentially via step-wise ligation of fragments containing naturally occurring restriction endonuclease sites. In this application, it is possible to use either cDNA or genomic DNA as the template for the PCR amplification. In the former case, the cDNA template can be obtained from commercially available or self-constructed cDNA libraries of various human brain tissues, including hippocampus and cerebellum.

Once obtained, the receptor-encoding DNA is incorporated for expression into any suitable expression vector, and host cells are transfected therewith using conventional procedures, such as DNA-mediated transformation, electroporation, or particle gun transformation. Expression vectors may be selected to provide transformed cell lines that express the receptor-encoding DNA either transiently or in a stable manner. For transient expression, host cells are typically transformed with an expression vector harboring an origin of replication functional in a mammalian cell. For stable expression, such replication origins are unnecessary, but the vectors will typically harbour a gene coding for a product that confers on the transformants a survival advantage, to enable their selection. Genes coding for such selectable markers include the E. coli *gpt* gene which confers resistance to mycophenolic acid, the *neo* gene from transposon Tn5 which confers resistance to the antibiotic G418 and to neomycin, the *dhfr* sequence from murine cells or E. coli which changes the phenotype of DHFR- cells into DHFR+ cells, and the *tk* gene of herpes simplex virus, which makes TK- cells phenotypically TK+ cells. Both transient expression and stable expression can provide transformed cell lines, and membrane preparations derived therefrom, for use in ligand screening assays.

For use in screening assays, cells transiently expressing the receptor-encoding DNA can be stored frozen for later use, but because the rapid rate of plasmid replication will lead ultimately to cell death, usually in a few days, the transformed cells should be used as soon as possible. Such assays may be performed either with intact cells, or with membrane preparations derived from such cells. The membrane preparations typically provide a more convenient substrate for the ligand binding experiments, and are therefore preferred as binding substrates. To prepare membrane preparations for screening purposes, i.e., ligand binding experiments, frozen intact cells are homogenized while in cold water suspension and a membrane pellet is collected after centrifugation. The pellet is then washed in cold water, and dialyzed to remove endogenous EAA ligands such as glutamate, that would otherwise compete for binding in the assays. The dialyzed membranes may then be used as such, or after storage in lyophilized form, in the ligand binding assays. Alternatively, intact, fresh cells harvested about two days after transient transfection or after about the same period following fresh plating of stably transfected cells, can be used for ligand binding assays by the same methods as used for membrane preparations. When cells are used, the cells must be harvested by more gentle centrifugation so as not to damage them, and all washing must be done in a buffered medium, for example in phosphate-buffered saline, to avoid osmotic shock and rupture of the cells.

The binding of a substance, i.e., a candidate ligand, to a human GluR receptor of the invention is evaluated typically using a predetermined amount of cell-derived membrane (measured for example by protein determination), generally from about 25µg to 100µg. Generally, competitive binding assays will be useful to evaluate the affinity of a test compound relative to AMPA. This competitive binding assay can be performed by incubating the membrane preparation with radiolabelled AMPA, for example [³H]-AMPA, in the presence of unlabelled test compound added at varying concentrations. Following incubation, either displaced or bound radiolabelled AMPA can be recovered and measured, to determine the relative binding affinities of the test compound and AMPA for the particular receptor used as substrate. In this way, the affinities of various compounds for the AMPA-binding human EAA receptors can be measured. Alternatively, a radiolabelled analogue of glutamate may be employed in place of radiolabelled AMPA, as competing ligand.

As an alternative to using cells that express receptor-encoding DNA, ligand characterization may also be performed using cells for example Xenopus oocytes, that yield functional membrane-bound receptor following introduction by injection either of receptor-encoding messenger RNA into the oocyte cytoplasm, or of receptor-encoding DNA into the oocyte nucleus. To generate the messenger RNA of cytoplasmic delivery, the receptor-encoding DNA is typically subcloned first into a plasmidic vector adjacent a suitable promoter region, such as the T3 or T7 bacteriophage promoters, to enable transcription into RNA message. RNA is then transcribed from the inserted gene *in vitro,* collected and then injected into Xenopus oocytes. Following the injection of nL volumes of an RNA solution, the oocytes are left to incubate for up to several days, and are then tested for the ability to respond to a particular ligand molecule supplied in a bathing solution. Since functional EAA receptors act in part by operating a membrane channel through which ions may selectively pass, the functioning of the receptor in response to a particular ligand molecule in the bathing solution may typically be measured as an electrical current utilizing microelectrodes inserted into the cell, in the established manner.

In addition to using the receptor-encoding DNA to construct cell lines useful for ligand screening, expression of the DNA can, according to another aspect of the invention, be performed to produce AMPA-binding fragments of the receptor in soluble form, for structure investigation, to raise antibodies and for other experimental uses. It is expected that the portion of the human GluR receptor responsible for AMPA-binding resides on the outside of the cell, i.e., is extracellular. It is therefore desirable in the first instance to facilitate the characterization of the receptor-ligand interaction by providing this extracellular ligand-binding domain in quantity and in isolated form, i.e., free from the remainder of the receptor. To accomplish this, the full-length human GluR receptor-encoding DNA may be modified by site-directed mutagenesis, so as to introduce a translational stop codon into the extracellular N-terminal region, immediately before the sequence encoding the first transmembrane domain (TM1), i.e., before residue 521 of GluR1B, before residue 525 in GluR2B, or before residue 527 of GluR3A and GluR3B. Since there will no longer be produced any transmembrane domain(s) to "anchor" the receptor into the membrane, expression of the modified gene will result in the secretion, in soluble form, of only the extracellular ligand-binding domain. Standard ligand-binding assays may then be performed to ascertain the degree of binding of a candidate compound to the extracellular domain so produced. It may of course be necessary, using site-directed mutagenesis, to produce several different versions of the extracellular regions, in order to optimize the degree of ligand binding to the isolated domains.

For use in ligand binding assays according to the present invention, AMPA-binding fragments of the receptor will first be anchored to a solid support using any one of various techniques. In one method, the C-terminal end of the receptor peptide fragment may be coupled to a derivatized, insoluble polymeric support, for example, cross-linked polystyrene or polyamide resin. Once anchored to the solid support, the frament is useful to screen candidate ligands for receptor binding affinity. For this purpose, competition-type ligand-binding assays, as described above using full-length receptor, are commonly used. Fragments secured to a solid support are bound with a natural ligand, i.e. AMPA, in the presence of a candidate ligand. One of AMPA or candidate ligand is labelled, for example radioactively, and following a suitable incubation period, the degree of AMPA displacement is determined by measuring the amount of bound or unbound label.

Alternatively, it may be desirable to produce an extracellular domain of the receptor which is not derived from the amino-terminus of the mature protein, but rather from the carboxy-terminus instead, for example domains immediately following the fourth transmembrane domain (TM4), i.e., residing between amino acid residues 809-888 of GluR1B, residues 813-862 of GluR2B, or residues 817-866 of GluR3A or GluR3B. In this case, site-directed mutagenesis and/or PCR-based amplification techniques may readily be used to provide a defined fragment of the gene encoding the receptor domain of interest. Such a DNA sequence may be used to direct the expression of the desired receptor fragment, either intracellularly, or in secreted fashion, provided that the DNA encoding the gene fragment is inserted adjacent to a translation start codon provided by the expression vector, and that the required translation reading frame is carefully conserved.

It will be appreciated that the production of such AMPA-binding fragments of a GluR receptor may be accomplished in a variety of host cells. Mammalian cells such as CHO cells may be used for this purpose, the expression typically being driven by an expression promoter capable of high-level expression, for example the CMV (cytomegalovirus) promoter. Alternately, non-mammalian cells, such as insect Sf9 (Spodoptera frugiperda) cells may be used, with the expression typically being driven by expression promoters of the baculovirus, for example the strong, late polyhedrin protein promoter. Filamentous fungal expression systems may also be used to secrete large quantities of such extracellular domains of the EAA receptor. Aspergillus nidulans, for example, with the expression being driven by the alcA promoter, would constitute such an acceptable system. In addition to such expression hosts, it will be further appreciated that any prokaryotic or other eukaryotic expression system capable of expressing heterologous genes or gene fragments, whether intracellularly or extracellularly would be similarly acceptable.

For use particularly in detecting the presence and/or location of a human GluR receptor, for example in brain tissue, the present invention also provides, in another of its aspects, labelled antibody to a human GluR receptor. To raise such antibodies, there may be used as immunogen either the intact, soluble receptor or an immunogenic fragment thereof i.e. a fragment capable of eliciting an immune response, produced in a microbial or mammalian cell host as described above or by standard peptide synthesis techniques. Regions of human GluR receptor particularly suitable for use as immunogenic fragments include those corresponding in sequence to an extracellular region of the receptor, or a portion of the extracellular region. For example, peptides consisting of residues 1-526 of the GluR3A receptor or a fragment thereof comprising at least about 10 residues, including particularly fragments containing residues 178-193 or 479-522; and peptides corresponding to the region between transmembrane domains TM-2 and TM-3 of the GluR3A receptor, such as a peptide consisting of residues 594-603. Peptides consisting of the C-terminal domain (residues 817-866 of the GluR3A receptor), or fragment thereof, may also be used for the raising of antibodies.

The raising of antibodies to the selected human GluR receptor or immunogenic fragment can be achieved, for polyclonal antibody production, using immunization protocols of conventional design, and any of a variety of mammalian hosts, such as sheep, goats and rabbits. Alternatively, for monoclonal antibody production, immunocytes such as splenocytes can be recovered from the immunized animal and fused, using hybridoma technology, to a myeloma cells. The fusion products are then screened by culturing in a selection medium, and cells producing antibody are recovered for continuous growth, and antibody recovery. Recovered antibody can then be coupled covalently to a detectable label, such as a radiolabel, enzyme label, luminescent label or the like, using linker technology established for this purpose.

In detectably labelled form, e.g. radiolabelled form, DNA or RNA coding for a human GluR receptor, and selected regions thereof, may also be used, in accordance with another aspect of the present invention, as hybridization probes for example to identify sequence-related genes resident in the human or other mammalian genomes (or cDNA libraries) or to locate the human GluR-encoding DNA in a specimen, such as brain tissue. This can be done using either the intact coding region, or a fragment thereof having radiolabelled e.g. ³²P, nucleotides incorporated therein. To identify the human GluR-encoding DNA in a specimen, it is desirable to use either the full length cDNA coding therefor, or a fragment which is unique thereto. With reference to Figures 1-4 (SEQ ID NOS: 1-8), such nucleotide fragments include those comprising at least about 17 nucleic acids, and otherwise corresponding in sequence to a region coding for an extracellular N-terminal or C-terminal region of the receptor, or representing a 5'-untranslated or 3'-untranslated region thereof. Such oligonucleotide sequences, and the intact gene itself, may also be used of course to clone human GluR-related human genes, particularly cDNA equivalents thereof, by standard hybridization techniques.

Embodiments of the present invention are described in detail in the following specific examples which are not to be construed as limiting:

### Example 1 - Isolation of DNA coding for the human GluR3A receptor

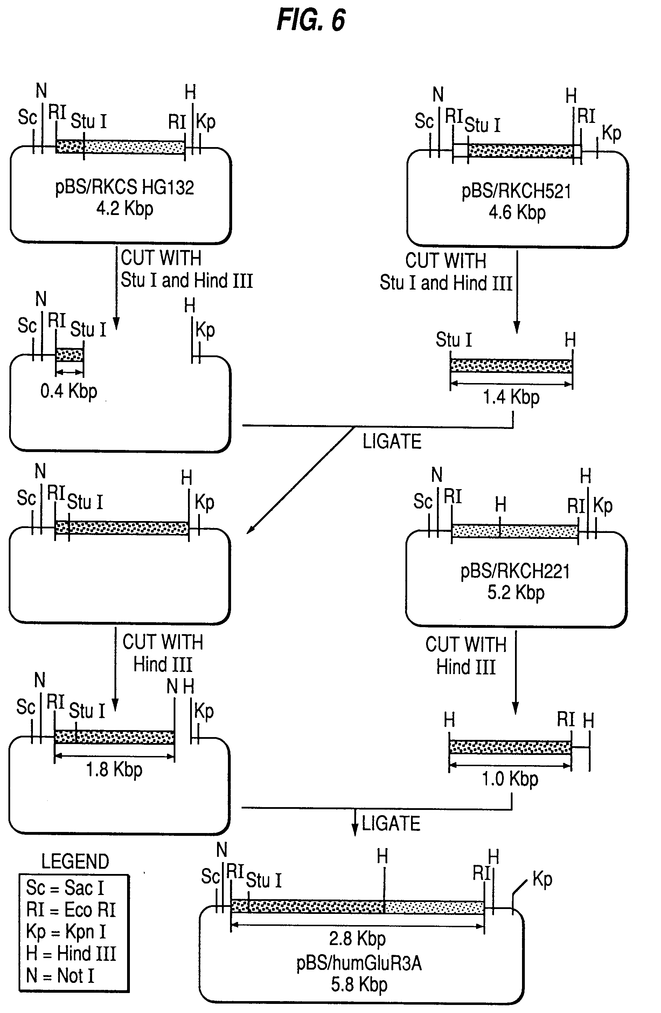

The particular strategy used to clone the human GluR3A receptor is depicted schematically in Figure 6, and described in greater detail below.

cDNA coding for the human GluR3A receptor was identified by probing human hippocampal cDNA that was obtained as an EcoRI-based lambda phage library (lambda ZAP) from Stratagene Cloning Systems (La Jolla, California, U.S.A.). The cDNA library was probed initially with a 1.1kb EcoRI/EcoRI DNA fragment constituting the 3' region of a kainate-binding human EAA receptor, designated humEAAla. This particular kainate-binding receptor is described in EP-A-0 529 994 incorporated herein by reference. DNA coding for the human EAA1a receptor, and from which the 1.1kb probe may be recovered, was deposited under terms of the Budapest Treaty, with the American Type Culture Collection in Rockville, Maryland U.S.A. on August 21, 1991 under accession number ATCC 75063.

Hybridizations using the probe were carried out at 30C overnight, and filters were washed with 2xSSC containing 0.5% SDS at 25C for 5 minutes, followed by a 15 minute wash at 50C with 2xSSC containing 0.5% SDS. The final wash was with 1xSSC containing 0.5% SDS at 50C for 15 minutes. Filters were exposed to X-ray film (Kodak) overnight. Of 10⁶ clones screened under the following hybridization conditions (6xSSC, 50% formamide, 5% Denhardt's solution, 0.5% SDS, 100ug/ml denatured salmon sperm DNA), only two hippocampal cDNA library inserts were identified, one about 1.6kb and designated RKCH521 and another about 2.2kb and designated RKCH221 (Fig.6). For sequencing, the '521 and the '221 phages were plaque purified, then excised as phagemids according to the supplier's specifications, to generate insert-carrying Bluescript-SK variants of the phagemid vector. Sequencing of the '221 clone across its entire sequence revealed a putative ATG initiation codon together with about 78 bases of 5'non-coding region and about 2.1 kb of coding region. Sequencing across the '521 insert revealed a significant region of overlap with the '221 insert, and provided some additional 3' sequence, although no termination codon was located.

There being no termination codon apparent in the '521 sequence, a 3' region of the gene was sought. For this purpose, there was first synthesized an oligonucleotide probe capable of annealing to the 3' region of the rat GluR3 receptor sequence reported by Keinanen et al, supra. The specific sequence of the 32-P-labelled probe is provided below (SEQ ID NO: 13):

5'-ACACTCAGAATTACGCTACATACAGAGAAGGCTACAACGT-3'

The same hippocampal cDNA library was then re-screened using the rat-based probe and under the following hybridization conditions; 6xSSC, 25% formamide, 5% Dernhardt's solution, 0.5% SDS, 100ug/ml denatured salmon sperm DNA, 42C. This revealed a 1.2kb insert, designated RKCSHG132. Sequencing of the entire insert revealed 5' overlap with the 3'end of the previously isolated '521 insert, and also revealed a termination codon as well as about 15 bases of 3'non-translated sequence.

To provide the entire coding region in an intact clone, the strategy shown in Figure 6 was employed, to generate the phagemid pBS/HumGluR3A which carries the hGluR3A-encoding DNA as a 2.8kb EcoRI/EcoRI insert in a 3.0kb Bluescript-SK phagemid background. The entire sequence of the EcoRI/EcoRI insert is provided in Figure 3 (SEQ ID NOS: 5 and 6).

The 5.8kb phagemid pBS/humGluR3A was deposited, under the terms of the Budapest Treaty, with the American Type Culture Collection in Rockville, Maryland USA on March 19, 1992, and has been assigned accession number ATCC 75218.

### Example 2 - Isolation of DNA coding for human GluR3B receptor

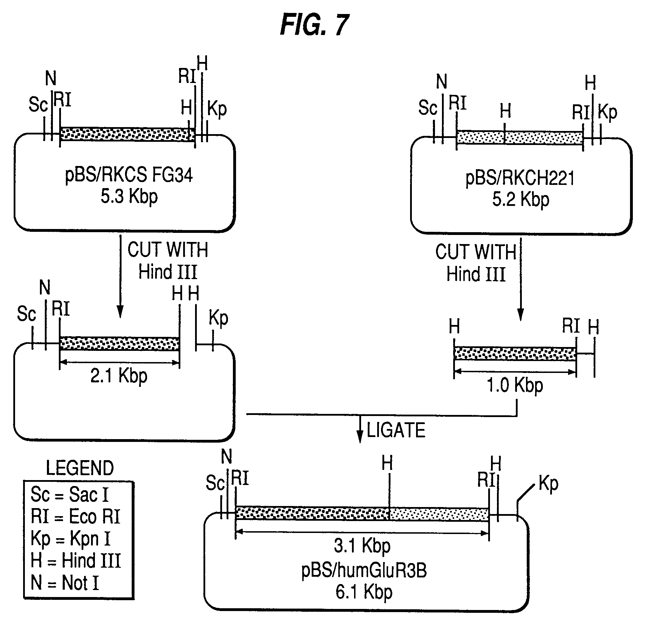

A human fetal brain cDNA library was also screened in the search for human GluR receptors. This-particular library was obtained as an EcoRI-based lambda gt10 library from Strategene Cloning Systems (La Jolla, California, U.S.A.). The library was first screened using as hybridization probe an oligonucleotide capable of hybridizing to a 3' region of the reported rat GluR3 gene sequence. Screening using hybridization conditions as noted above (6xSSC, 25% formamide, 42C, etc.) revealed one insert about 2.3kb in size, designated RKCSFG34. After excision to release Bluescript-SK phagemids carrying the insert, sequencing revealed substantial sequence identity between the '34 insert and the 3'end of the earlier isolated GluR3A clone, and suggested that the 5'end of the gene encoded on partially on the '34 insert was missing. To provide an assembled gene, a 5' region was excised from the GluR3A insert and used to generate the 5'end of the '34 insert, at an internal HindIII site. This was achieved as depicted schematically in Figure 7. The resulting intact clone was designated human GluR3B.

Sequence comparison between the GluR3A clone of Example 1 and the GluR3B clone of this Example revealed only a short region of dissimilarity which is illustrated, in terms of amino acid sequence, in Figure 5 (SEQ ID NOS: 9 and 10).

The 6.1kb phagemid pBS/humGluR3B was deposited, under the terms of the Budapest Treaty, with the American Type Culture Collection in Rockville, Maryland USA on March 19, 1992, and has been assigned accession number ATCC 75219.

### Example 3 - Isolation of DNA coding for the human GluR1B receptor

cDNA coding for the human GluR1B receptor was identified by probing human fetal brain cDNA that was obtained as an EcoRI-based lambda phage library (lambda ZAP) from Stratagene Cloning Systems (La Jolla, California, U.S.A.). The cDNA library was screened using an oligonucleotide probe capable of annealing to the 5' region of the rat GluR1 receptor sequence reported by Hollmann et al, supra. The specific sequence of the 32-P-labelled probe is provided below (SEQ ID NO: 14):

5'-CCAGATCGATATTGTGAACATCAGCGACACGTTTGAGATG-3'

The fetal brain cDNA library was screened under the following hybridization conditions; 6xSSC, 25% formamide, 5% Dernhardt's solution, 0.5% SDS, 100ug/ml denatured salmon sperm DNA, 42C. Filters were washed with 2xSSC containing 0.5% SDS at 25C for 5 minutes, followed by a 15 minute wash at 50C with 2xSSC containing 0.5% SDS. The final wash was with 1xSSC containing 0.5% SDS at 50C for 15 minutes. Filters were exposed to X-ray film (Kodak) overnight. Of 10⁶ clones screened, only one cDNA insert, of about 3.2kb, was identified, and designated RKCSFG91. For sequencing, the '91 phage was plaque purified, then excised as a phagemid according to the supplier's specifications, to generate an insert-carrying Bluescript-SK variant of the phagemid vector. Sequencing of the '91 clone across its entire sequence revealed a putative ATG initiation codon together with about 61 bases of 5'non-coding region and 2,718 bases of coding region. Also revealed was a termination codon, as well as about 438 bases of 3' non-translated sequence. The entire sequence of the EcoRI/EcoRI insert is provided in Figure 1 (SEQ ID NOS: 1 and 2).

A 6.2kb phagemid designated pBS/humGluR1B, carrying the receptor-encoding DNA as a 3.2kb EcoRI/EcoRI insert in a 3.0kb Bluescript-SK phagemid background, was deposited, under the terms of the Budapest Treaty, with the American Type Culture Collection in Rockville, Maryland USA on May 28, 1992, and has been assigned accession number ATCC 75246.

### Example 4 - Isolation of DNA coding for the human GluR2B receptor

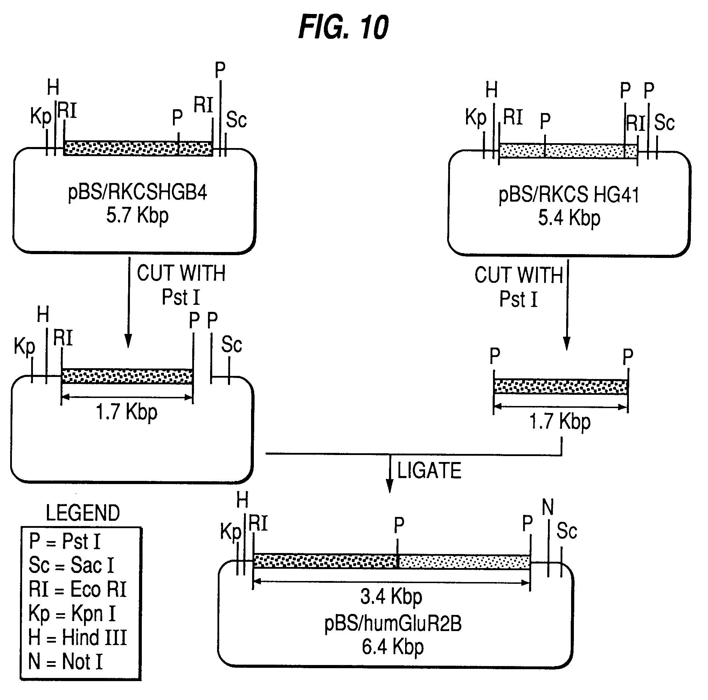

The particular strategy used to clone the human Glu2B receptor is depicted schematically in Figure 10, and described in greater detail below.

cDNA coding for the human GluR2B receptor was identified by probing human hippocampal cDNA that was obtained as an EcoRI-based lambda phage library (lambda ZAP) from Stratagene Cloning Systems (La Jolla, California, U.S.A.). The cDNA library was screened using an oligonucleotide probe capable of annealing to the 3' region of the rat GluR2 receptor sequence reported by Keinanen et al, supra. The specific sequence of the 32-P-labelled probe is provided below (SEQ ID NO: 15):

5'-GTGAATGTGGAGCCAAGGACTCGGGAAGTAAG-3'

The hippocampal cDNA library was screened under the following hybridization conditions; 6xSSC, 25% formamide, 5% Dernhardt's solution, 0.5% SDS, 100ug/ml denatured salmon sperm DNA, 42C. Filters were washed with 2xSSC containing 0.5% SDS at 25C for 5 minutes, followed by a 15 minute wash at 50C with 2xSSC containing 0.5% SDS. The final wash was with 1xSSC containing 0.5% SDS at 50C for 15 minutes. Filters were exposed to X-ray film (Kodak) overnight. Of 10⁶ clones screened, only two cDNA inserts were identified, one about 2.7kb and designated RKCSHG84 and another about 2.9kb and designated RKCSHG41 (Fig.10). For sequencing, the '84 and the '41 phages were plaque purified, then excised as phagemids according to the supplier's specifications, to generate insert-carrying Bluescript-SK variants of the phagemid vector. Sequencing of the '84 clone across its entire sequence revealed a putative ATG initiation codon together with about 314 bases of 5'non-coding region and about 2.4 kb of coding region. Sequencing across the '41 insert revealed a significant region of overlap with the '84 insert, and also revealed a termination codon not found in the '84 insert as well as about 441 bases of 3' non-translated sequence.

To provide the entire coding region in an intact clone, the strategy shown in Figure 10 was employed, to generate the phagemid pBS/HumGluR2B which carries the hGluR2B-encoding DNA as a 3.4kb EcoRI/PstI insert in a 3.Okb Bluescript-SK phagemid background. The entire sequence of the EcoRI/PstI insert is provided in Figure 2 (SEQ ID NOS: 3 and 4).

The 6.4kb phagemid pBS/humGluR2B was deposited, under the terms of the Budapest Treaty, with the American Type Culture Collection in Rockville, Maryland USA on March 19, 1992, and has been assigned accession number ATCC 75217.

### Example 5 - Construction of genetically engineered cells producing human GluR3A receptors

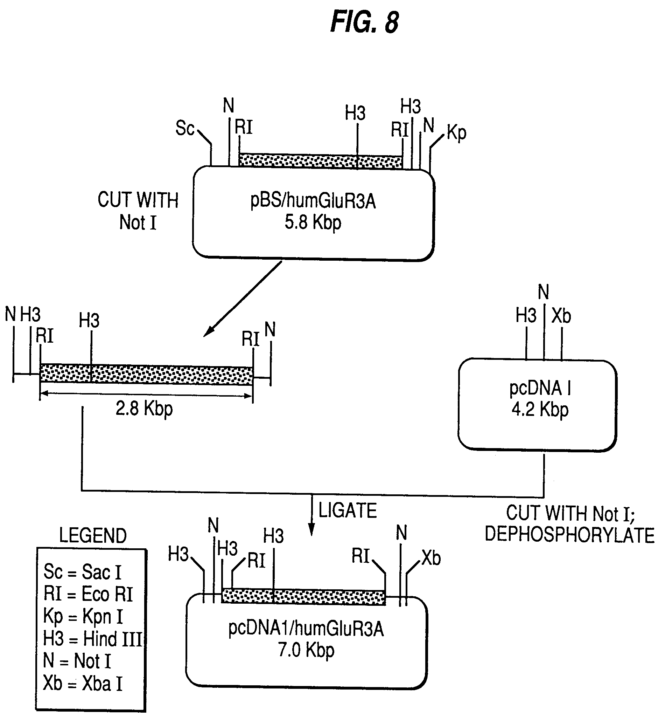

The strategy depicted in Figure 8 was employed to facilitate incorporation of the GluR3A receptor-encoding cDNA into an expression vector.

For transient expression in mammalian cells, cDNA coding for the human GluR3A receptor was incorporated into the mammalian expression vector pcDNAI, which is available commercially from Invitrogen Corporation (San Diego, California, USA; catalogue number V490-20). This is a multifunctional 4.2kb plasmid vector designed for cDNA expression in eukaryotic systems, and cDNA analysis in prokaryotes. Incorporated on the vector are the CMV promoter and enhancer, splice segment and polyadenylation signal, an SV40 and Polyoma virus origin of replication, and M13 origin to rescue single strand DNA for sequencing and mutagenesis, Sp6 and T7 RNA promoters for the production of sense and anti-sense RNA transcripts and a Col E1-like high copy plasmid origin. A polylinker is located appropriately downstream of the CMV promoter (and 3' of the T7 promoter).

To facilitate incorporation of the GluR3A receptor-encoding cDNA into an expression vector, a NotI site was introduced onto the 5' flank of the Bluescript-SK cDNA insert, and the cDNA insert was then released from pBS/humGluR3A as a 2.8 kb NotI/NotI fragment, which was then incorporated at the NotI site in the pcDNAI polylinker. Sequencing across the NotI junction was performed, to confirm proper insert orientation in pcDNAI. The resulting plasmid, designated pcDNAI/humGluR3A, was then introduced for transient expression into a selected mammalian cell host, in this case the monkey-derived, fibroblast like cells of the COS-1 lineage (available from the American Type Culture Collection, Rockville, Maryland as ATCC CRL 1650).

For transient expression of the GluR3A-encoding DNA, COS-1 cells were transfected with approximately 8ug DNA (as pcDNA1/humGluR3A) per 10⁶ COS cells, by DEAE-mediated DNA transfection and treated with chloroquine according to the procedures described by Maniatis et al, supra. Briefly, COS-1 cells were plated at a density of 5 x 10⁶ cells/dish and then grown for 24 hours in FBS-supplemented DMEM/F12 medium. Medium was then removed and cells were washed in PBS and then in medium. There was then applied on the cells 10ml of a transfection solution containing DEAE dextran (0.4mg/ml), 100uM chloroquine, 10% NuSerum, DNA (0.4mg/ml) in DMEM/F12 medium. After incubation for 3 hours at 37C, cells were washed in PBS and medium as just described and then shocked for 1 minute with 10% DMSO in DMEM/F12 medium. Cells were allowed to grow for 2-3 days in 10% FBS-supplemented medium, and at the end of incubation dishes were placed on ice, washed with ice cold PBS and then removed by scraping. Cells were then harvested by centrifugation at 1000 rpm for 10 minutes and the cellular pellet was frozen in liquid nitrogen, for subsequent use in ligand binding assays. Northern blot analysis of a thawed aliquot of frozen cells confirmed expression of receptor-encoding cDNA in cells under storage.

In a like manner, stably transfected cell lines can also prepared using two different cell types as host: CHO K1 and CHO Pro5. To construct these cell lines, cDNA coding for human GluR3A was incorporated into the mammalian expression vector pRC/CMV (Invitrogen), which enables stable expression. Insertion at this site placed the cDNA under the expression control of the cytomegalovirus promoter and upstream of the polyadenylation site and terminator of the bovine growth hormone gene, and into a vector background comprising the neomycin resistance gene (driven by the SV40 early promoter) as selectable marker.

To introduce plasmids constructed as described above, the host CHO cells are first seeded at a density of 5 x 10⁵ in 10% FBS-supplemented MEM medium. After growth for 24 hours, fresh medium are added to the plates and three hours later, the cells are transfected using the calcium phosphate-DNA co-precipitation procedure (Maniatis et al, supra). Briefly, 3ug of DNA is mixed and incubated with buffered calcium solution for 10 minutes at room temperature. An equal volume of buffered phosphate solution is added and the suspension is incubated for 15 minutes at room temperature. Next, the incubated suspension is applied to the cells for 4 hours, removed and cells were shocked with medium containing 15% glycerol. Three minutes later, cells are washed with medium and incubated for 24 hours at normal growth conditions. Cells resistant to neomycin are selected in 10% FBS-supplemented alpha-MEM medium containing G418 (1mg/ml). Individual colonies of G418-resistant cells are isolated about 2-3 weeks later, clonally selected and then propogated for assay purposes.

### Example 6 - Construction of genetically engineered cells producing human GluR1B receptors

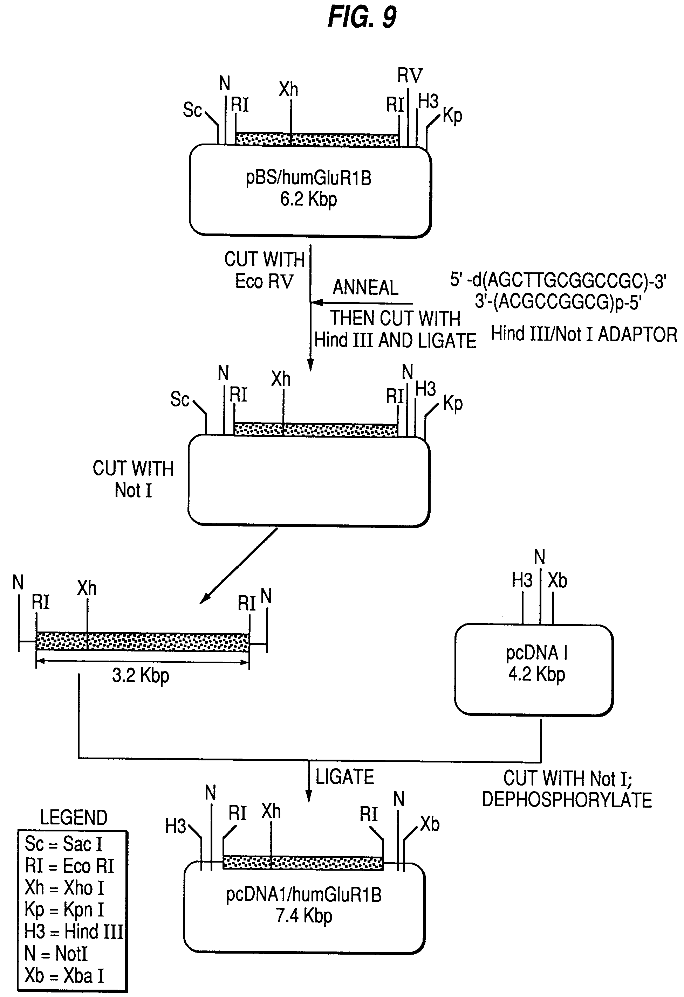

The strategy depicted in Figure 9 was employed to facilitate incorporation of the GluR1B receptor-encoding cDNA into an expression vector. Particularly, a NotI site was introduced onto the 3' flank of the Bluescript-SK cDNA insert, and the cDNA insert was then released from pBS/humGluR1B as a 3.2kb NotI/NotI fragment, which was then incorporated at the NotI site in the pcDNAI polylinker. Sequencing across the junctions was performed, to confirm proper insert orientation in pcDNA1. The resulting plasmid, designated pcDNA1/ humGluR1B, was then introduced for transient expression into monkey-derived, fibroblast like cells of the COS-1 lineage as described above.

For transient expression of the GluR1B-encoding DNA, COS-1 cells were transfected with approximately 8ug DNA (as pcDNA1/humGluR1B) per 10⁶ COS cells using the method described in Example 5.

### Example 7 - Construction of genetically engineered cells producing human GluR2B receptors

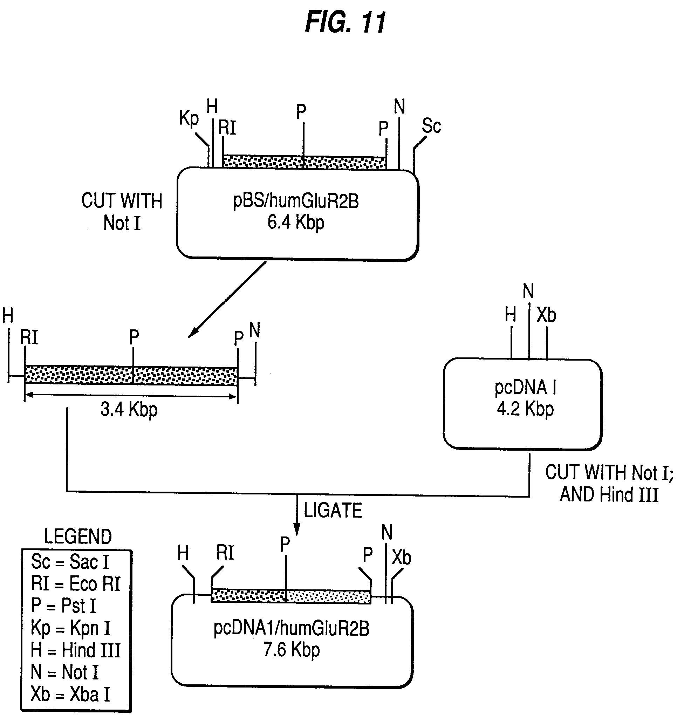

The strategy depicted in Figure 11 was employed to facilitate incorporation of the GluR2B receptor-encoding cDNA into an expression vector. Particularly, a NotI site was introduced onto the 5' flank of the Bluescript-SK cDNA insert, and the cDNA insert was then released from pBS/humGluR2B as a 3.4kb HindIII/NotI fragment, which was then incorporated at the HindIII/NotI sites in the pcDNAI polylinker. Sequencing across the junctions was performed, to confirm proper insert orientation in pcDNA1. The resulting plasmid, designated pcDNA1/humGluR2B, was then introduced for transient expression into a selected mammalian cell host, in this case the monkey-derived, fibroblast like cells of the COS-1 lineage (available from the American Type Culture Collection, Rockville, Maryland as ATCC CRL 1650).

For transient expression of the GluR2B-encoding DNA, COS-1 cells were transfected with approximately 8ug DNA (as pcDNA1/humGluR2B) per 10⁶ COS cells as set out in Example 5.

### Example 8 - Ligand binding assays

Transfected cells in the frozen state were resuspended in ice-cold distilled water using a hand homogenizer, sonicated for 5 seconds, and then centrifuged for 20 minutes at 50,000g. The supernatant was discarded and the membrane pellet stored frozen at -70C.

COS cell membrane pellets were suspended in ice cold 50mM Tris-HCl (pH 7.55, 5C) and centrifuged again at 50,000g for 10 minutes in order to remove endogenous glutamate that would compete for binding. Pellets were resuspended in ice cold 50mM Tris-HCl (pH 7.55) buffer and the resultant membrane preparation was used as tissue source for binding experiments described below. Proteins were determined using the Pierce Reagent with BSA as standard.

Binding assays were then performed, using an amount of COS-derived membrane equivalent to from 25-100ug as judged by protein determination and selected radiolabelled ligand. In particular, for AMPA-binding assays, incubation mixtures consisted of 25-100ug tissue protein and D,L-alpha-[5-methyl-³H]amino-3-hydroxy-5-methylisoxazole-4-propionic acid (³H-AMPA, 27.6Ci/mmole, 10nM final) with 0.1M KSCN and 2.5mM CaCl₂ in the 1ml final volume. Non-specific binding was determined in the presence of 1mM L-glutamate. Samples were incubated on ice for 60 minutes in plastic minivials, and bound and free ligand was separated by centrifugation for 30 minutes at 50,000g. Pellets were washed twice in 6ml of the cold incubation buffer, then 5ml of Beckman Ready-Protein Plus scintillation cocktail was added, for counting.

For kainate-binding assays, incubation mixtures consisted of 25-100ug tissue protein and [vinylidene-³H] kainic acid (58Ci/mmole, 5nM final) in the cold incubation buffer, 1ml final volume. Non-specific binding was determined in the presence of 1mM L-glutamate. Samples were incubated as for the AMPA-binding assays, and bound and free ligand were separated by rapid filtration using a Brandel cell harvester and GF/B filters pre-soaked in ice-cold 0.3% polyethyleneimine. Filters were washed twice in 6ml of the cold incubation buffer, then placed in scintillation vials with 5ml of Beckman Ready-Protein Plus scintillation cocktail for counting.

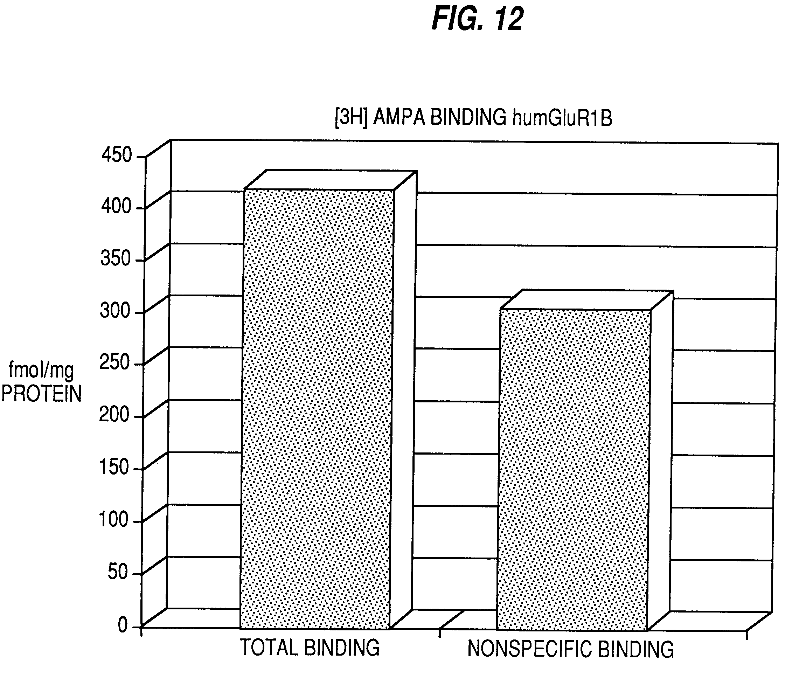

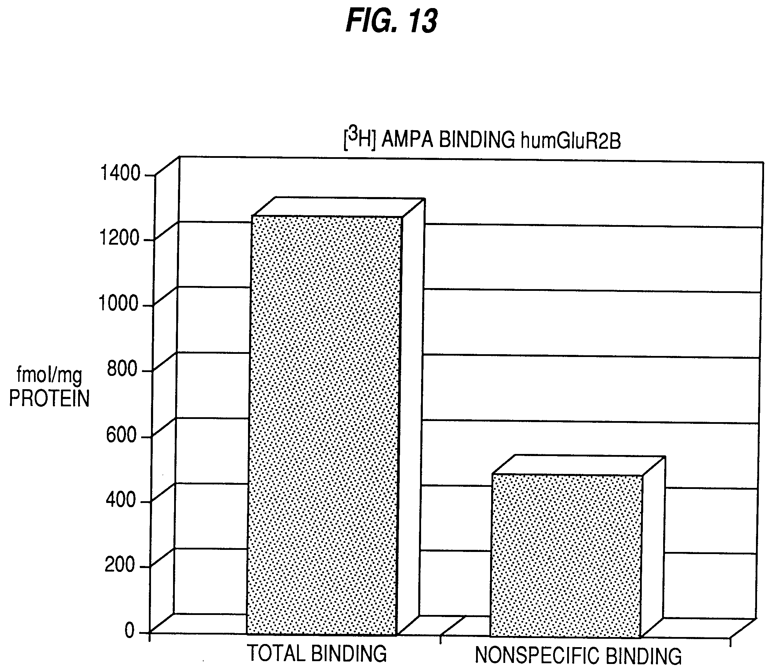

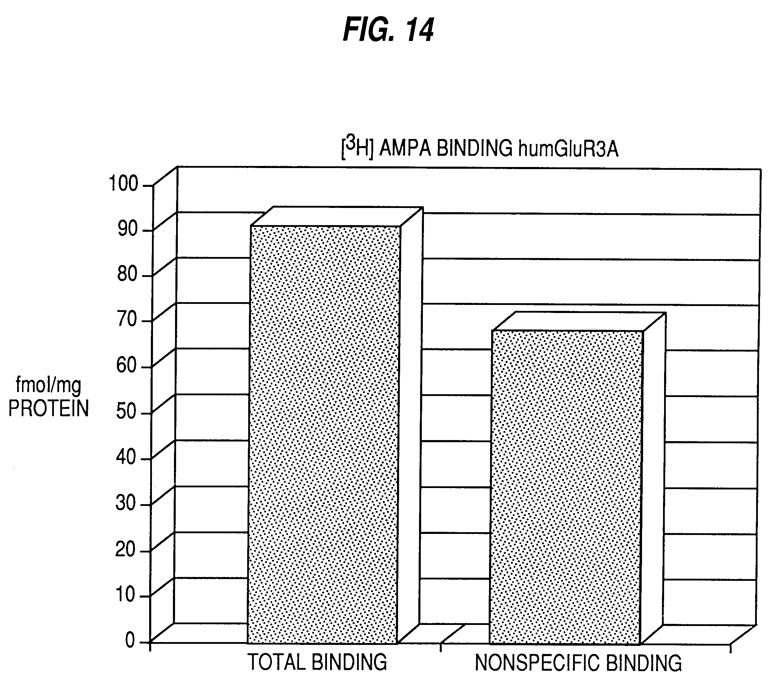

Assays performed in this manner, using membrane preparations derived from the human GluR3A receptor-producing COS cells, revealed specific binding of 25-30 fmole/mg protein at 10nM [³H]-AMPA (Figure 14); using membrane preparations derived from the human GluR1B receptor-producing COS cells, specific binding of about 100-150 fmole/mg protein at 10nM [³H]-AMPA was revealed (Figure 12); and using membrane preparations derived from the human GluR2B receptor-producing COS cells, specific binding of 750-850 fmol/mg protein at 10nM [³H]-AMPA was revealed (Figure 13). Mock transfected cells exhibited no specific binding of any of the ligands tested.

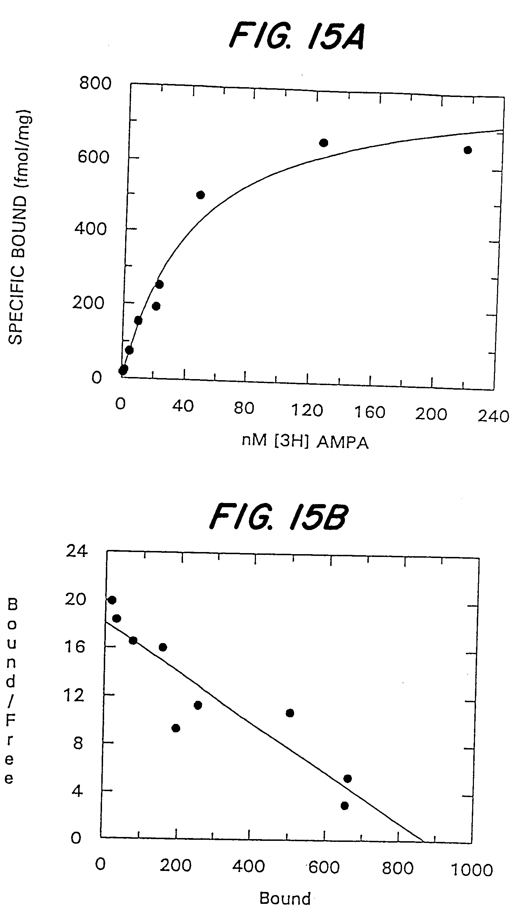

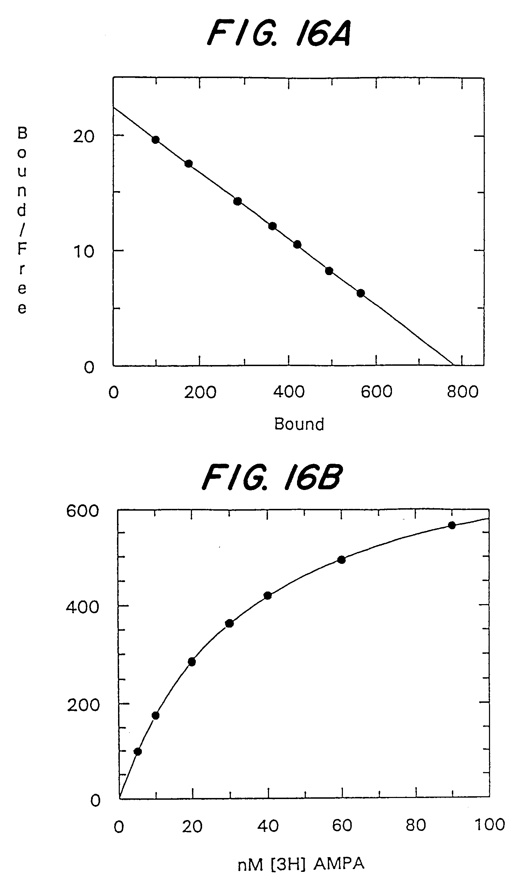

Scatchard analysis indicated that the recombinantly expressed human GluR1B and GluR2B receptors each contain a single class of [³H]-labelled AMPA binding sites with a dissociation constants (Kd) of 46 nM (Figure 15) and about 36.3 ± 7.4 nM (Figure 16), respectively. Further, the maximum AMPA-binding of the GluR1B and GluR2B receptors has been found to be 847 and 816 ± 302 fmol/mg protein, respectively.

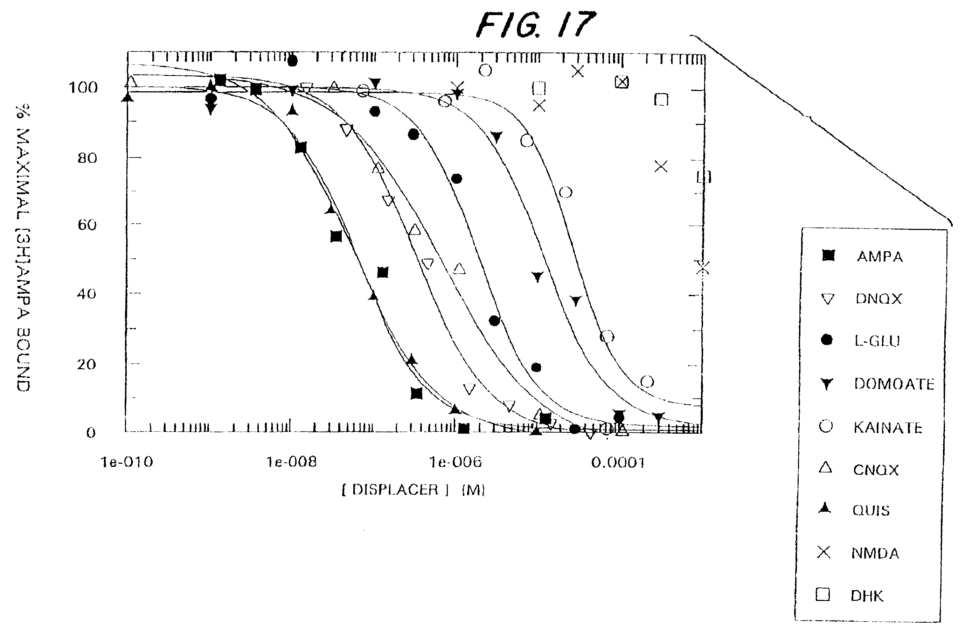

[³H]-AMPA displacement assays have also been performed for the GluR2B receptors in COS cells to determine the relative binding affinity of selected ligands. These results, as illustrated in Figure 17, indicate the rank order of potency of the ligands in displacing ³H-AMPA binding to the GluR2B receptor to be as follows:
quisqualate = AMPA > DNQX > CNQX > glutamate > domoate > kainate

These results demonstrate clearly that the human GluR receptors bind AMPA with specificity. This activity, coupled with the fact that there is little or no demonstrable binding of either kainate or NMDA, clearly assigns the human GluR receptors to be of the AMPA type of EAA receptor. Furthermore, this binding profile indicates that the receptor is binding in an authentic manner, and can therefore reliably predict the ligand binding "signature" of its non-recombinant counterpart from the human brain. These features make the recombinant receptor especially useful for selecting and characterizing ligand compounds which bind to the receptor, and/or for selecting and characterizing compounds which may act by displacing other ligands from the receptor. The isolation of the GluR receptor genes in substantially pure form, capable of being expressed as a single, homogeneous receptor species, therefore frees the ligand binding assay from the lack of precision introduced when complex, heterogeneous receptor preparations from human and other mammalian brains are used to attempt such characterizations.

## Claims

1. An isolated polynucleotide comprising a region which encodes an AMPA-binding human GluR receptor selected from the group consisting of human GluR1B, GluR2B, GluR3A and GluR3B receptors, and AMPA-binding fragments thereof.

2. An isolated polynucleotide according to claim 1, which encodes said GluR1B receptor, said GluR2B receptor, said GluR3A receptor or said GluR3B receptor.

3. An isolated polynucleotide comprising a region which encodes an AMPA-binding variant of a GluR receptor selected from the group consisting of human GluR1B, GluR2B, GluR3A and GluR3B receptors, wherein said variant has the binding profile of said receptor and varies from said receptor by conservative amino acid substitution.

4. An isolated polynucleotide according to any one of claims 1 to 3, which consists of DNA.

5. A recombinant DNA construct having incorporated therein a polynucleotide as defined in any one of claims 1 to 4.

6. A recombinant DNA construct according to claim 5, wherein the polynucleotide incorporated therein is linked operably with DNA enabling expression and secretion of said receptor in a cellular host.

7. A recombinant DNA construct according to claim 5, which is plasmid pBS/humGluR3A (ATCC 75218), plasmid pBS/humGluR3B (ATCC 75219); plasmid pBS/humGluR1B (ATCC 75246) or plasmid pBS/humGluR2B (ATCC 75217).

8. A cellular host having incorporated therein a heterologous polynucleotide as defined in any one of claims 1 to 4.

9. A cellular host according to claim 8, which is a mammalian cell.

10. An AMPA-binding membrane preparation derived from a cellular host as defined in claim 8 or claim 9.

11. A process for obtaining a substantially homogeneous source of human GluR receptor, which comprises the step of culturing a cellular host as defined in claim 8 or claim 9, and then recovering the cells so cultured.

12. A process for obtaining a substantially homogeneous source of human GluR receptor according to claim 11 comprising the subsequent step of obtaining a membrane preparation from the cultured cells.

13. A method of assaying a substance for binding to a human EAA receptor, which comprises the steps of incubating the substance under appropriate conditions with a cellular host as defined in claim 8 or claim 9, or with an AMPA-binding membrane preparation derived therefrom, and determining the extent of binding between the human GluR receptor and the substance.

14. An isolated human GluR receptor selected from the group consisting of GluR1B, GluR2B, GluR3A and GluR3B receptors, and AMPA-binding fragments thereof, in a form essentially free from other proteins of human origin.

15. An AMPA-binding fragment of a human GluR receptor selected from the group consisting of GluR1B, GluR2B, GluR3A and GluR3A receptors.

16. An antibody which binds a human GluR receptor selected from the group consisting of GluR1B, GluR2B, GluR3A and GluR3B receptors.

17. An immunogenic fragment of a human GluR receptor selected from the group consisting of GluR1B, GluR2B, GluR3A and GluR3B receptors.

18. An oligonucleotide which comprises at least about 17 nucleic acids and which hybridizes selectively with a polynucleotide defined in any one of claims 1 to 4.


SEQUENCE LISTING

(1) GENERAL INFORMATION:

(i) APPLICANT:
(A) NAME: ALLELIX BIOPHARMACEUTICALS
(B) STREET: 6850 Goreway Drive
(C) CITY: Mississauga
(D) STATE OR PROVINCE: Ontario
(E) COUNTRY: Canada
(F) POSTAL CODE: L4V 1P1
(G) TELEPHONE: (416) 677-0831
(H) FAX: (416) 677-9595

(ii) TITLE OF INVENTION: AMPA-BINDING HUMAN GLUTAMATE RECEPTORS

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v) CURRENT APPLICATION DATA:
(A) APPLICATION NUMBER: Unknown

(vi) PRIOR APPLICATION DATA:
(A) APPLICATION NUMBER: US 07/896,437
(B) FILING DATE: 10-JUN-1992

(vi) PRIOR APPLICATION DATA:
(A) APPLICATION NUMBER: US 07/896,611
(B) FILING DATE: 10-JUN-1992

(vi) PRIOR APPLICATION DATA:
(A) APPLICATION NUMBER: US 07/896,612
(B) FILING DATE: 10-JUN-1992

(2) INFORMATION FOR SEQ ID NO:1:

(i) SEQUENCE CHARACTERISTICS:
(A) LENGTH: 3220 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION: 62..2782

(ix) FEATURE:
(A) NAME/KEY: sig_peptide
(B) LOCATION: 62..115

(ix) FEATURE:
(A) NAME/KEY: mat_peptide
(B) LOCATION: 116..2782

(xi) SEQUENCE DESCRIPTION: SEQ ID NO:1:

GAATTCCACA CCAAATCTAT GATTGGACCT GGGCTTCTTT TTCGCCAATG CAAAAAGGAA 60

T ATG CAG CAC ATT TTT GCC TTC TTC TGC ACC GGT TTC CTA GGC GCG 106
Met Gln His Ile Phe Ala Phe Phe Cys Thr Gly Phe Leu Gly Ala
-18 -15 -10 -5

GTA GTA GGT GCC AAT TTC CCC AAC AAT ATC CAG ATC GGG GGA TTA TTT 154
Val Val Gly Ala Asn Phe Pro Asn Asn Ile Gln Ile Gly Gly Leu Phe
1 5 10

CCA AAC CAG CAG TCA CAG GAA CAT GCT GCT TTT AGA TTT GCT TTG TCG 202
Pro Asn Gln Gln Ser Gln Glu His Ala Ala Phe Arg Phe Ala Leu Ser
15 20 25

CAA CTC ACA GAG CCC CCG AAG CTG CTC CCC CAG ATT GAT ATT GTG AAC 250
Gln Leu Thr Glu Pro Pro Lys Leu Leu Pro Gln Ile Asp Ile Val Asn
30 35 40 45

ATC AGC GAC ACG TTT GAG ATG ACC TAT AGA TTC TGT TCC CAG TTC TCC 298
Ile Ser Asp Thr Phe Glu Met Thr Tyr Arg Phe Cys Ser Gln Phe Ser
    50                  55                  60

AAA GGA GTC TAT GCC ATC TTT GGG TTT TAT GAA CGT AGG ACT GTC AAC 346
Lys Gly Val Tyr Ala Ile Phe Gly Phe Tyr Glu Arg Arg Thr Val Asn
65 70 75

ATG CTG ACC TCC TTT TGT GGG GCC CTC CAC GTC TGC TTC ATT ACG CCG 394
Met Leu Thr Ser Phe Cys Gly Ala Leu His Val Cys Phe Ile Thr Pro
80 85 90

AGC TTT CCC GTT GAT ACA TCC AAT CAG TTT GTC CTT CAG CTG CGC CCT 442
Ser Phe Pro Val Asp Thr Ser Asn Gln Phe Val Leu Gln Leu Arg Pro
95 100 105

GAA CTG CAG GAT GCC CTC ATC AGC ATC ATT GAC CAT TAC AAG TGG CAG 490
Glu Leu Gln Asp Ala Leu Ile Ser Ile Ile Asp His Tyr Lys Trp Gln
110 115 120 125

AAA TTT GTC TAC ATT TAT GAT GCC GAC CGG GGC TTA TCC GTC CTG CAG 538
Lys Phe Val Tyr Ile Tyr Asp Ala Asp Arg Gly Leu Ser Val Leu Gln
    130                 135                 140

AAA GTC CTG GAT ACA GCT GCT GAG AAG AAC TGG CAG GTG ACA GCA GTC 586
Lys Val Leu Asp Thr Ala Ala Glu Lys Asn Trp Gln Val Thr Ala Val
145 150 155

AAC ATT TTG ACA ACC ACA GAG GAG GGA TAC CGG ATG CTC TTT CAG GAC 634
Asn Ile Leu Thr Thr Thr Glu Glu Gly Tyr Arg Met Leu Phe Gln Asp
160 165 170

CTG GAG AAG AAA AAG GAG CGG CTG GTG GTG GTG GAC TGT GAA TCA GAA 682
Leu Glu Lys Lys Lys Glu Arg Leu Val Val Val Asp Cys Glu Ser Glu
175 180 185

CGC CTC AAT GCT ATC TTG GGC CAG ATT ATA AAG CTA GAG AAG AAT GGC 730
Arg Leu Asn Ala Ile Leu Gly Gln Ile Ile Lys Leu Glu Lys Asn Gly
190 195 200 205

ATC GGC TAC CAC TAC ATT CTT GCA AAT CTG GGC TTC ATG GAC ATT GAC 778
Ile Gly Tyr His Tyr Ile Leu Ala Asn Leu Gly Phe Met Asp Ile Asp
    210                 215                 220

TTA AAC AAA TTC AAG GAG AGT GGC GCC AAT GTG ACA GGT TTC CAG CTG 826
Leu Asn Lys Phe Lys Glu Ser Gly Ala Asn Val Thr Gly Phe Gln Leu
225 230 235

GTG AAC TAC ACA GAC ACT ATT CCG GCC AAG ATC ATG CAG CAG TGG AAG 874
Val Asn Tyr Thr Asp Thr Ile Pro Ala Lys Ile Met Gln Gln Trp Lys
240 245 250

AAT AGT GAT GCT CGA GAC CAC ACA CGG GTG GAC TGG AAG AGA CCC AAG 922
Asn Ser Asp Ala Arg Asp His Thr Arg Val Asp Trp Lys Arg Pro Lys
255 260 265

TAC ACC TCT GCG CTC ACC TAC GAT GGG GTG AAG GTG ATG GCT GAG GCT 970
Tyr Thr Ser Ala Leu Thr Tyr Asp Gly Val Lys Val Met Ala Glu Ala
270 275 280 285

TTC CAG AGC CTG CGG AGG CAG AGA ATT GAT ATA TCT CGC CGG GGG AAT 1018
Phe Gln Ser Leu Arg Arg Gln Arg Ile Asp Ile Ser Arg Arg Gly Asn
    290                 295                 300

GCT GGG GAT TGT CTG GCT AAC CCA GCT GTT CCC TGG GGC CAA GGG ATC 1066
Ala Gly Asp Cys Leu Ala Asn Pro Ala Val Pro Trp Gly Gln Gly Ile
305 310 315

GAC ATC CAG AGA GCT CTG CAG CAG GTG CGA TTT GAA GGT TTA ACA GGA 1114
Asp Ile Gln Arg Ala Leu Gln Gln Val Arg Phe Glu Gly Leu Thr Gly
320 325 330

AAC GTG CAG TTT AAT GAG AAA GGA CGC CGG ACC AAC TAC ACG CTC CAC 1162
Asn Val Gln Phe Asn Glu Lys Gly Arg Arg Thr Asn Tyr Thr Leu His
335 340 345

GTG ATT GAA ATG AAA CAT GAC GGC ATC CGA AAG ATT GGT TAC TGG AAT 1210
Val Ile Glu Met Lys His Asp Gly Ile Arg Lys Ile Gly Tyr Trp Asn
350 355 360 365

GAA GAT GAT AAG TTT GTC CCT GCA GCC ACC GAT GCC CAA GCT GGG GGC 1258
Glu Asp Asp Lys Phe Val Pro Ala Ala Thr Asp Ala Gln Ala Gly Gly
    370                 375                 380

GAT AAT TCA AGT GTT CAG AAC AGA ACA TAC ATC GTC ACA ACA ATC CTA 1306
Asp Asn Ser Ser Val Gln Asn Arg Thr Tyr Ile Val Thr Thr Ile Leu
385 390 395

GAA GAT CCT TAT GTG ATG CTC AAG AAG AAC GCC AAT CAG TTT GAG GGC 1354
Glu Asp Pro Tyr Val Met Leu Lys Lys Asn Ala Asn Gln Phe Glu Gly
400 405 410

AAT GAC CGT TAC GAG GGC TAC TGT GTA GAG CTG GCG GCA GAG ATT GCC 1402
Asn Asp Arg Tyr Glu Gly Tyr Cys Val Glu Leu Ala Ala Glu Ile Ala
415 420 425

AAG CAC GTG GGC TAC TCC TAC CGT CTG GAG ATT GTC AGT GAT GGA AAA 1450
Lys His Val Gly Tyr Ser Tyr Arg Leu Glu Ile Val Ser Asp Gly Lys
430 435 440 445

TAC GGA GCC CGA GAC CCT GAC ACG AAG GCC TGG AAT GGC ATG GTG GGA 1498
Tyr Gly Ala Arg Asp Pro Asp Thr Lys Ala Trp Asn Gly Met Val Gly
    450                 455                 460

GAG CTG GTC TAT GGA AGA GCA GAT GTG GCT GTG GCT CCC TTA ACT ATC 1546
Glu Leu Val Tyr Gly Arg Ala Asp Val Ala Val Ala Pro Leu Thr Ile
465 470 475

ACT TTG GTC CGG GAA GAA GTT ATA GAT TTC TCC AAA CCA TTT ATG AGT 1594
Thr Leu Val Arg Glu Glu Val Ile Asp Phe Ser Lys Pro Phe Met Ser
480 485 490

TTG GGG ATC TCC ATC ATG ATT AAA AAA CCA CAG AAA TCC AAG CCG GGT 1642
Leu Gly Ile Ser Ile Met Ile Lys Lys Pro Gln Lys Ser Lys Pro Gly
495 500 505

GTC TTC TCC TTC CTT GAT CCT TTG GCT TAT GAG ATT TGG ATG TGC ATT 1690
Val Phe Ser Phe Leu Asp Pro Leu Ala Tyr Glu Ile Trp Met Cys Ile
510 515 520 525

GTT TTT GCC TAC ATT GGA GTG AGT GTT GTC CTC TTC CTG GTC AGC CGC 1738
Val Phe Ala Tyr Ile Gly Val Ser Val Val Leu Phe Leu Val Ser Arg
    530                 535                 540

TTC AGT CCC TAT GAA TGG CAC AGT GAA GAG TTT GAG GAA GGA CGG GAC 1786
Phe Ser Pro Tyr Glu Trp His Ser Glu Glu Phe Glu Glu Gly Arg Asp
545 550 555

CAG ACA ACC AGT GAC CAG TCC AAT GAG TTT GGG ATA TTC AAC AGT TTG 1834
Gln Thr Thr Ser Asp Gln Ser Asn Glu Phe Gly Ile Phe Asn Ser Leu
560 565 570

TGG TTC TCC CTG GGA GCC TTC ATG CAG CAA GGA TGT GAC ATT TCT CCC 1882
Trp Phe Ser Leu Gly Ala Phe Met Gln Gln Gly Cys Asp Ile Ser Pro
575 580 585

AGG TCC CTG TCT GGT CGC ATC GTT GGT GGC GTC TGG TGG TTC TTC ACC 1930
Arg Ser Leu Ser Gly Arg Ile Val Gly Gly Val Trp Trp Phe Phe Thr
590 595 600 605

TTA ATC ATC ATC TCC TCA TAT ACA GCC AAT CTG GCC GCC TTC CTG ACC 1978
Leu Ile Ile Ile Ser Ser Tyr Thr Ala Asn Leu Ala Ala Phe Leu Thr
    610                 615                 620

GTG GAG AGG ATG GTG TCT CCC ATT GAG AGT GCA GAG GAC CTA GCG AAC 2026
Val Glu Arg Met Val Ser Pro Ile Glu Ser Ala Glu Asp Leu Ala Asn
625 630 635

GAG ACA GAA ATT GCC TAC GGG ACG CTG GAA GCA GGA TCT ACT AAG GAG 2074
Glu Thr Glu Ile Ala Tyr Gly Thr Leu Glu Ala Gly Ser Thr Lys Glu
640 645 650

TTC TTC AGG AGG TCT AAA ATT GCT GTG TTT GAG AAG ATG TGG ACA TAC 2122
Phe Phe Arg Arg Ser Lys Ile Ala Val Phe Glu Lys Met Trp Thr Tyr
655 660 665

ATG AAG TCA GCA GAG CCA TCA GTT TTT GTG CGG ACC ACA GAG GAG GGG 2170
Met Lys Ser Ala Glu Pro Ser Val Phe Val Arg Thr Thr Glu Glu Gly
670 675 680 685

ATG ATT CGA GTG AGG AAA TCC AAA GGC AAA TAT GCC TAC CTC CTG GAG 2218
Met Ile Arg Val Arg Lys Ser Lys Gly Lys Tyr Ala Tyr Leu Leu Glu
    690                 695                 700

TCC ACC ATG AAT GAG TAC ATT GAG CAG CGG AAA CCC TGT GAC ACC ATG 2266
Ser Thr Met Asn Glu Tyr Ile Glu Gln Arg Lys Pro Cys Asp Thr Met
705 710 715

AAG GTG GGA GGT AAC TTG GAT TCC AAA GGC TAT GGC ATT GCA ACA CCC 2314
Lys Val Gly Gly Asn Leu Asp Ser Lys Gly Tyr Gly Ile Ala Thr Pro
720 725 730

AAG GGG TCT GCC CTG AGA GGT CCC GTA AAC CTA GCG GTT TTG AAA CTC 2362
Lys Gly Ser Ala Leu Arg Gly Pro Val Asn Leu Ala Val Leu Lys Leu
735 740 745

AGT GAG CAA GGC GTC TTA GAC AAG CTG AAA AGC AAA TGG TGG TAC GAT 2410
Ser Glu Gln Gly Val Leu Asp Lys Leu Lys Ser Lys Trp Trp Tyr Asp
750 755 760 765

AAA GGG GAA TGT GGA AGC AAG GAC TCC GGA AGT AAG GAC AAG ACA AGC 2458
Lys Gly Glu Cys Gly Ser Lys Asp Ser Gly Ser Lys Asp Lys Thr Ser
    770                 775                 780

GCT CTG AGC CTC AGC AAT GTG GCA GGC GTG TTC TAC ATC CTG ATC GGA 2506
Ala Leu Ser Leu Ser Asn Val Ala Gly Val Phe Tyr Ile Leu Ile Gly
785 790 795

GGA CTT GGA CTA GCC ATG CTG GTT GCC TTA ATC GAG TTC TGC TAC AAA 2554
Gly Leu Gly Leu Ala Met Leu Val Ala Leu Ile Glu Phe Cys Tyr Lys
800 805 810

TCC CGT AGT GAA TCC AAG CGG ATG AAG GGT TTT TGT TTG ATC CCA CAG 2602
Ser Arg Ser Glu Ser Lys Arg Met Lys Gly Phe Cys Leu Ile Pro Gln
815 820 825

CAA TCC ATC AAC GAA GCC ATA CGG ACA TCG ACC CTC CCC CGC AAC AGC 2650
Gln Ser Ile Asn Glu Ala Ile Arg Thr Ser Thr Leu Pro Arg Asn Ser
830 835 840 845

GGG GCA GGA GCC AGC AGC GGC GGC AGT GGA GAG AAT GGT CGG GTG GTC 2698
Gly Ala Gly Ala Ser Ser Gly Gly Ser Gly Glu Asn Gly Arg Val Val
    850                 855                 860

AGC CAT GAC TTC CCC AAG TCC ATG CAA TCG ATT CCT TGC ATG AGC CAC 2746
Ser His Asp Phe Pro Lys Ser Met Gln Ser Ile Pro Cys Met Ser His
865 870 875

AGT TCA GGG ATG CCC TTG GGA GCC ACG GGA TTG TAACTGGAGC AGATGGAGAC 2799
Ser Ser Gly Met Pro Leu Gly Ala Thr Gly Leu
880 885

CCCTTGGGGA GCAGGCTCGG GCTCCCCAGC CCCATCCCAA ACCCTTCAGT GCCAAAAACA 2859

ACAACAAAAT AGAAAGCGCA ACCACCACCA ACCACTGCGA CCACAAGAAG GATGATTCAA 2919

CAGGTTTTCC TGAAGAATTG AAAAACCATT TTGCTGTCCC TTTTCCTTTT TTGATGTTCT 2979

TTCACCCTTT TCTGTTTGCT AAGTGAGGAT GAAAAAATAA CACTGTACTG CAATAAGGGG 3039

AGAGTAACCC TGTCTAATGA AACCTGTGTC TCTGAGAGTA GAGTCACTGG AACACTAATG 3099

AGGAAACTGC ACTGTTTTAT TTTAATTCAG TTGTTAGTGT GTCTTAGTGT GTGCAATTTT 3159

TTTTCTTACT AATATCCATG GTTTGCAGGT TCTGTTAGGC CCTTTCCTTC TCCTGGAATT 3219

C 3220

(2) INFORMATION FOR SEQ ID NO:2:

(i) SEQUENCE CHARACTERISTICS:
(A) LENGTH: 906 amino acids
(B) TYPE: amino acid
(D) TOPOLOGY: linear

(ii) MOLECULE TYPE: protein

(xi) SEQUENCE DESCRIPTION: SEQ ID NO:2:

Met Gln His Ile Phe Ala Phe Phe Cys Thr Gly Phe Leu Gly Ala Val
-18 -15 -10 -5

Val Gly Ala Asn Phe Pro Asn Asn Ile Gln Ile Gly Gly Leu Phe Pro
1 5 10

Asn Gln Gln Ser Gln Glu His Ala Ala Phe Arg Phe Ala Leu Ser Gln
15 20 25 30

Leu Thr Glu Pro Pro Lys Leu Leu Pro Gln Ile Asp Ile Val Asn Ile
        35                  40                  45

Ser Asp Thr Phe Glu Met Thr Tyr Arg Phe Cys Ser Gln Phe Ser Lys
    50                  55                  60

Gly Val Tyr Ala Ile Phe Gly Phe Tyr Glu Arg Arg Thr Val Asn Met
65 70 75

Leu Thr Ser Phe Cys Gly Ala Leu His Val Cys Phe Ile Thr Pro Ser
80 85 90

Phe Pro Val Asp Thr Ser Asn Gln Phe Val Leu Gln Leu Arg Pro Glu
95 100 105 110

Leu Gln Asp Ala Leu Ile Ser Ile Ile Asp His Tyr Lys Trp Gln Lys
        115                 120                 125

Phe Val Tyr Ile Tyr Asp Ala Asp Arg Gly Leu Ser Val Leu Gln Lys
    130                 135                 140

Val Leu Asp Thr Ala Ala Glu Lys Asn Trp Gln Val Thr Ala Val Asn
145 150 155

Ile Leu Thr Thr Thr Glu Glu Gly Tyr Arg Met Leu Phe Gln Asp Leu
160 165 170

Glu Lys Lys Lys Glu Arg Leu Val Val Val Asp Cys Glu Ser Glu Arg
175 180 185 190

Leu Asn Ala Ile Leu Gly Gln Ile Ile Lys Leu Glu Lys Asn Gly Ile
        195                 200                 205

Gly Tyr His Tyr Ile Leu Ala Asn Leu Gly Phe Met Asp Ile Asp Leu
    210                 215                 220

Asn Lys Phe Lys Glu Ser Gly Ala Asn Val Thr Gly Phe Gln Leu Val
225 230 235

Asn Tyr Thr Asp Thr Ile Pro Ala Lys Ile Met Gln Gln Trp Lys Asn
240 245 250

Ser Asp Ala Arg Asp His Thr Arg Val Asp Trp Lys Arg Pro Lys Tyr
255 260 265 270

Thr Ser Ala Leu Thr Tyr Asp Gly Val Lys Val Met Ala Glu Ala Phe
        275                 280                 285

Gln Ser Leu Arg Arg Gln Arg Ile Asp Ile Ser Arg Arg Gly Asn Ala
    290                 295                 300

Gly Asp Cys Leu Ala Asn Pro Ala Val Pro Trp Gly Gln Gly Ile Asp
305 310 315

Ile Gln Arg Ala Leu Gln Gln Val Arg Phe Glu Gly Leu Thr Gly Asn
320 325 330

Val Gln Phe Asn Glu Lys Gly Arg Arg Thr Asn Tyr Thr Leu His Val
335 340 345 350

Ile Glu Met Lys His Asp Gly Ile Arg Lys Ile Gly Tyr Trp Asn Glu
        355                 360                 365

Asp Asp Lys Phe Val Pro Ala Ala Thr Asp Ala Gln Ala Gly Gly Asp
    370                 375                 380

Asn Ser Ser Val Gln Asn Arg Thr Tyr Ile Val Thr Thr Ile Leu Glu
385 390 395

Asp Pro Tyr Val Met Leu Lys Lys Asn Ala Asn Gln Phe Glu Gly Asn
    400                 405                 410

Asp Arg Tyr Glu Gly Tyr Cys Val Glu Leu Ala Ala Glu Ile Ala Lys
415                 420                 425                 430

His Val Gly Tyr Ser Tyr Arg Leu Glu Ile Val Ser Asp Gly Lys Tyr
                435                 440                 445

Gly Ala Arg Asp Pro Asp Thr Lys Ala Trp Asn Gly Met Val Gly Glu
            450                 455                 460

Leu Val Tyr Gly Arg Ala Asp Val Ala Val Ala Pro Leu Thr Ile Thr
    465                 470                 475

Leu Val Arg Glu Glu Val Ile Asp Phe Ser Lys Pro Phe Met Ser Leu
    480                 485                 490

Gly Ile Ser Ile Met Ile Lys Lys Pro Gln Lys Ser Lys Pro Gly Val
495                 500                 505                 510

Phe Ser Phe Leu Asp Pro Leu Ala Tyr Glu Ile Trp Met Cys Ile Val
                515                 520                 525

Phe Ala Tyr Ile Gly Val Ser Val Val Leu Phe Leu Val Ser Arg Phe
            530                 535                 540

Ser Pro Tyr Glu Trp His Ser Glu Glu Phe Glu Glu Gly Arg Asp Gln
        545                 550                 555

Thr Thr Ser Asp Gln Ser Asn Glu Phe Gly Ile Phe Asn Ser Leu Trp
    560                 565                 570

Phe Ser Leu Gly Ala Phe Met Gln Gln Gly Cys Asp Ile Ser Pro Arg
575                 580                 585                 590

Ser Leu Ser Gly Arg Ile Val Gly Gly Val Trp Trp Phe Phe Thr Leu
                595                 600                 605

Ile Ile Ile Ser Ser Tyr Thr Ala Asn Leu Ala Ala Phe Leu Thr Val
            610                 615                 620

Glu Arg Met Val Ser Pro Ile Glu Ser Ala Glu Asp Leu Ala Asn Glu
        625                 630                 635

Thr Glu Ile Ala Tyr Gly Thr Leu Glu Ala Gly Ser Thr Lys Glu Phe
    640                 645                 650

Phe Arg Arg Ser Lys Ile Ala Val Phe Glu Lys Met Trp Thr Tyr Met
655                 660                 665                 670

Lys Ser Ala Glu Pro Ser Val Phe Val Arg Thr Thr Glu Glu Gly Met
                675                 680                 685

Ile Arg Val Arg Lys Ser Lys Gly Lys Tyr Ala Tyr Leu Leu Glu Ser
            690                 695                 700

Thr Met Asn Glu Tyr Ile Glu Gln Arg Lys Pro Cys Asp Thr Met Lys
        705                 710                 715

Val Gly Gly Asn Leu Asp Ser Lys Gly Tyr Gly Ile Ala Thr Pro Lys
    720                 725                 730

Gly Ser Ala Leu Arg Gly Pro Val Asn Leu Ala Val Leu Lys Leu Ser
735                 740                 745                 750

Glu Gln Gly Val Leu Asp Lys Leu Lys Ser Lys Trp Trp Tyr Asp Lys
                755                 760                 765

Gly Glu Cys Gly Ser Lys Asp Ser Gly Ser Lys Asp Lys Thr Ser Ala
            770                 775                 780

Leu Ser Leu Ser Asn Val Ala Gly Val Phe Tyr Ile Leu Ile Gly Gly
        785                 790                 795

Leu Gly Leu Ala Met Leu Val Ala Leu Ile Glu Phe Cys Tyr Lys Ser
    800                 805                 810

Arg Ser Glu Ser Lys Arg Met Lys Gly Phe Cys Leu Ile Pro Gln Gln
815                 820                 825                 830

Ser Ile Asn Glu Ala Ile Arg Thr Ser Thr Leu Pro Arg Asn Ser Gly
                835                 840                 845

Ala Gly Ala Ser Ser Gly Gly Ser Gly Glu Asn Gly Arg Val Val Ser
            850                 855                 860

His Asp Phe Pro Lys Ser Met Gln Ser Ile Pro Cys Met Ser His Ser
        865                 870                 875

Ser Gly Met Pro Leu Gly Ala Thr Gly Leu
    880                 885

(2) INFORMATION FOR SEQ ID NO:3:

     (i) SEQUENCE CHARACTERISTICS:
          (A) LENGTH: 3407 base pairs
          (B) TYPE: nucleic acid
          (C) STRANDEDNESS: double
          (D) TOPOLOGY: linear

    (ii) MOLECULE TYPE: cDNA

    (ix) FEATURE:
          (A) NAME/KEY: CDS
          (B) LOCATION: 315..2966

    (ix) FEATURE:
          (A) NAME/KEY: sig_peptide
          (B) LOCATION: 315..374

    (ix) FEATURE:
          (A) NAME/KEY: mat_peptide
          (B) LOCATION: 375..2966

    (xi) SEQUENCE DESCRIPTION: SEQ ID NO:3:

GAATTCCGTG AGTGCATGGG AGGGTGCTGA ATATTCCGAG ACACTGGGAC CACAGCGGCA      60

GCTCCGCTGA AAACTGCATT CAGCCAGTCC TCCGGACTTC TGGAGCGGGG ACAGGGCGCA     120

GGGCATCAGC AGCCACCAGC AGGACCTGGG AAATAGGGAT TCTTCTGCCT CCACTTCAGG     180

TTTTAGCAGC TTGGTGCTAA ATTGCTGTCT CAAAATGCAG AGGATCTAAT TTGCAGAGGA     240

AAACAGCCAA AGAAGGAAGA GGAGGAAAAG GAAAAAAAAA GGGGTATATT GTGGATGCTC     300

TACTTTTCTT GGAA ATG CAA AAG ATT ATG CAT ATT TCT GTC CTC CTT TCT       350
                Met Gln Lys Ile Met His Ile Ser Val Leu Leu Ser
                -20                 -15                 -10

CCT GTT TTA TGG GGA CTG ATT TTT GGT GTC TCT TCT AAC AGC ATA CAG 398
Pro Val Leu Trp Gly Leu Ile Phe Gly Val Ser Ser Asn Ser Ile Gln
-5 1 5

ATA GGG GGG CTA TTT CCT AGG GGC GCC GAT CAA GAA TAC AGT GCA TTT 446
Ile Gly Gly Leu Phe Pro Arg Gly Ala Asp Gln Glu Tyr Ser Ala Phe
10 15 20

CGA GTA GGG ATG GTT CAG TTT TCC ACT TCG GAG TTC AGA CTG ACA CCC 494
Arg Val Gly Met Val Gln Phe Ser Thr Ser Glu Phe Arg Leu Thr Pro
25 30 35 40

CAC ATC GAC AAT TTG GAG GTG GCA AAC AGC TTC GCA GTC ACT AAT GCT 542
His Ile Asp Asn Leu Glu Val Ala Asn Ser Phe Ala Val Thr Asn Ala
45 50 55

TTC TGC TCC CAG TTT TCG AGA GGA GTC TAT GCT ATT TTT GGA TTT TAT 590
Phe Cys Ser Gln Phe Ser Arg Gly Val Tyr Ala Ile Phe Gly Phe Tyr
60 65 70

GAC AAG AAG TCT GTA AAT ACC ATC ACA TCA TTT TGC GGA ACA CTC CAC 638
Asp Lys Lys Ser Val Asn Thr Ile Thr Ser Phe Cys Gly Thr Leu His
75 80 85

GTC TCC TTC ATC ACT CCC AGC TTC CCA ACA GAT GGC ACA CAT CCA TTT 686
Val Ser Phe Ile Thr Pro Ser Phe Pro Thr Asp Gly Thr His Pro Phe
90 95 100

GTC ATT CAG ATG AGA CCC GAC CTC AAA GGA GCT CTC CTT AGC TTG ATT 734
Val Ile Gln Met Arg Pro Asp Leu Lys Gly Ala Leu Leu Ser Leu Ile
105 110 115 120

GAA TAC TAT CAA TGG GAC AAG TTT GCA TAC CTC TAT GAC AGT GAC AGA 782
Glu Tyr Tyr Gln Trp Asp Lys Phe Ala Tyr Leu Tyr Asp Ser Asp Arg
125 130 135

GGC TTA TCA ACA CTG CAA GCT GTG CTG GAT TCT GCT GCT GAA AAG AAA 830
Gly Leu Ser Thr Leu Gln Ala Val Leu Asp Ser Ala Ala Glu Lys Lys
140 145 150

TGG CAA GTG ACT GCT ATC AAT GTG GGA AAC ATT AAC AAT GAC AAG AAA 878
Trp Gln Val Thr Ala Ile Asn Val Gly Asn Ile Asn Asn Asp Lys Lys
155 160 165

GAT GAG ATG TAC CGA TCA CTT TTT CAA GAT CTG GAG TTA AAA AAG GAA 926
Asp Glu Met Tyr Arg Ser Leu Phe Gln Asp Leu Glu Leu Lys Lys Glu
170 175 180

CGG CGT GTA ATT CTG GAC TGT GAA AGG GAT AAA GTA AAC GAC ATT GTA 974
Arg Arg Val Ile Leu Asp Cys Glu Arg Asp Lys Val Asn Asp Ile Val
185 190 195 200

GAC CAG GTT ATT ACC ATT GGA AAA CAC GTT AAA GGG TAC CAC TAC ATC 1022
Asp Gln Val Ile Thr Ile Gly Lys His Val Lys Gly Tyr His Tyr Ile
205 210 215

ATT GCA AAT CTG GGA TTT ACT GAT GGA GAC CTA TTA AAA ATC CAG TTT 1070
Ile Ala Asn Leu Gly Phe Thr Asp Gly Asp Leu Leu Lys Ile Gln Phe
220 225 230

GGA GGT GCA AAT GTC TCT GGA TTT CAG ATA GTG GAC TAT GAT GAT TCG 1118
Gly Gly Ala Asn Val Ser Gly Phe Gln Ile Val Asp Tyr Asp Asp Ser
235 240 245

TTG GTA TCT AAA TTT ATA GAA AGA TGG TCA ACA CTG GAA GAA AAA GAA 1166
Leu Val Ser Lys Phe Ile Glu Arg Trp Ser Thr Leu Glu Glu Lys Glu
250 255 260

TAC CCT GGA GCT CAC ACA ACA ACA ATT AAG TAT ACT TCT GCT CTG ACC 1214
Tyr Pro Gly Ala His Thr Thr Thr Ile Lys Tyr Thr Ser Ala Leu Thr
265 270 275 280

TAT GAT GCC GTT CAA GTG ATG ACT GAA GCC TTC CGC AAC CTA AGG AAG 1262
Tyr Asp Ala Val Gln Val Met Thr Glu Ala Phe Arg Asn Leu Arg Lys
285 290 295

CAA AGA ATT GAA ATC TCC CGA AGG GGG AAT GCA GGA GAC TGT CTG GCA 1310
Gln Arg Ile Glu Ile Ser Arg Arg Gly Asn Ala Gly Asp Cys Leu Ala
300 305 310

AAC CCA GCA GTG CCC TGG GGA CAA GGT GTA GAA ATA GAA AGG GCC CTC 1358
Asn Pro Ala Val Pro Trp Gly Gln Gly Val Glu Ile Glu Arg Ala Leu
315 320 325

AAA CAG GTT CAG GTT GAA GGT CTC TCA GGA AAT ATA AAG TTT GAC CAG 1406
Lys Gln Val Gln Val Glu Gly Leu Ser Gly Asn Ile Lys Phe Asp Gln
330 335 340

AAT GGA AAA AGA ATA AAC TAT ACA ATT AAC ATC ATG GAG CTC AAA ACT 1454
Asn Gly Lys Arg Ile Asn Tyr Thr Ile Asn Ile Met Glu Leu Lys Thr
345 350 355 360

AAT GGG CCC CGG AAG ATT GGC TAC TGG AGT GAA GTG GAC AAA ATG GTT 1502
Asn Gly Pro Arg Lys Ile Gly Tyr Trp Ser Glu Val Asp Lys Met Val
365 370 375

GTT ACC CTT ACT GAG CTC CCT TCT GGA AAT GAC ACC TCT GGG CTT GAG 1550
Val Thr Leu Thr Glu Leu Pro Ser Gly Asn Asp Thr Ser Gly Leu Glu
380 385 390

AAT AAG ACT GTT GTT GTC ACC ACA ATT TTG GAA TCT CCG TAT GTT ATG 1598
Asn Lys Thr Val Val Val Thr Thr Ile Leu Glu Ser Pro Tyr Val Met
395 400 405

ATG AAG AAA AAT CAT GAA ATG CTT GAA GGC AAT GAG CGC TAT GAG GGC 1646
Met Lys Lys Asn His Glu Met Leu Glu Gly Asn Glu Arg Tyr Glu Gly
410 415 420

TAC TGT GTT GAC CTG GCT GCA GAA ATC GCC AAA CAT TGT GGG TTC AAG 1694
Tyr Cys Val Asp Leu Ala Ala Glu Ile Ala Lys His Cys Gly Phe Lys
425 430 435 440

TAC AAG TTG ACA ATT GTT GGT GAT GGC AAG TAT GGG GCC AGG GAT GCA 1742
Tyr Lys Leu Thr Ile Val Gly Asp Gly Lys Tyr Gly Ala Arg Asp Ala
445 450 455

GAC ACG AAA ATT TGG AAT GGG ATG GTT GGA GAA CTT GTA TAT GGG AAA 1790
Asp Thr Lys Ile Trp Asn Gly Met Val Gly Glu Leu Val Tyr Gly Lys
460 465 470

GCT GAT ATT GCA ATT GCT CCA TTA ACT ATT ACC CTT GTG AGA GAA GAG 1838
Ala Asp Ile Ala Ile Ala Pro Leu Thr Ile Thr Leu Val Arg Glu Glu
475 480 485

GTG ATT GAC TTC TCA AAG CCC TTC ATG AGC CTC GGG ATA TCT ATC ATG 1886
Val Ile Asp Phe Ser Lys Pro Phe Met Ser Leu Gly Ile Ser Ile Met
490 495 500

ATC AAG AAG CCT CAG AAG TCC AAA CCA GGA GTG TTT TCC TTT CTT GAT 1934
Ile Lys Lys Pro Gln Lys Ser Lys Pro Gly Val Phe Ser Phe Leu Asp
505 510 515 520

CCT TTA GCC TAT GAG ATC TGG ATG TGC ATT GTT TTT GCC TAC ATT GGG 1982
Pro Leu Ala Tyr Glu Ile Trp Met Cys Ile Val Phe Ala Tyr Ile Gly
525 530 535

GTC AGT GTA GTT TTA TTC CTG GTC AGC AGA TTT AGC CCC TAC GAG TGG 2030
Val Ser Val Val Leu Phe Leu Val Ser Arg Phe Ser Pro Tyr Glu Trp
540 545 550

CAC ACT GAG GAG TTT GAA GAT GGA AGA GAA ACA CAA AGT AGT GAA TCA 2078
His Thr Glu Glu Phe Glu Asp Gly Arg Glu Thr Gln Ser Ser Glu Ser
555 560 565

ACT AAT GAA TTT GGG ATT TTT AAT AGT CTC TGG TTT TCC TTG GGT GCC 2126
Thr Asn Glu Phe Gly Ile Phe Asn Ser Leu Trp Phe Ser Leu Gly Ala
570 575 580

TTT ATG CGG CAA GGA TGC GAT ATT TCG CCA AGA TCC CTC TCT GGG CGC 2174
Phe Met Arg Gln Gly Cys Asp Ile Ser Pro Arg Ser Leu Ser Gly Arg
585 590 595 600

ATT GTT GGA GGT GTG TGG TGG TTC TTT ACC CTG ATC ATA ATC TCC TCC 2222
Ile Val Gly Gly Val Trp Trp Phe Phe Thr Leu Ile Ile Ile Ser Ser
605 610 615

TAC ACG GCT AAC TTA GCT GCC TTC CTG ACT GTA GAG AGG ATG GTG TCT 2270
Tyr Thr Ala Asn Leu Ala Ala Phe Leu Thr Val Glu Arg Met Val Ser
620 625 630

CCC ATC GAA AGT GCT GAG GAT CTT TCT AAG CAA ACA GAA ATT GCT TAT 2318
Pro Ile Glu Ser Ala Glu Asp Leu Ser Lys Gln Thr Glu Ile Ala Tyr
635 640 645

GGA ACA TTA GAC TCT GGC TCC ACT AAA GAG TTT TTC AGG AGA TCT AAA 2366
Gly Thr Leu Asp Ser Gly Ser Thr Lys Glu Phe Phe Arg Arg Ser Lys
650 655 660

ATT GCA GTG TTT GAT AAA ATG TGG ACC TAC ATG CGG AGT GCG GAG CCC 2414
Ile Ala Val Phe Asp Lys Met Trp Thr Tyr Met Arg Ser Ala Glu Pro
665 670 675 680

TCT GTG TTT GTG AGG ACT ACG GCC GAA GGG GTG GCT AGA GTG CGG AAG 2462
Ser Val Phe Val Arg Thr Thr Ala Glu Gly Val Ala Arg Val Arg Lys
685 690 695

TCC AAA GGG AAA TAT GCC TAC TTG TTG GAG TCC ACG ATG AAC GAG TAC 2510
Ser Lys Gly Lys Tyr Ala Tyr Leu Leu Glu Ser Thr Met Asn Glu Tyr
700 705 710

ATT GAG CAA AGG AAG CCT TGC GAC ACC ATG AAA GTT GGT GGA AAC CTG 2558
Ile Glu Gln Arg Lys Pro Cys Asp Thr Met Lys Val Gly Gly Asn Leu
715 720 725

GAT TCC AAA GGC TAT GGC ATC GCA ACA CCT AAA GGA TCC TCA TTA GGA 2606
Asp Ser Lys Gly Tyr Gly Ile Ala Thr Pro Lys Gly Ser Ser Leu Gly
730 735 740

ACC CCA GTA AAT CTT GCA GTA TTG AAA CTC AGT GAG CAA GGC GTC TTA 2654
Thr Pro Val Asn Leu Ala Val Leu Lys Leu Ser Glu Gln Gly Val Leu
745 750 755 760

GAC AAG CTG AAA AAC AAA TGG TGG TAC GAT AAA GGT GAA TGT GGA GCC 2702
Asp Lys Leu Lys Asn Lys Trp Trp Tyr Asp Lys Gly Glu Cys Gly Ala
765 770 775

AAG GAC TCT GGA AGT AAG GAA AAG ACC AGT GCC CTC AGT CTG AGC AAC 2750
Lys Asp Ser Gly Ser Lys Glu Lys Thr Ser Ala Leu Ser Leu Ser Asn
780 785 790

GTT GCT GGA GTA TTC TAC ATC CTT GTC GGG GGC CTT GGT TTG GCA ATG 2798
Val Ala Gly Val Phe Tyr Ile Leu Val Gly Gly Leu Gly Leu Ala Met
795 800 805

CTG GTG GCT TTG ATT GAG TTC TGT TAC AAG TCA AGG GCC GAG GCG AAA 2846
Leu Val Ala Leu Ile Glu Phe Cys Tyr Lys Ser Arg Ala Glu Ala Lys
810 815 820

CGA ATG AAG GTG GCA AAG AAT GCA CAG AAT ATT AAC CCA TCT TCC TCG 2894
Arg Met Lys Val Ala Lys Asn Ala Gln Asn Ile Asn Pro Ser Ser Ser
825 830 835 840

CAG AAT TCA CAG AAT TTT GCA ACT TAT AAG GAA GGT TAC AAC GTA TAT 2942
Gln Asn Ser Gln Asn Phe Ala Thr Tyr Lys Glu Gly Tyr Asn Val Tyr
845 850 855

GGC ATC GAA AGT GTT AAA ATT TAGGGGATGA CCTTGAATGA TGCCATGAGG 2993
Gly Ile Glu Ser Val Lys Ile
860

AACAAGGCAA GGCTGTCAAT TACAGGAAGT ACTGGAGAAA ATGGACGTGT TATGACTCCA 3053

GAATTTCCCA AAGCNGTGCA TGCTGTCCCT TACGTGAGTC CTGGCATGGG AATGAATGTC 3113

AGTGTGACTG ATCTCTCGTG ATTGATAAGA ACCTTTTGAG TGCCTTACAC AATGGTTTTC 3173

TTGTGTGTTT ATTGTCAAAG TGGTGAGAGG CATCCAGTAT CTTGAAGACT TTTCTTTCAG 3233

CCAAGAATTC TTAAATATGT GGAGTTCATC TTGAATTGTA AGGAATGATT AATTAAAACA 3293

CAACATCTTT TTCTACTCGA GTTACAGACA AAGCGTGGTG GACATGCACA GCTAACATGG 3353

AAGTACTATA ATTTACCTGA AGTCTTTGTA CAGACAACAA ACCTGTTTCT GCAG 3407

(2) INFORMATION FOR SEQ ID NO:4:

(i) SEQUENCE CHARACTERISTICS:
(A) LENGTH: 883 amino acids
(B) TYPE: amino acid
(D) TOPOLOGY: linear

(ii) MOLECULE TYPE: protein

(xi) SEQUENCE DESCRIPTION: SEQ ID NO:4:

Met Gln Lys Ile Met His Ile Ser Val Leu Leu Ser Pro Val Leu Trp
-20 -15 -10 -5

Gly Leu Ile Phe Gly Val Ser Ser Asn Ser Ile Gln Ile Gly Gly Leu
1 5 10

Phe Pro Arg Gly Ala Asp Gln Glu Tyr Ser Ala Phe Arg Val Gly Met
15 20 25

Val Gln Phe Ser Thr Ser Glu Phe Arg Leu Thr Pro His Ile Asp Asn
30 35 40

Leu Glu Val Ala Asn Ser Phe Ala Val Thr Asn Ala Phe Cys Ser Gln
45 50 55 60

Phe Ser Arg Gly Val Tyr Ala Ile Phe Gly Phe Tyr Asp Lys Lys Ser
65 70 75

Val Asn Thr Ile Thr Ser Phe Cys Gly Thr Leu His Val Ser Phe Ile
80 85 90

Thr Pro Ser Phe Pro Thr Asp Gly Thr His Pro Phe Val Ile Gln Met
95 100 105

Arg Pro Asp Leu Lys Gly Ala Leu Leu Ser Leu Ile Glu Tyr Tyr Gln
    110                 115                 120

Trp Asp Lys Phe Ala Tyr Leu Tyr Asp Ser Asp Arg Gly Leu Ser Thr
125                 130                 135                 140

Leu Gln Ala Val Leu Asp Ser Ala Ala Glu Lys Lys Trp Gln Val Thr
                145                 150                 155

Ala Ile Asn Val Gly Asn Ile Asn Asn Asp Lys Lys Asp Glu Met Tyr
            160                 165                 170

Arg Ser Leu Phe Gln Asp Leu Glu Leu Lys Lys Glu Arg Arg Val Ile
        175                 180                 185

Leu Asp Cys Glu Arg Asp Lys Val Asn Asp Ile Val Asp Gln Val Ile
    190                 195                 200

Thr Ile Gly Lys His Val Lys Gly Tyr His Tyr Ile Ile Ala Asn Leu
205                 210                 215                 220

Gly Phe Thr Asp Gly Asp Leu Leu Lys Ile Gln Phe Gly Gly Ala Asn
                225                 230                 235

Val Ser Gly Phe Gln Ile Val Asp Tyr Asp Asp Ser Leu Val Ser Lys
            240                 245                 250

Phe Ile Glu Arg Trp Ser Thr Leu Glu Glu Lys Glu Tyr Pro Gly Ala
        255                 260                 265

His Thr Thr Thr Ile Lys Tyr Thr Ser Ala Leu Thr Tyr Asp Ala Val
    270                 275                 280

Gln Val Met Thr Glu Ala Phe Arg Asn Leu Arg Lys Gln Arg Ile Glu
285                 290                 295                 300

Ile Ser Arg Arg Gly Asn Ala Gly Asp Cys Leu Ala Asn Pro Ala Val
                305                 310                 315

Pro Trp Gly Gln Gly Val Glu Ile Glu Arg Ala Leu Lys Gln Val Gln
            320                 325                 330

Val Glu Gly Leu Ser Gly Asn Ile Lys Phe Asp Gln Asn Gly Lys Arg
        335                 340                 345

Ile Asn Tyr Thr Ile Asn Ile Met Glu Leu Lys Thr Asn Gly Pro Arg
    350                 355                 360

Lys Ile Gly Tyr Trp Ser Glu Val Asp Lys Met Val Val Thr Leu Thr
365                 370                 375                 380

Glu Leu Pro Ser Gly Asn Asp Thr Ser Gly Leu Glu Asn Lys Thr Val
                385                 390                 395

Val Val Thr Thr Ile Leu Glu Ser Pro Tyr Val Met Met Lys Lys Asn
            400                 405                 410

His Glu Met Leu Glu Gly Asn Glu Arg Tyr Glu Gly Tyr Cys Val Asp
        415                 420                 425

Leu Ala Ala Glu Ile Ala Lys His Cys Gly Phe Lys Tyr Lys Leu Thr
    430                 435                 440

Ile Val Gly Asp Gly Lys Tyr Gly Ala Arg Asp Ala Asp Thr Lys Ile
445                 450                 455                 460

Trp Asn Gly Met Val Gly Glu Leu Val Tyr Gly Lys Ala Asp Ile Ala
                465                 470                 475

Ile Ala Pro Leu Thr Ile Thr Leu Val Arg Glu Glu Val Ile Asp Phe
            480                 485                 490

Ser Lys Pro Phe Met Ser Leu Gly Ile Ser Ile Met Ile Lys Lys Pro
        495                 500                 505

Gln Lys Ser Lys Pro Gly Val Phe Ser Phe Leu Asp Pro Leu Ala Tyr
    510                 515                 520

Glu Ile Trp Met Cys Ile Val Phe Ala Tyr Ile Gly Val Ser Val Val
525                 530                 535                 540

Leu Phe Leu Val Ser Arg Phe Ser Pro Tyr Glu Trp His Thr Glu Glu
                545                 550                 555

Phe Glu Asp Gly Arg Glu Thr Gln Ser Ser Glu Ser Thr Asn Glu Phe
            560                 565                 570

Gly Ile Phe Asn Ser Leu Trp Phe Ser Leu Gly Ala Phe Met Arg Gln
        575                 580                 585

Gly Cys Asp Ile Ser Pro Arg Ser Leu Ser Gly Arg Ile Val Gly Gly
    590                 595                 600

Val Trp Trp Phe Phe Thr Leu Ile Ile Ile Ser Ser Tyr Thr Ala Asn
605                 610                 615                 620

Leu Ala Ala Phe Leu Thr Val Glu Arg Met Val Ser Pro Ile Glu Ser
                625                 630                 635

Ala Glu Asp Leu Ser Lys Gln Thr Glu Ile Ala Tyr Gly Thr Leu Asp
            640                 645                 650

Ser Gly Ser Thr Lys Glu Phe Phe Arg Arg Ser Lys Ile Ala Val Phe
        655                 660                 665

Asp Lys Met Trp Thr Tyr Met Arg Ser Ala Glu Pro Ser Val Phe Val
    670                 675                 680

Arg Thr Thr Ala Glu Gly Val Ala Arg Val Arg Lys Ser Lys Gly Lys
685                 690                 695                 700

Tyr Ala Tyr Leu Leu Glu Ser Thr Met Asn Glu Tyr Ile Glu Gln Arg
                705                 710                 715

Lys Pro Cys Asp Thr Met Lys Val Gly Gly Asn Leu Asp Ser Lys Gly
            720                 725                 730

Tyr Gly Ile Ala Thr Pro Lys Gly Ser Ser Leu Gly Thr Pro Val Asn
        735                 740                 745

Leu Ala Val Leu Lys Leu Ser Glu Gln Gly Val Leu Asp Lys Leu Lys
    750                 755                 760

Asn Lys Trp Trp Tyr Asp Lys Gly Glu Cys Gly Ala Lys Asp Ser Gly
765                 770                 775                 780

Ser Lys Glu Lys Thr Ser Ala Leu Ser Leu Ser Asn Val Ala Gly Val
                785                 790                 795

Phe Tyr Ile Leu Val Gly Gly Leu Gly Leu Ala Met Leu Val Ala Leu
            800                 805                 810

Ile Glu Phe Cys Tyr Lys Ser Arg Ala Glu Ala Lys Arg Met Lys Val
815 820 825

Ala Lys Asn Ala Gln Asn Ile Asn Pro Ser Ser Ser Gln Asn Ser Gln
830 835 840

Asn Phe Ala Thr Tyr Lys Glu Gly Tyr Asn Val Tyr Gly Ile Glu Ser
845 850 855 860

Val Lys Ile

(2) INFORMATION FOR SEQ ID NO:5:

(i) SEQUENCE CHARACTERISTICS:
(A) LENGTH: 2761 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: sig_peptide
(B) LOCATION: 79..144

(ix) FEATURE:
(A) NAME/KEY: mat_peptide
(B) LOCATION: 145..2745

(ix) FEATURE:
(A) NAME/KEY: CDS
(B) LOCATION: 79..2745

(xi) SEQUENCE DESCRIPTION: SEQ ID NO:5:

GAATTCCTGA CGACTCCTGA GTTGCGCCCA TGCTCTTGTC AGCTTCGTTT TAGGCGTAGC 60

ATGGCCAGGC AGAAGAAA ATG GGG CAA AGC GTG CTC CGG GCG GTC TTC TTT 111
Met Gly Gln Ser Val Leu Arg Ala Val Phe Phe
-22 -20 -15

TTA GTC CTG GGG CTT TTG GGT CAT TCT CAC GGA GGA TTC CCC AAC ACC 159
Leu Val Leu Gly Leu Leu Gly His Ser His Gly Gly Phe Pro Asn Thr
-10 -5 1 5

ATC AGC ATA GGT GGA CTT TTC ATG AGA AAC ACA GTG CAG GAG CAC AGC 207
Ile Ser Ile Gly Gly Leu Phe Met Arg Asn Thr Val Gln Glu His Ser
10 15 20

GCT TTC CGC TTT GCC GTG CAG TTA TAC AAC ACC AAC CAG AAC ACC ACC 255
Ala Phe Arg Phe Ala Val Gln Leu Tyr Asn Thr Asn Gln Asn Thr Thr
25 30 35

GAG AAG CCC TTC CAT TTG AAT TAC CAC GTA GAT CAC TTG GAT TCC TCC 303
Glu Lys Pro Phe His Leu Asn Tyr His Val Asp His Leu Asp Ser Ser
40 45 50

AAT AGT TTT TCC GTG ACA AAT GCT TTC TGC TCC CAG TTC TCG AGA GGG 351
Asn Ser Phe Ser Val Thr Asn Ala Phe Cys Ser Gln Phe Ser Arg Gly
55 60 65

GTG TAT GCC ATC TTT GGA TTC TAT GAC CAG ATG TCA ATG AAC ACC CTG 399
Val Tyr Ala Ile Phe Gly Phe Tyr Asp Gln Met Ser Met Asn Thr Leu
70 75 80 85

ACC TCC TTC TGT GGG GCC CTG CAC ACA TCC TTT GTT ACG CCT AGC TTC 447
Thr Ser Phe Cys Gly Ala Leu His Thr Ser Phe Val Thr Pro Ser Phe
90 95 100

CCC ACT GAC GCA GAT GTG CAG TTT GTC ATC CAG ATG CGC CCA GCC TTG 495
Pro Thr Asp Ala Asp Val Gln Phe Val Ile Gln Met Arg Pro Ala Leu
105 110 115

AAG GGC GCT ATT CTG AGT CTT CTG GGT CAT TAC AAG TGG GAG AAG TTT 543
Lys Gly Ala Ile Leu Ser Leu Leu Gly His Tyr Lys Trp Glu Lys Phe
120 125 130

GTG TAC CTC TAT GAC ACA GAA CGA GGA TTT TCC ATC CTC CAA GCG ATT 591
Val Tyr Leu Tyr Asp Thr Glu Arg Gly Phe Ser Ile Leu Gln Ala Ile
135 140 145

ATG GAA GCA GCA GTG CAA AAC AAC TGG CAA GTA ACA GCA AGG TCT GTG 639
Met Glu Ala Ala Val Gln Asn Asn Trp Gln Val Thr Ala Arg Ser Val
150 155 160 165

GGA AAC ATA AAG GAC GTC CAA GAA TTC AGG CGC ATC ATT GAA GAA ATG 687
Gly Asn Ile Lys Asp Val Gln Glu Phe Arg Arg Ile Ile Glu Glu Met
170 175 180

GAC AGG AGG CAG GAA AAG CGA TAC TTG ATT GAC TGC GAA GTC GAA AGG 735
Asp Arg Arg Gln Glu Lys Arg Tyr Leu Ile Asp Cys Glu Val Glu Arg
185 190 195

ATT AAC ACA ATT TTG GAA CAG GTT GTG ATC CTA GGG AAA CAC TCA AGA 783
Ile Asn Thr Ile Leu Glu Gln Val Val Ile Leu Gly Lys His Ser Arg
200 205 210

GGT TAT CAC TAC ATG CTC GCT AAC CTG GGT TTT ACT GAT ATT TTA CTG 831
Gly Tyr His Tyr Met Leu Ala Asn Leu Gly Phe Thr Asp Ile Leu Leu
215 220 225

GAA AGA GTC ATG CAT GGG GGA GCC AAC ATT ACA GGT TTC CAG ATT GTC 879
Glu Arg Val Met His Gly Gly Ala Asn Ile Thr Gly Phe Gln Ile Val
230 235 240 245

AAC AAT GAA AAC CCT ATG GTT CAG CAG TTC ATA CAG CGC TGG GTG AGG 927
Asn Asn Glu Asn Pro Met Val Gln Gln Phe Ile Gln Arg Trp Val Arg
250 255 260

CTG GAT GAA AGG GAA TTC CCT GAA GCC AAG AAT GCA CCA CTA AAG TAT 975
Leu Asp Glu Arg Glu Phe Pro Glu Ala Lys Asn Ala Pro Leu Lys Tyr
265 270 275

ACA TCT GCA TTG ACA CAC GAC GCA ATA CTG GTC ATA GCA GAA GCT TTC 1023
Thr Ser Ala Leu Thr His Asp Ala Ile Leu Val Ile Ala Glu Ala Phe
280 285 290

CGC TAC CTG AGG AGG CAG CGA GTA GAT GTG TCC CGG AGA GGA AGT GCT 1071
Arg Tyr Leu Arg Arg Gln Arg Val Asp Val Ser Arg Arg Gly Ser Ala
295 300 305

GGA GAC TGC TTA GCA AAT CCT GCT GTG CCC TGG AGT CAA GGA ATT GAT 1119
Gly Asp Cys Leu Ala Asn Pro Ala Val Pro Trp Ser Gln Gly Ile Asp
310 315 320 325

ATT GAG AGA GCT CTG AAA ATG GTG CAA GTA CAA GGA ATG ACT GGA AAT 1167
Ile Glu Arg Ala Leu Lys Met Val Gln Val Gln Gly Met Thr Gly Asn
330 335 340

ATT CAA TTT GAC ACT TAT GGA CGT AGG ACA AAT TAT ACC ATC GAT GTG 1215
Ile Gln Phe Asp Thr Tyr Gly Arg Arg Thr Asn Tyr Thr Ile Asp Val
345 350 355

TAT GAA ATG AAA GTC AGT GGC TCT CGA AAA GCT GGC TAC TGG AAC GAG 1263
Tyr Glu Met Lys Val Ser Gly Ser Arg Lys Ala Gly Tyr Trp Asn Glu
360 365 370

TAT GAA AGG TTT GTG CCT TTC TCA GAT CAG CAA ATC AGC AAT GAC AGT 1311
Tyr Glu Arg Phe Val Pro Phe Ser Asp Gln Gln Ile Ser Asn Asp Ser
375 380 385

GCA TCC TCA GAG AAT CGG ACC ATA GTA GTG ACT ACC ATT CTG GAA TCA 1359
Ala Ser Ser Glu Asn Arg Thr Ile Val Val Thr Thr Ile Leu Glu Ser
390 395 400 405

CCA TAT GTA ATG TAC AAG AAG AAC CAT GAG CAA CTG GAA GGA AAT GAA 1407
Pro Tyr Val Met Tyr Lys Lys Asn His Glu Gln Leu Glu Gly Asn Glu
410 415 420

CGA TAT GAA GGC TAT TGT GTA GAC CTA GCC TAT GAA ATA GCC AAA CAT 1455
Arg Tyr Glu Gly Tyr Cys Val Asp Leu Ala Tyr Glu Ile Ala Lys His
425 430 435

GTA AGG ATC AAA TAC AAA TTG TCC ATC GTT GGT GAC GGG AAA TAT GGT 1503
Val Arg Ile Lys Tyr Lys Leu Ser Ile Val Gly Asp Gly Lys Tyr Gly
440 445 450

GCA AGG GAT CCA GAG ACT AAA ATA TGG AAC GGC ATG GTT GGG GAA CTT 1551
Ala Arg Asp Pro Glu Thr Lys Ile Trp Asn Gly Met Val Gly Glu Leu
455 460 465

GTC TAT GGG AGA GCT GAT ATA GCT GTT GCT CCA CTC ACT ATA ACA TTG 1599
Val Tyr Gly Arg Ala Asp Ile Ala Val Ala Pro Leu Thr Ile Thr Leu
470 475 480 485

GTC CGT GAA GAA GTC ATA GAT TTT TCA AAG CCA TTA ATG AGC CTG GGC 1647
Val Arg Glu Glu Val Ile Asp Phe Ser Lys Pro Leu Met Ser Leu Gly
490 495 500

ATC TCC ATC ATG ATA AAG AAG CCT CAG AAA TCA AAA CCA GGC GTA TTC 1695
Ile Ser Ile Met Ile Lys Lys Pro Gln Lys Ser Lys Pro Gly Val Phe
505 510 515

TCA TTT CTG GAT CCC CTG GCT TAT GAA ATC TGG ATG TGC ATT GTC TTT 1743
Ser Phe Leu Asp Pro Leu Ala Tyr Glu Ile Trp Met Cys Ile Val Phe
520 525 530

GCT TAC ATT GGA GTC AGC GTA GTT CTT TTC CTA GTC AGC AGG TTC AGT 1791
Ala Tyr Ile Gly Val Ser Val Val Leu Phe Leu Val Ser Arg Phe Ser
535 540 545

CCT TAT GAA TGG CAC TTG GAA GAC AAC AAT GAA GAA CCT CGT GAC CCA 1839
Pro Tyr Glu Trp His Leu Glu Asp Asn Asn Glu Glu Pro Arg Asp Pro
550 555 560 565

CAA AGT CCT CCT GAT CCT CCA AAT GAA TTT GGA ATA TTT AAC AGT CTT 1887
Gln Ser Pro Pro Asp Pro Pro Asn Glu Phe Gly Ile Phe Asn Ser Leu
570 575 580

TGG TTT TCC TTG GGT GCC TTT ATG CAG CAA GGA TGT GAT ATT TCT CCA 1935
Trp Phe Ser Leu Gly Ala Phe Met Gln Gln Gly Cys Asp Ile Ser Pro
585 590 595

AGA TCA CTC TCC GGG CGC ATT GTT GGA GGG GTT TGG TGG TTC TTC ACC 1983
Arg Ser Leu Ser Gly Arg Ile Val Gly Gly Val Trp Trp Phe Phe Thr
600 605 610

CTG ATC ATA ATT TCT TCC TAT ACT GCC AAT CTC GCT GCT TTC CTG ACT 2031
Leu Ile Ile Ile Ser Ser Tyr Thr Ala Asn Leu Ala Ala Phe Leu Thr
615 620 625

GTG GAG AGG ATG GTT TCT CCC ATA GAG AGT GCT GAA GAC TTA GCT AAA 2079
Val Glu Arg Met Val Ser Pro Ile Glu Ser Ala Glu Asp Leu Ala Lys
630 635 640 645

CAG ACT GAA ATT GCA TAT GGG ACC CTG GAC TCC GGT TCA ACA AAA GAA 2127
Gln Thr Glu Ile Ala Tyr Gly Thr Leu Asp Ser Gly Ser Thr Lys Glu
650 655 660

TTT TTC AGA AGA TCC AAA ATT GCT GTG TAC GAG AAA ATG TGG TCT TAC 2175
Phe Phe Arg Arg Ser Lys Ile Ala Val Tyr Glu Lys Met Trp Ser Tyr
665 670 675

ATG AAA TCA GCG GAG CCA TCT GTG TTT ACC AAA ACA ACA GCA GAC GGA 2223
Met Lys Ser Ala Glu Pro Ser Val Phe Thr Lys Thr Thr Ala Asp Gly
680 685 690

GTG GCC CGA GTG CGA AAG TCC AAG GGA AAG TTC GCC TTC CTG CTG GAG 2271
Val Ala Arg Val Arg Lys Ser Lys Gly Lys Phe Ala Phe Leu Leu Glu
695 700 705

TCA ACC ATG AAT GAG TAC ATT GAG CAG AGA AAA CCA TGT GAT ACG ATG 2319
Ser Thr Met Asn Glu Tyr Ile Glu Gln Arg Lys Pro Cys Asp Thr Met
710 715 720 725

AAA GTT GGT GGA AAT CTG GAT TCC AAA GGC TAT GGT GTG GCA ACC CCT 2367
Lys Val Gly Gly Asn Leu Asp Ser Lys Gly Tyr Gly Val Ala Thr Pro
730 735 740

AAA GGC TCA GCA TTA GGA AAT GCT GTT AAC CTG GCA GTA TTA AAA CTG 2415
Lys Gly Ser Ala Leu Gly Asn Ala Val Asn Leu Ala Val Leu Lys Leu
745 750 755

AAT GAG CAA GGC CTC TTG GAC AAA TTG AAA AAC AAA TGG TGG TAC GAC 2463
Asn Glu Gln Gly Leu Leu Asp Lys Leu Lys Asn Lys Trp Trp Tyr Asp
760 765 770

AAA GGA GAG TGC GGC AGC GGG GGC GGT GAC TCC AAG GAC AAG ACC AGC 2511
Lys Gly Glu Cys Gly Ser Gly Gly Gly Asp Ser Lys Asp Lys Thr Ser
775 780 785

GCT CTG AGC CTG AGC AAT GTG GCA GGC GTT TTC TAT ATA CTT GTC GGA 2559
Ala Leu Ser Leu Ser Asn Val Ala Gly Val Phe Tyr Ile Leu Val Gly
790 795 800 805

GGT CTG GGG CTG GCC ATG ATG GTG GCT TTG ATA GAA TTC TGT TAC AAA 2607
Gly Leu Gly Leu Ala Met Met Val Ala Leu Ile Glu Phe Cys Tyr Lys
810 815 820

TCA CGG GCA GAG TCC AAA CGC ATG AAA CTC ACA AAG AAC ACC CAA AAC 2655
Ser Arg Ala Glu Ser Lys Arg Met Lys Leu Thr Lys Asn Thr Gln Asn
825 830 835

TTT AAG CCT GCT CCT GCC ACC AAC ACT CAG AAT TAT GCT ACA TAC AGA 2703
Phe Lys Pro Ala Pro Ala Thr Asn Thr Gln Asn Tyr Ala Thr Tyr Arg
840 845 850

GAA GGC TAC AAC GTG TAT GGA ACA GAG AGT GTT AAG ATC TAGGGATCCC 2752
Glu Gly Tyr Asn Val Tyr Gly Thr Glu Ser Val Lys Ile
855 860 865

TTGGAATTC 2761

(2) INFORMATION FOR SEQ ID NO:6:

(i) SEQUENCE CHARACTERISTICS:
(A) LENGTH: 888 amino acids
(B) TYPE: amino acid
(D) TOPOLOGY: linear

(ii) MOLECULE TYPE: protein

(xi) SEQUENCE DESCRIPTION: SEQ ID NO:6:

Met Gly Gln Ser Val Leu Arg Ala Val Phe Phe Leu Val Leu Gly Leu
-22 -20 -15 -10

Leu Gly His Ser His Gly Gly Phe Pro Asn Thr Ile Ser Ile Gly Gly
-5 1 5 10

Leu Phe Met Arg Asn Thr Val Gln Glu His Ser Ala Phe Arg Phe Ala
15 20 25

Val Gln Leu Tyr Asn Thr Asn Gln Asn Thr Thr Glu Lys Pro Phe His
30 35 40

Leu Asn Tyr His Val Asp His Leu Asp Ser Ser Asn Ser Phe Ser Val
45 50 55

Thr Asn Ala Phe Cys Ser Gln Phe Ser Arg Gly Val Tyr Ala Ile Phe
60 65 70

Gly Phe Tyr Asp Gln Met Ser Met Asn Thr Leu Thr Ser Phe Cys Gly
75 80 85 90

Ala Leu His Thr Ser Phe Val Thr Pro Ser Phe Pro Thr Asp Ala Asp
95 100 105

Val Gln Phe Val Ile Gln Met Arg Pro Ala Leu Lys Gly Ala Ile Leu
110 115 120

Ser Leu Leu Gly His Tyr Lys Trp Glu Lys Phe Val Tyr Leu Tyr Asp
125 130 135

Thr Glu Arg Gly Phe Ser Ile Leu Gln Ala Ile Met Glu Ala Ala Val
140 145 150

Gln Asn Asn Trp Gln Val Thr Ala Arg Ser Val Gly Asn Ile Lys Asp
155 160 165 170

Val Gln Glu Phe Arg Arg Ile Ile Glu Glu Met Asp Arg Arg Gln Glu
175 180 185

Lys Arg Tyr Leu Ile Asp Cys Glu Val Glu Arg Ile Asn Thr Ile Leu
190 195 200

Glu Gln Val Val Ile Leu Gly Lys His Ser Arg Gly Tyr His Tyr Met
205 210 215

Leu Ala Asn Leu Gly Phe Thr Asp Ile Leu Leu Glu Arg Val Met His
220 225 230

Gly Gly Ala Asn Ile Thr Gly Phe Gln Ile Val Asn Asn Glu Asn Pro
235 240 245 250

Met Val Gln Gln Phe Ile Gln Arg Trp Val Arg Leu Asp Glu Arg Glu
255 260 265

Phe Pro Glu Ala Lys Asn Ala Pro Leu Lys Tyr Thr Ser Ala Leu Thr
270 275 280

His Asp Ala Ile Leu Val Ile Ala Glu Ala Phe Arg Tyr Leu Arg Arg
        285                 290                 295

Gln Arg Val Asp Val Ser Arg Arg Gly Ser Ala Gly Asp Cys Leu Ala
    300                 305                 310

Asn Pro Ala Val Pro Trp Ser Gln Gly Ile Asp Ile Glu Arg Ala Leu
315                 320                 325                 330

Lys Met Val Gln Val Gln Gly Met Thr Gly Asn Ile Gln Phe Asp Thr
                335                 340                 345

Tyr Gly Arg Arg Thr Asn Tyr Thr Ile Asp Val Tyr Glu Met Lys Val
            350                 355                 360

Ser Gly Ser Arg Lys Ala Gly Tyr Trp Asn Glu Tyr Glu Arg Phe Val
        365                 370                 375

Pro Phe Ser Asp Gln Gln Ile Ser Asn Asp Ser Ala Ser Ser Glu Asn
    380                 385                 390

Arg Thr Ile Val Val Thr Thr Ile Leu Glu Ser Pro Tyr Val Met Tyr
395                 400                 405                 410

Lys Lys Asn His Glu Gln Leu Glu Gly Asn Glu Arg Tyr Glu Gly Tyr
                415                 420                 425

Cys Val Asp Leu Ala Tyr Glu Ile Ala Lys His Val Arg Ile Lys Tyr
            430                 435                 440

Lys Leu Ser Ile Val Gly Asp Gly Lys Tyr Gly Ala Arg Asp Pro Glu
        445                 450                 455

Thr Lys Ile Trp Asn Gly Met Val Gly Glu Leu Val Tyr Gly Arg Ala
    460                 465                 470

Asp Ile Ala Val Ala Pro Leu Thr Ile Thr Leu Val Arg Glu Glu Val
475                 480                 485                 490

Ile Asp Phe Ser Lys Pro Leu Met Ser Leu Gly Ile Ser Ile Met Ile
                495                 500                 505

Lys Lys Pro Gln Lys Ser Lys Pro Gly Val Phe Ser Phe Leu Asp Pro
            510                 515                 520

Leu Ala Tyr Glu Ile Trp Met Cys Ile Val Phe Ala Tyr Ile Gly Val
        525                 530                 535

Ser Val Val Leu Phe Leu Val Ser Arg Phe Ser Pro Tyr Glu Trp His
    540                 545                 550

Leu Glu Asp Asn Asn Glu Glu Pro Arg Asp Pro Gln Ser Pro Pro Asp
555                 560                 565                 570

Pro Pro Asn Glu Phe Gly Ile Phe Asn Ser Leu Trp Phe Ser Leu Gly
                575                 580                 585

Ala Phe Met Gln Gln Gly Cys Asp Ile Ser Pro Arg Ser Leu Ser Gly
            590                 595                 600

Arg Ile Val Gly Gly Val Trp Trp Phe Phe Thr Leu Ile Ile Ile Ser
        605                 610                 615

Ser Tyr Thr Ala Asn Leu Ala Ala Phe Leu Thr Val Glu Arg Met Val
    620                 625                 630

Ser Pro Ile Glu Ser Ala Glu Asp Leu Ala Lys Gln Thr Glu Ile Ala
635                 640                 645                 650

Tyr Gly Thr Leu Asp Ser Gly Ser Thr Lys Glu Phe Phe Arg Arg Ser
                655                 660                 665

Lys Ile Ala Val Tyr Glu Lys Met Trp Ser Tyr Met Lys Ser Ala Glu
            670                 675                 680

Pro Ser Val Phe Thr Lys Thr Thr Ala Asp Gly Val Ala Arg Val Arg
        685                 690                 695

Lys Ser Lys Gly Lys Phe Ala Phe Leu Leu Glu Ser Thr Met Asn Glu
    700                 705                 710

Tyr Ile Glu Gln Arg Lys Pro Cys Asp Thr Met Lys Val Gly Gly Asn
715                 720                 725                 730

Leu Asp Ser Lys Gly Tyr Gly Val Ala Thr Pro Lys Gly Ser Ala Leu
                735                 740                 745

Gly Asn Ala Val Asn Leu Ala Val Leu Lys Leu Asn Glu Gln Gly Leu
            750                 755                 760

Leu Asp Lys Leu Lys Asn Lys Trp Trp Tyr Asp Lys Gly Glu Cys Gly
        765                 770                 775

Ser Gly Gly Gly Asp Ser Lys Asp Lys Thr Ser Ala Leu Ser Leu Ser
    780                 785                 790

Asn Val Ala Gly Val Phe Tyr Ile Leu Val Gly Gly Leu Gly Leu Ala
795                 800                 805                 810

Met Met Val Ala Leu Ile Glu Phe Cys Tyr Lys Ser Arg Ala Glu Ser
                815                 820                 825

Lys Arg Met Lys Leu Thr Lys Asn Thr Gln Asn Phe Lys Pro Ala Pro
            830                 835                 840

Ala Thr Asn Thr Gln Asn Tyr Ala Thr Tyr Arg Glu Gly Tyr Asn Val
        845                 850                 855

Tyr Gly Thr Glu Ser Val Lys Ile
    860                 865

(2) INFORMATION FOR SEQ ID NO:7:

(i) SEQUENCE CHARACTERISTICS:
(A) LENGTH: 3070 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: sig_peptide
(B) LOCATION: 79..144

(ix) FEATURE:
(A) NAME/KEY: mat_peptide
(B) LOCATION: 145..2745

(ix) FEATURE:
(A) NAME/KEY: CDS
(B) LOCATION: 79..2745

(xi) SEQUENCE DESCRIPTION: SEQ ID NO:7:

GAATTCCTGA CGACTCCTGA GTTGCGCCCA TGCTCTTGTC AGCTTCGTTT TAGGCGTAGC 60

ATGGCCAGGC AGAAGAAA ATG GGG CAA AGC GTG CTC CGG GCG GTC TTC TTT 111
Met Gly Gln Ser Val Leu Arg Ala Val Phe Phe
-22 -20 -15

TTA GTC CTG GGG CTT TTG GGT CAT TCT CAC GGA GGA TTC CCC AAC ACC 159
Leu Val Leu Gly Leu Leu Gly His Ser His Gly Gly Phe Pro Asn Thr
-10 -5 1 5

ATC AGC ATA GGT GGA CTT TTC ATG AGA AAC ACA GTG CAG GAG CAC AGC 207
Ile Ser Ile Gly Gly Leu Phe Met Arg Asn Thr Val Gln Glu His Ser
10 15 20

GCT TTC CGC TTT GCC GTG CAG TTA TAC AAC ACC AAC CAG AAC ACC ACC 255
Ala Phe Arg Phe Ala Val Gln Leu Tyr Asn Thr Asn Gln Asn Thr Thr
25 30 35

GAG AAG CCC TTC CAT TTG AAT TAC CAC GTA GAT CAC TTG GAT TCC TCC 303
Glu Lys Pro Phe His Leu Asn Tyr His Val Asp His Leu Asp Ser Ser
40 45 50

AAT AGT TTT TCC GTG ACA AAT GCT TTC TGC TCC CAG TTC TCG AGA GGG 351
Asn Ser Phe Ser Val Thr Asn Ala Phe Cys Ser Gln Phe Ser Arg Gly
55 60 65

GTG TAT GCC ATC TTT GGA TTC TAT GAC CAG ATG TCA ATG AAC ACC CTG 399
Val Tyr Ala Ile Phe Gly Phe Tyr Asp Gln Met Ser Met Asn Thr Leu
70 75 80 85

ACC TCC TTC TGT GGG GCC CTG CAC ACA TCC TTT GTT ACG CCT AGC TTC 447
Thr Ser Phe Cys Gly Ala Leu His Thr Ser Phe Val Thr Pro Ser Phe
90 95 100

CCC ACT GAC GCA GAT GTG CAG TTT GTC ATC CAG ATG CGC CCA GCC TTG 495
Pro Thr Asp Ala Asp Val Gln Phe Val Ile Gln Met Arg Pro Ala Leu
105 110 115

AAG GGC GCT ATT CTG AGT CTT CTG GGT CAT TAC AAG TGG GAG AAG TTT 543
Lys Gly Ala Ile Leu Ser Leu Leu Gly His Tyr Lys Trp Glu Lys Phe
120 125 130

GTG TAC CTC TAT GAC ACA GAA CGA GGA TTT TCC ATC CTC CAA GCG ATT 591
Val Tyr Leu Tyr Asp Thr Glu Arg Gly Phe Ser Ile Leu Gln Ala Ile
135 140 145

ATG GAA GCA GCA GTG CAA AAC AAC TGG CAA GTA ACA GCA AGG TCT GTG 639
Met Glu Ala Ala Val Gln Asn Asn Trp Gln Val Thr Ala Arg Ser Val
150 155 160 165

GGA AAC ATA AAG GAC GTC CAA GAA TTC AGG CGC ATC ATT GAA GAA ATG 687
Gly Asn Ile Lys Asp Val Gln Glu Phe Arg Arg Ile Ile Glu Glu Met
170 175 180

GAC AGG AGG CAG GAA AAG CGA TAC TTG ATT GAC TGC GAA GTC GAA AGG 735
Asp Arg Arg Gln Glu Lys Arg Tyr Leu Ile Asp Cys Glu Val Glu Arg
185 190 195

ATT AAC ACA ATT TTG GAA CAG GTT GTG ATC CTA GGG AAA CAC TCA AGA 783
Ile Asn Thr Ile Leu Glu Gln Val Val Ile Leu Gly Lys His Ser Arg
200 205 210

GGT TAT CAC TAC ATG CTC GCT AAC CTG GGT TTT ACT GAT ATT TTA CTG 831
Gly Tyr His Tyr Met Leu Ala Asn Leu Gly Phe Thr Asp Ile Leu Leu
215 220 225

GAA AGA GTC ATG CAT GGG GGA GCC AAC ATT ACA GGT TTC CAG ATT GTC 879
Glu Arg Val Met His Gly Gly Ala Asn Ile Thr Gly Phe Gln Ile Val
230 235 240 245

AAC AAT GAA AAC CCT ATG GTT CAG CAG TTC ATA CAG CGC TGG GTG AGG 927
Asn Asn Glu Asn Pro Met Val Gln Gln Phe Ile Gln Arg Trp Val Arg
250 255 260

CTG GAT GAA AGG GAA TTC CCT GAA GCC AAG AAT GCA CCA CTA AAG TAT 975
Leu Asp Glu Arg Glu Phe Pro Glu Ala Lys Asn Ala Pro Leu Lys Tyr
265 270 275

ACA TCT GCA TTG ACA CAC GAC GCA ATA CTG GTC ATA GCA GAA GCT TTC 1023
Thr Ser Ala Leu Thr His Asp Ala Ile Leu Val Ile Ala Glu Ala Phe
280 285 290

CGC TAC CTG AGG AGG CAG CGA GTA GAT GTG TCC CGG AGA GGA AGT GCT 1071
Arg Tyr Leu Arg Arg Gln Arg Val Asp Val Ser Arg Arg Gly Ser Ala
295 300 305

GGA GAC TGC TTA GCA AAT CCT GCT GTG CCC TGG AGT CAA GGA ATT GAT 1119
Gly Asp Cys Leu Ala Asn Pro Ala Val Pro Trp Ser Gln Gly Ile Asp
310 315 320 325

ATT GAG AGA GCT CTG AAA ATG GTG CAA GTA CAA GGA ATG ACT GGA AAT 1167
Ile Glu Arg Ala Leu Lys Met Val Gln Val Gln Gly Met Thr Gly Asn
330 335 340

ATT CAA TTT GAC ACT TAT GGA CGT AGG ACA AAT TAT ACC ATC GAT GTG 1215
Ile Gln Phe Asp Thr Tyr Gly Arg Arg Thr Asn Tyr Thr Ile Asp Val
345 350 355

TAT GAA ATG AAA GTC AGT GGC TCT CGA AAA GCT GGC TAC TGG AAC GAG 1263
Tyr Glu Met Lys Val Ser Gly Ser Arg Lys Ala Gly Tyr Trp Asn Glu
360 365 370

TAT GAA AGG TTT GTG CCT TTC TCA GAT CAG CAA ATC AGC AAT GAC AGT 1311
Tyr Glu Arg Phe Val Pro Phe Ser Asp Gln Gln Ile Ser Asn Asp Ser
375 380 385

GCA TCC TCA GAG AAT CGG ACC ATA GTA GTG ACT ACC ATT CTG GAA TCA 1359
Ala Ser Ser Glu Asn Arg Thr Ile Val Val Thr Thr Ile Leu Glu Ser
390 395 400 405

CCA TAT GTA ATG TAC AAG AAG AAC CAT GAG CAA CTG GAA GGA AAT GAA 1407
Pro Tyr Val Met Tyr Lys Lys Asn His Glu Gln Leu Glu Gly Asn Glu
410 415 420

CGA TAT GAA GGC TAT TGT GTA GAC CTA GCC TAT GAA ATA GCC AAA CAT 1455
Arg Tyr Glu Gly Tyr Cys Val Asp Leu Ala Tyr Glu Ile Ala Lys His
425 430 435

GTA AGG ATC AAA TAC AAA TTG TCC ATC GTT GGT GAC GGG AAA TAT GGT 1503
Val Arg Ile Lys Tyr Lys Leu Ser Ile Val Gly Asp Gly Lys Tyr Gly
440 445 450

GCA AGG GAT CCA GAG ACT AAA ATA TGG AAC GGC ATG GTT GGG GAA CTT 1551
Ala Arg Asp Pro Glu Thr Lys Ile Trp Asn Gly Met Val Gly Glu Leu
455 460 465

GTC TAT GGG AGA GCT GAT ATA GCT GTT GCT CCA CTC ACT ATA ACA TTG 1599
Val Tyr Gly Arg Ala Asp Ile Ala Val Ala Pro Leu Thr Ile Thr Leu
470 475 480 485

GTC CGT GAA GAA GTC ATA GAT TTT TCA AAG CCA TTA ATG AGC CTG GGC 1647
Val Arg Glu Glu Val Ile Asp Phe Ser Lys Pro Leu Met Ser Leu Gly
490 495 500

ATC TCC ATC ATG ATA AAG AAG CCT CAG AAA TCA AAA CCA GGC GTA TTC 1695
Ile Ser Ile Met Ile Lys Lys Pro Gln Lys Ser Lys Pro Gly Val Phe
505 510 515

TCA TTT CTG GAT CCC CTG GCT TAT GAA ATC TGG ATG TGC ATT GTC TTT 1743
Ser Phe Leu Asp Pro Leu Ala Tyr Glu Ile Trp Met Cys Ile Val Phe
520 525 530

GCT TAC ATT GGA GTC AGC GTA GTT CTT TTC CTA GTC AGC AGG TTC AGT 1791
Ala Tyr Ile Gly Val Ser Val Val Leu Phe Leu Val Ser Arg Phe Ser
535 540 545

CCT TAT GAA TGG CAC TTG GAA GAC AAC AAT GAA GAA CCT CGT GAC CCA 1839
Pro Tyr Glu Trp His Leu Glu Asp Asn Asn Glu Glu Pro Arg Asp Pro
550 555 560 565

CAA AGT CCT CCT GAT CCT CCA AAT GAA TTT GGA ATA TTT AAC AGT CTT 1887
Gln Ser Pro Pro Asp Pro Pro Asn Glu Phe Gly Ile Phe Asn Ser Leu
570 575 580

TGG TTT TCC TTG GGT GCC TTT ATG CAG CAA GGA TGT GAT ATT TCT CCA 1935
Trp Phe Ser Leu Gly Ala Phe Met Gln Gln Gly Cys Asp Ile Ser Pro
585 590 595

AGA TCA CTC TCC GGG CGC ATT GTT GGA GGG GTT TGG TGG TTC TTC ACC 1983
Arg Ser Leu Ser Gly Arg Ile Val Gly Gly Val Trp Trp Phe Phe Thr
600 605 610

CTG ATC ATA ATT TCT TCC TAT ACT GCC AAT CTC GCT GCT TTC CTG ACT 2031
Leu Ile Ile Ile Ser Ser Tyr Thr Ala Asn Leu Ala Ala Phe Leu Thr
615 620 625

GTG GAG AGG ATG GTT TCT CCC ATA GAG AGT GCT GAA GAC TTA GCT AAA 2079
Val Glu Arg Met Val Ser Pro Ile Glu Ser Ala Glu Asp Leu Ala Lys
630 635 640 645

CAG ACT GAA ATT GCA TAT GGG ACC CTG GAC TCC GGT TCA ACA AAA GAA 2127
Gln Thr Glu Ile Ala Tyr Gly Thr Leu Asp Ser Gly Ser Thr Lys Glu
650 655 660

TTT TTC AGA AGA TCC AAA ATT GCT GTG TAC GAG AAA ATG TGG TCT TAC 2175
Phe Phe Arg Arg Ser Lys Ile Ala Val Tyr Glu Lys Met Trp Ser Tyr
665 670 675

ATG AAA TCA GCG GAG CCA TCT GTG TTT ACC AAA ACA ACA GCA GAC GGA 2223
Met Lys Ser Ala Glu Pro Ser Val Phe Thr Lys Thr Thr Ala Asp Gly
680 685 690

GTG GCC CGA GTG CGA AAG TCC AAG GGA AAG TTC GCC TTC CTG CTG GAG 2271
Val Ala Arg Val Arg Lys Ser Lys Gly Lys Phe Ala Phe Leu Leu Glu
695 700 705

TCA ACC ATG AAT GAG TAC ATT GAG CAG AGA AAA CCA TGT GAT ACG ATG 2319
Ser Thr Met Asn Glu Tyr Ile Glu Gln Arg Lys Pro Cys Asp Thr Met
710 715 720 725

AAA GTT GGT GGA AAT CTG GAT TCC AAA GGC TAT GGT GTG GCA ACC CCT 2367
Lys Val Gly Gly Asn Leu Asp Ser Lys Gly Tyr Gly Val Ala Thr Pro
730 735 740

AAA GGC TCA GCA TTA GGA ACG CCT GTA AAC CTT GCA GTA TTG AAA CTC     2415
Lys Gly Ser Ala Leu Gly Thr Pro Val Asn Leu Ala Val Leu Lys Leu
            745                 750                 755

AGT GAA CAA GGC ATC TTA GAC AAG CTG AAA AAC AAA TGG TGG TAC GAT     2463
Ser Glu Gln Gly Ile Leu Asp Lys Leu Lys Asn Lys Trp Trp Tyr Asp
        760                 765                 770

AAG GGG GAA TGT GGA GCC AAG GAC TCC GGG AGT AAG GAC AAG ACC AGC     2511
Lys Gly Glu Cys Gly Ala Lys Asp Ser Gly Ser Lys Asp Lys Thr Ser
    775                 780                 785

GCT CTG AGC CTG AGC AAT GTG GCA GGC GTT TTC TAT ATA CTT GTC GGA     2559
Ala Leu Ser Leu Ser Asn Val Ala Gly Val Phe Tyr Ile Leu Val Gly
790                 795                 800                 805

GGT CTG GGG CTG GCC ATG ATG GTG GCT TTG ATA GAA TTC TGT TAC AAA     2607
Gly Leu Gly Leu Ala Met Met Val Ala Leu Ile Glu Phe Cys Tyr Lys
                810                 815                 820

TCA CGG GCA GAG TCC AAA CGC ATG AAA CTC ACA AAG AAC ACC CAA AAC     2655
Ser Arg Ala Glu Ser Lys Arg Met Lys Leu Thr Lys Asn Thr Gln Asn
            825                 830                 835

TTT AAG CCT GCT CCT GCC ACC AAC ACT CAG AAT TAT GCT ACA TAC AGA     2703
Phe Lys Pro Ala Pro Ala Thr Asn Thr Gln Asn Tyr Ala Thr Tyr Arg
        840                 845                 850

GAA GGC TAC AAC GTG TAT GGA ACA GAG AGT GTT AAG ATC TAGGGATCCC     2752
Glu Gly Tyr Asn Val Tyr Gly Thr Glu Ser Val Lys Ile
    855                 860                 865

TTCCCACTGG AGGCATGTGA TGAGAGGAAA TCACCGAAAA CGTGGCTGCT TCAAGGATCC     2812

TGAGCCAGAT TTCACTCTCC TTGGTGTCGG GCATGACACG AATATTGCTG ATGGTGCAAT     2872

GACCTTTCAA TAGGAAAAAC TGATTTTTTT TTTCCTTCAG TGCCTTATGG AACACTCTGA     2932

GACTCGCGAC AATGCAAACC ATCATTGAAA TCTTTTTGCT TTGCTTGAAA AAAAATAATT     2992

AAAATAAAAA CCAACAAAAA TGGACATGCA TCAAACCCTT GATGTATTAA TATTTATTAT     3052

AGTTTTCATT AGGAATTC                                                   3070

(2) INFORMATION FOR SEQ ID NO:8:

(i) SEQUENCE CHARACTERISTICS:
(A) LENGTH: 888 amino acids
(B) TYPE: amino acid
(D) TOPOLOGY: linear

(ii) MOLECULE TYPE: protein

(xi) SEQUENCE DESCRIPTION: SEQ ID NO:8:

Met Gly Gln Ser Val Leu Arg Ala Val Phe Phe Leu Val Leu Gly Leu
-22     -20                 -15                 -10

Leu Gly His Ser His Gly Gly Phe Pro Asn Thr Ile Ser Ile Gly Gly
     -5                   1               5                  10

Leu Phe Met Arg Asn Thr Val Gln Glu His Ser Ala Phe Arg Phe Ala
                15                  20                  25

Val Gln Leu Tyr Asn Thr Asn Gln Asn Thr Thr Glu Lys Pro Phe His
            30                  35                  40

Leu Asn Tyr His Val Asp His Leu Asp Ser Ser Asn Ser Phe Ser Val
        45                  50                  55

Thr Asn Ala Phe Cys Ser Gln Phe Ser Arg Gly Val Tyr Ala Ile Phe
    60                  65                  70

Gly Phe Tyr Asp Gln Met Ser Met Asn Thr Leu Thr Ser Phe Cys Gly
75                  80                  85                  90

Ala Leu His Thr Ser Phe Val Thr Pro Ser Phe Pro Thr Asp Ala Asp
                95                  100                 105

Val Gln Phe Val Ile Gln Met Arg Pro Ala Leu Lys Gly Ala Ile Leu
            110                 115                 120

Ser Leu Leu Gly His Tyr Lys Trp Glu Lys Phe Val Tyr Leu Tyr Asp
        125                 130                 135

Thr Glu Arg Gly Phe Ser Ile Leu Gln Ala Ile Met Glu Ala Ala Val
    140                 145                 150

Gln Asn Asn Trp Gln Val Thr Ala Arg Ser Val Gly Asn Ile Lys Asp
155                 160                 165                 170

Val Gln Glu Phe Arg Arg Ile Ile Glu Glu Met Asp Arg Arg Gln Glu
                175                 180                 185

Lys Arg Tyr Leu Ile Asp Cys Glu Val Glu Arg Ile Asn Thr Ile Leu
            190                 195                 200

Glu Gln Val Val Ile Leu Gly Lys His Ser Arg Gly Tyr His Tyr Met
        205                 210                 215

Leu Ala Asn Leu Gly Phe Thr Asp Ile Leu Leu Glu Arg Val Met His
    220                 225                 230

Gly Gly Ala Asn Ile Thr Gly Phe Gln Ile Val Asn Asn Glu Asn Pro
235                 240                 245                 250

Met Val Gln Gln Phe Ile Gln Arg Trp Val Arg Leu Asp Glu Arg Glu
                255                 260                 265

Phe Pro Glu Ala Lys Asn Ala Pro Leu Lys Tyr Thr Ser Ala Leu Thr
            270                 275                 280

His Asp Ala Ile Leu Val Ile Ala Glu Ala Phe Arg Tyr Leu Arg Arg
        285                 290                 295

Gln Arg Val Asp Val Ser Arg Arg Gly Ser Ala Gly Asp Cys Leu Ala
    300                 305                 310

Asn Pro Ala Val Pro Trp Ser Gln Gly Ile Asp Ile Glu Arg Ala Leu
315                 320                 325                 330

Lys Met Val Gln Val Gln Gly Met Thr Gly Asn Ile Gln Phe Asp Thr
                335                 340                 345

Tyr Gly Arg Arg Thr Asn Tyr Thr Ile Asp Val Tyr Glu Met Lys Val
            350                 355                 360

Ser Gly Ser Arg Lys Ala Gly Tyr Trp Asn Glu Tyr Glu Arg Phe Val
        365                 370                 375

Pro Phe Ser Asp Gln Gln Ile Ser Asn Asp Ser Ala Ser Ser Glu Asn
    380                 385                 390

Arg Thr Ile Val Val Thr Thr Ile Leu Glu Ser Pro Tyr Val Met Tyr
395                 400                 405                 410

Lys Lys Asn His Glu Gln Leu Glu Gly Asn Glu Arg Tyr Glu Gly Tyr
                415                 420                 425

Cys Val Asp Leu Ala Tyr Glu Ile Ala Lys His Val Arg Ile Lys Tyr
            430                 435                 440

Lys Leu Ser Ile Val Gly Asp Gly Lys Tyr Gly Ala Arg Asp Pro Glu
        445                 450                 455

Thr Lys Ile Trp Asn Gly Met Val Gly Glu Leu Val Tyr Gly Arg Ala
    460                 465                 470

Asp Ile Ala Val Ala Pro Leu Thr Ile Thr Leu Val Arg Glu Glu Val
475                 480                 485                 490

Ile Asp Phe Ser Lys Pro Leu Met Ser Leu Gly Ile Ser Ile Met Ile
                495                 500                 505

Lys Lys Pro Gln Lys Ser Lys Pro Gly Val Phe Ser Phe Leu Asp Pro
            510                 515                 520

Leu Ala Tyr Glu Ile Trp Met Cys Ile Val Phe Ala Tyr Ile Gly Val
        525                 530                 535

Ser Val Val Leu Phe Leu Val Ser Arg Phe Ser Pro Tyr Glu Trp His
    540                 545                 550

Leu Glu Asp Asn Asn Glu Glu Pro Arg Asp Pro Gln Ser Pro Pro Asp
555                 560                 565                 570

Pro Pro Asn Glu Phe Gly Ile Phe Asn Ser Leu Trp Phe Ser Leu Gly
                575                 580                 585

Ala Phe Met Gln Gln Gly Cys Asp Ile Ser Pro Arg Ser Leu Ser Gly
            590                 595                 600

Arg Ile Val Gly Gly Val Trp Trp Phe Phe Thr Leu Ile Ile Ile Ser
        605                 610                 615

Ser Tyr Thr Ala Asn Leu Ala Ala Phe Leu Thr Val Glu Arg Met Val
    620                 625                 630

Ser Pro Ile Glu Ser Ala Glu Asp Leu Ala Lys Gln Thr Glu Ile Ala
635                 640                 645                 650

Tyr Gly Thr Leu Asp Ser Gly Ser Thr Lys Glu Phe Phe Arg Arg Ser
                655                 660                 665

Lys Ile Ala Val Tyr Glu Lys Met Trp Ser Tyr Met Lys Ser Ala Glu
            670                 675                 680

Pro Ser Val Phe Thr Lys Thr Thr Ala Asp Gly Val Ala Arg Val Arg
        685                 690                 695

Lys Ser Lys Gly Lys Phe Ala Phe Leu Leu Glu Ser Thr Met Asn Glu
    700                 705                 710

Tyr Ile Glu Gln Arg Lys Pro Cys Asp Thr Met Lys Val Gly Gly Asn
715                 720                 725                 730

Leu Asp Ser Lys Gly Tyr Gly Val Ala Thr Pro Lys Gly Ser Ala Leu
                735                 740                 745

Gly Thr Pro Val Asn Leu Ala Val Leu Lys Leu Ser Glu Gln Gly Ile
750 755 760

Leu Asp Lys Leu Lys Asn Lys Trp Trp Tyr Asp Lys Gly Glu Cys Gly
765 770 775

Ala Lys Asp Ser Gly Ser Lys Asp Lys Thr Ser Ala Leu Ser Leu Ser
780 785 790

Asn Val Ala Gly Val Phe Tyr Ile Leu Val Gly Gly Leu Gly Leu Ala
795 800 805 810

Met Met Val Ala Leu Ile Glu Phe Cys Tyr Lys Ser Arg Ala Glu Ser
815 820 825

Lys Arg Met Lys Leu Thr Lys Asn Thr Gln Asn Phe Lys Pro Ala Pro
830 835 840

Ala Thr Asn Thr Gln Asn Tyr Ala Thr Tyr Arg Glu Gly Tyr Asn Val
845 850 855

Tyr Gly Thr Glu Ser Val Lys Ile
860 865

(2) INFORMATION FOR SEQ ID NO:9:

(i) SEQUENCE CHARACTERISTICS:
(A) LENGTH: 46 amino acids
(B) TYPE: amino acid
(D) TOPOLOGY: linear

(ii) MOLECULE TYPE: peptide

(xi) SEQUENCE DESCRIPTION: SEQ ID NO:9:

Gly Ser Ala Leu Gly Asn Ala Val Asn Leu Ala Val Leu Lys Leu Asn
1               5                   10                  15

Glu Gln Gly Leu Leu Asp Lys Leu Lys Asn Lys Trp Trp Tyr Asp Lys
            20                  25                  30

Gly Glu Cys Gly Ser Gly Gly Gly Asp Ser Lys Asp Lys Thr
        35                  40                  45

(2) INFORMATION FOR SEQ ID NO:10:

(i) SEQUENCE CHARACTERISTICS:
(A) LENGTH: 46 amino acids
(B) TYPE: amino acid
(D) TOPOLOGY: linear

(ii) MOLECULE TYPE: peptide

(xi) SEQUENCE DESCRIPTION: SEQ ID NO:10:

Gly Ser Ala Leu Gly Thr Pro Val Asn Leu Ala Val Leu Lys Leu Ser
1               5                   10                  15

Glu Gln Gly Ile Leu Asp Lys Leu Lys Asn Lys Trp Trp Tyr Asp Lys
            20                  25                  30

Gly Glu Cys Gly Ala Lys Asp Ser Gly Ser Lys Asp Lys Thr
        35                  40                  45

(2) INFORMATION FOR SEQ ID NO:11:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: Other nucleic acid;
(A) DESCRIPTION: Synthetic DNA oligonucleotide

(xi) SEQUENCE DESCRIPTION: SEQ ID NO:11:

AGCTTGCGGC CGC 13

(2) INFORMATION FOR SEQ ID NO:12:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: Other nucleic acid;
(A) DESCRIPTION: Synthetic DNA oligonucleotide

(iv) ANTI-SENSE: YES

(xi) SEQUENCE DESCRIPTION: SEQ ID NO:12:

GCGGCCGCA 9

(2) INFORMATION FOR SEQ ID NO:13:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: Other nucleic acid;
(A) DESCRIPTION: Synthetic DNA oligonucleotide

(xi) SEQUENCE DESCRIPTION: SEQ ID NO:13:

ACACTCAGAA TTACGCTACA TACAGAGAAG GCTACAACGT 40

(2) INFORMATION FOR SEQ ID NO:14:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: Other nucleic acid;
(A) DESCRIPTION: Synthetic DNA oligonucleotide

(xi) SEQUENCE DESCRIPTION: SEQ ID NO:14:

CCAGATCGAT ATTGTGAACA TCAGCGACAC GTTTGAGATG 40

(2) INFORMATION FOR SEQ ID NO:15:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: Other nucleic acid;
(A) DESCRIPTION: Synthetic DNA oligonucleotide

(xi) SEQUENCE DESCRIPTION: SEQ ID NO:15:

GTGAATGTGG AGCCAAGGAC TCGGGAAGTA AG 32